(19) Europäisches Patentamt
European Patent Office
Office européen des brevets

(11) EP 4 086 082 A1

(12) EUROPEAN PATENT APPLICATION

(43) Date of publication:
**09.11.2022 Bulletin 2022/45**

(21) Application number: **22159578.8**

(22) Date of filing: **03.02.2016**

(51) International Patent Classification (IPC):
***B41M 5/40*** *(2006.01)* ***H05K 7/20*** *(2006.01)*
***H01L 23/36*** *(2006.01)* ***A61F 7/02*** *(2006.01)*
***C09K 5/06*** *(2006.01)* ***F28D 20/02*** *(2006.01)*

(52) Cooperative Patent Classification (CPC):
**F28D 20/025; A61F 7/02; C09K 5/06; C09K 5/063; F28D 20/02; H01L 23/3737; H01L 23/42; H01L 23/427; H01L 23/4275; H01M 10/659;**
A61F 2007/0292; A61K 9/7007; B60K 2015/03092; F16L 53/30; F28D 1/06; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2015 US 201514614236**

(62) Document number(s) of the earlier application(s) in accordance with Art. 76 EPC:
**16747185.3 / 3 253 583**

(71) Applicant: **Latent Heat Solutions, LLC**
**Golden CO 80403 (US)**

(72) Inventors:
- **Hartmann, Mark**
  **Boulder, 80302 (US)**
- **Kelly, Joseph**
  **Arvada, 80004 (US)**

(74) Representative: **Markfort, Iris-Anne Lucie**
**Lorenz & Kollegen**
**Patentanwälte Partnerschaftsgesellschaft mbB**
**Alte Ulmer Straße 2**
**89522 Heidenheim (DE)**

Remarks:
This application was filed on 02-03-2022 as a divisional application to the application mentioned under INID code 62.

# (54) THERMAL MANAGEMENT MATERIAL FOR THERMAL MANAGEMENT

(57) A method is provided for manufacturing a polymeric film with thermal management capabilities. The method comprises mixing at least three compounds, wherein a first compound comprises a polymeric phase change material, a second compound comprises an additive, and a third compound comprises a molecule that, in its liquid form, acts as a solvent of the first and second compounds, and which is curable into a solid form as a polymer. Then, the method comprises applying a mixture of the first, second, and third compounds in a liquid form to a substrate, and curing the mixture into a solid state.

EP 4 086 082 A1

(52) Cooperative Patent Classification (CPC): (Cont.)
F28D 20/0034; F28D 2020/0013;
F28D 2020/0017; F28F 2270/00; Y02E 60/10;
Y02E 60/14

## Description

### CROSS-REFERENCE TO RELATED APPLICATIONS

This application claims priority to U.S. Application No. 14614236 filed February 4, 2015 and entitled "THERMAL MANAGEMENT FILMS CONTAINING PHASE CHANGE MATERIALS," the entire disclosure of which is hereby incorporated by reference for all proper purposes.

### FIELD OF THE DISCLOSURE

In general, the present disclosure relates to systems, structures, and compositions of films used for thermal management purposes, and methods of forming thereof.

### BACKGROUND OF THE DISCLOSURE

Energy is neither created nor destroyed, but rather moves from one form to another, which is often experienced in the physical world as the gain or loss of heat from an object. In almost every industry, some process, method, or product may be improved, and energy saved, by managing the heat transfer properties of some object. To that end, various mechanisms and materials have been used to either cool, heat, or insulate such objects. Some mechanisms require energy themselves to heat or cool objects. For example, refrigeration, air conditioning, fans, or cooling pipes with water all require electricity and substantial structures to accomplish cooling. Similarly, heating often requires substantial energy and structures. Insulation is used to both protect an object from losing heat and to provide heat dissipation from a heat-generating object. Insulation is typically a passive system of thermal management that relies on materials to accomplish the objectives of the insulation. Insulating materials are often specially formed to fit the shape of the object (e.g., an individual beverage cooler, a winter jacket, a pipe insulator). Sometimes, when insulating materials are used to fill a uniquely-shaped space, the materials are amorphous (e.g., blown-in attic insulation, gel, etc.).

There are many objects in many industries that might benefit from thermal management such as heating, cooling, and insulation, but which do not commonly employ thermal management techniques because the current methods and materials are cost prohibitive or simply do not exist. In particular, for smaller objects, uniquely shaped objects, and objects that do not experience drastic temperature changes under normal operating conditions, thermal management is often ignored. That is, if the complexity, cost, or size of creating a thermal management system for an object outweighs the benefit of the thermal management of the object, no thermal management will likely be attempted. However, as businesses and consumers have become aware of the importance of conserving energy and creating efficiency wherever possible, they have looked to previously ignored objects to thermally manage. For small objects, uniquely shaped objects, objects that only require minimal thermal management, or objects that have more than one of these attributes, a thermal management system or structure must have properties that allow it to be cost-effective, easy to use, and scalable for various applications.

Latent heat storage materials, otherwise known as phase change materials, have been used in a wide variety of industries and applications to achieve thermal management. However, phase change materials have not always been cost-effective, easy to use, or scalable for unique applications. Therefore, a need exists to remedy the deficiencies in current thermal management systems.

### SUMMARY OF THE DISCLOSURE

Exemplary embodiments are summarized below. These and other embodiments are more fully described in the Detailed Description section. It is to be understood, however, that there is no intention to limit the disclosure to the forms described in this Summary of the Disclosure or in the Detailed Description. One skilled in the art can recognize that there are numerous modifications, equivalents and alternative constructions that fall within the spirit and scope of the disclosure as expressed in the claims.

In one embodiment, a method is provided for manufacturing a polymeric film with thermal management capabilities. The method comprises mixing at least three compounds, wherein a first compound comprises a polymeric phase change material, a second compound comprises an additive, and a third compound comprises a molecule that, in its liquid form, acts as a solvent of the first and second compounds, and which is curable into a solid form as a polymer. Then, the method comprises applying a mixture of the first, second, and third compounds in a liquid form to a substrate, and curing the mixture into a solid state.

Another embodiment of the disclosure provides a film with thermal management capabilities, comprising at least one polymeric phase change material, at least one additive, and a solid polymeric matrix formed from a cured liquid monomeric solvent.

Another embodiment of the disclosure provides a system for thermal management comprising an object that emits heat and a film with thermal management capabilities wrapped around the object that emits heat. The film comprises at least one polymeric phase change material, at least one additive, and a solid polymeric matrix formed from a cured liquid monomeric solvent, wherein the phase change material has a transition temperature and a latent heat storage range that dissipates heat from the object that emits heat.

Many additional aspects and embodiments are described herein as would be recognized by one of ordinary skill in the art.

## BRIEF DESCRIPTION OF THE DRAWINGS

Various objects and advantages and a more complete understanding of the present disclosure are apparent and more readily appreciated by reference to the following Detailed Description and to the appended claims when taken in conjunction with the accompanying Drawings wherein:

Figure 1A depicts a film in accordance with embodiments of this disclosure.
Figure 1B depicts a film as in 1A, specifically illustrating its pliability.
Figure 1C depicts a film wrapped multiple times around a cylindrical object, in accordance with embodiments of this disclosure.
Figure 2 depicts components of a liquid temperature management material and how the components change through a process of curing into a solid temperature management material.
Figure 3 is a high-level depiction of an elastomer.
Figure 4 depicts components of a liquid temperature management material and how the components change to form an interpenetrating polymer network.
Figures 5A and 5B depict an interpenetrating polymer network and a semi-interpenetrating polymer network.
Figures 6A-6C show various layering embodiments of compositions of temperature management films in accordance with aspects of this disclosure.
Figures 7-10D show various embodiments of functional polymeric phase change materials;
Figure 11 shows one embodiment of the precisely branched polymers compared with randomly distributed polymers;
Figure 12 is a graph depicting the peak melting point of various copolymers;
Figure 13 is a graph depicting the heat of crystallization of various copolymers;
Figure 14 is a graph depicting the latent heat and melting point of various copolymers;
Figures 15A and 15B show details of a microcapsule as used in connection with various aspects of the present disclosure;
Figures 16A-16C show various layering embodiments that may be used in connection with aspects of the present disclosure;
Figure 17 shows embodiments of a temperature management film wrapped around industrial pipes and tanks.
Figure 18 shows how a temperature management film may be wrapped around a pipe.
Figure 19 shows a temperature management film wrapped around an air duct.
Figure 20 shows a temperature management film wrapped around electrical wires.
Figure 20A shows a temperature management film wrapped around a water heater.
Figures 21A and 21B show temperature management films wrapped around hydraulic pistons.
Figure 22 shows a temperature management film wrapped around a hydraulic pump.
Figure 23 shows a temperature management film implemented as athletic tape.
Figure 24 shows a temperature management film implemented as a cosmetic patch.
Figure 25 shows a temperature management film implemented as a first aid bandage.
Figure 26 shows a temperature management film implemented as a transdermal patch.
Figure 27 shows a temperature management film implemented as a wrap for forming casts.
Figures 28A-28D show temperature management films used for dissipating heat from solar panels.
Figure 29 shows a temperature management film wrapped around an electrochemical cell.
Figure 30 shows a temperature management film attached to a computer server.
Figure 31 is a flowchart showing a method of manufacturing temperature management films which may be traversed in accordance with embodiments of the present disclosure.

Other embodiments and aspects are disclosed herein, including various figures and process descriptions described and illustrated throughout the specification.

## DETAILED DESCRIPTION

Throughout this specification references are made to the use of various materials, combinations, chemical formulations and other aspects that may be used in various combinations to form one or more materials, end products, or compositions in accordance with aspects of the present disclosure. It should be understood, both to one of skill in the art as well as the examining divisions in the United States Patent Office and Patent Offices throughout the world, that each of the lists of materials, examples, and other embodiments are included herein in order to teach one of skill in the art that they may be combined into various alternative embodiments, without requiring specific claim permutations of these individual features. The claims as presented herein, as well as any potential future amendments to those claims, may include one or more combinations of these materials, ranges and other alternatives without departing from the spirit and scope of the disclosure described herein. In particular it is contemplated that one of skill in the art would recognize and find adequate support in the written description for any combination of the features disclosed herein, whether described in a single example or embodiment, or described in different sections of the written description. The description of these various examples and options is specifically drafted to comply with 35 U.S.C. § 112 of the United States Patent Laws, Article 123(2) of the European Patent Laws as well as other similar national country laws relating to the adequacy of the written description.

It should be clearly understood that by providing examples of specific compositions and methods in the later part of this description, applicant does not intend to limit the scope of the claims to any of those specific compositions. To the contrary, it is anticipated that any combination of the functional groups, phase change materials, and articles described herein may be utilized to achieve the novel aspects of the present disclosure. The claims are not intended to be limited to any of the specific compounds described in this disclosure or any disclosure incorporated herein.

The present disclosure provides various descriptions of materials, apparatuses, devices, systems, methods, and methods of manufacturing relating to films for the thermal management of a variety of objects. Throughout the disclosure, reference will be made to "films," which may alternatively be referred to as "tape," "sheets," or "layers," depending on the context. The terms should be given their ordinary meaning in the art and should not be construed to limit the thickness, thinness, size, shape, or pliability of any embodiments of the disclosure. Further, while designations such as "tape," may, for example, imply that an embodiment has adhesive properties, the use of any of the above-mentioned terms should not be construed to limit an embodiment to a particular composition of particular materials, or to a particular method of application. For example, a "tape" may or may not comprise an adhesive, or a film may be applied by wrapping a pre-made film or by coating directly on to an object.

There are many objects, devices, and materials that could benefit from thermal management by being wrapped or coated with films comprising temperature management materials (TMMs). Various types of TMMs will be described throughout the disclosure. One particularly useful class of TMM is latent heat storage materials, also known as phase change materials (PCMs). For the purposes of this disclosure, TMM can refer to any material or mixture of materials that have temperature management properties. Because PCMs are a subset of TMMs, a TMM that is not a PCM may be described as "containing" a PCM. Alternatively, a TMM composition may purely be a mixture of multiple PCMs; that is, one PCM could contain another PCM, and the composition may still be called a TMM. An aspect of the present disclosure is that a film containing PCMs may be manufactured by casting or coating a substrate. The substrate may have temperature management properties itself, such as being energy conductive. Alternatively, it may be reflective, and function as a reflecting insulating layer against the cold. The substrate may also have adhesive properties. In many embodiments, the film may be in a liquid state when it is initially cast or coated, and upon its application to the substrate, may be cured, such that it forms a solid TMM containing PCMs. Known properties of solid TMMs with dispersed PCMs include that they are non-corrosive, non-toxic, and chemically inert, making them easy to handle. Another aspect of this disclosure is that once the film is cast onto the substrate and cured, it may be thin and pliable enough to be applied to, or wrapped around, any number of objects that could benefit from thermal management. The objects may be small or large, uniquely shaped, or simple. Another aspect of the disclosure is that the cured film may be manufactured into various sizes of sheets, or may be easily cut to suit particular applications. Throughout this disclosure, films in accordance with embodiments of the disclosure may be referred to as PCM-TMMs, PCM-TMM compositions (of either liquid or solid form), or PCM-TMM films (of either liquid or solid form). Additionally, though many embodiments comprise a PCM-TMM composition that is in a liquid state at some point during the manufacturing process, such liquid compositions may be dried into a powder form before or during their application, and before their curing. Therefore, though reference is made to various liquid TMM compositions, it is to be understood that these compositions may become solid and converted into powder form in certain embodiments.

Coating objects with polymers, in general, by spraying and otherwise coating liquid or powder polymer compositions is known and widely disclosed. For example, polymers may be coated onto substrates by a process known as "thermal spraying." In this process, a polymer powder (or other sprayable polymeric forms) is injected into a heat source, such as a flame or plasma, and fired through a spray gun onto a heated substrate. Another known method of coating polymers is known as "spin coating." In this process, a polymer is dissolved into a volatile solvent so it exists in liquid

form. An amount of the polymer solution is place on top of a substrate, and the substrate is rotated at a high speed at an angular velocity in order to spread the fluid by centrifugal force. This rotation thins the coating. The rotation may be adjusted to achieve the desired thickness of the polymer. The volatile solvent may evaporate during this process, such that the remaining polymer film is solid. Further disclosures on spin coating can be found in W. Flack, D. Soong, A. Bell. And D. Hess, "A Mathematical Model for Spin Coating Polymer Resists," J. Appl. Phys., 56, 1199 (1984).

Additionally, various methods for curing films, including polymer films, are known. UV and radiation curing are used in a variety of applications, and have been disclosed in "UV Coatings: Basics, Recent Developments, and New Applications," by Reinhold Schwalm, Elsevier, 2007, which is incorporated herein by reference. Such processes and techniques are also disclosed in the technical bulletin "Ultraviolet and Electron (UV/EB) Cured Coatings, Inks, and Adhesives," published by the United States Environmental Protection Agency, July 2001, which is incorporated herein by reference.

Additionally, the use of latent heat storage materials, such as PCMs, in various industries is known and widely disclosed. PCMs are latent heat storage materials that absorb and store thermal heat during a change in material phase. The use of various forms and compositions of PCMs (microencapsulated or raw, for example), their methods of manufacture and applications thereof have been widely disclosed in fields such as textiles and fabrics, building insulation, and electronics. For example, see U.S. Patent No. 8,587,945, "Systems Structures and Materials for Electronic Device Cooling," incorporated herein by reference in its entirety.

However, it has been difficult to create coatings or films of TMMs with high particulate loading of PCMs or other additives using the known methods of coating and curing described above. High particulate loading of PCMs and other additives in TMMs is often desirable because of the thermal management requirements of the objects themselves. For example, a TMM with desirable thermal management properties may be comprised of 80% or more PCM and/or thermal conductivity additives. Such high particulate loading formulations are often very thick and viscous in a liquid form, such that solvent is required for application in known ways, such as thermal spraying or spin coating, and other ways listed throughout this disclosure. Films with high particulate loading of PCMs may be thicker than other simple polymer coatings because of the properties of the formulation itself. Additionally, the desired thickness of a film for thermal management purposes may be greater than the desired thickness of polymer coatings used for other types of protection. For example, some polymer coatings are used to protect objects from scratching, impact, water, or corrosion. Polymer coatings for thermal management may need to be thicker.

It is difficult to coat or cast thick polymer films and then dry and cure them by driving the solvent from the film. Generally, the film is too thick to release the solvent, and the solvent becomes trapped within the solution. This trapping results in problems such as foaming, bubbling, and uneven thickness in the dried or cured film. One aspect of the present disclosure is that a thick polymer solution, with high particulate loading of PCMs, may be coated onto a substrate when the solvent used in the solution is a molecule or monomer that is polymerizable (or reactable) itself through a curing process. The molecule or monomer may even be polymerizable into a polymeric PCM itself. Various embodiments of this method will be described in more detail throughout the disclosure.

In many applications, it would be desirable to have a film or tape that is thick enough to provide meaningful thermal management in a single layer, but also pliable enough to wrap around an object multiple times to adjust the overall thickness (and thermal management properties) of the TMM if desired. There are a number of applications for a film, tape, or sheet manufactured according to various embodiments of the present disclosure. The following paragraphs name several examples, but they are exemplary only and should not be construed to limit embodiments of the disclosure.

In industrial settings, there is a need to thermally manage pipes that transport hot materials. Currently, various materials and methods are employed to insulate industrial pipes. Foam and fiberglass pads are sometimes used, but they have the drawbacks of being bulky and requiring custom-fitting. Spray coating of insulating polymers is also used, but the coating often must be applied by a professional. Films or tapes according to embodiments of the present disclosure may be wrapped around pipes transporting hot materials, such as water or other chemicals, and may provide advantages of being thin, easily customizable, and easily applied by the end user of the equipment. The film may be wrapped around multiple times if, for example, multiple layers of the TMM are required to mitigate the heat generated from the pipe. A single piece of industrial equipment may have pipes of various sizes or which generate varying amounts of heat. Additionally, some pipes may have more clearance room around them than others. Certain companies may have multiple such machines, each having multiple pipes, with additional variety in sizes and other properties. For these reasons, it may be advantageous to provide a film configured as a sheet. The film may be substantially solid at room temperature and non-toxic, making it easy to transport and handle. The film may be transported in layers of flat sheets, or in a roll, for example. The film may have properties that allow it to be fairly easily cut into smaller pieces with a knife or a blade. Alternatively, the sheet may be perforated such that it may be torn manually. The film may have an adhesive backing, or alternatively, the film may have "sticky" or "tacky" properties such that it grips to certain substrates and to itself. The properties listed herein would allow a user of one or more industrial machine with heat-generating pipes to receive sheets of the PCM-TMM film, then cut and wrap individual pipes in a customizable manner. The potential advantages are wide-ranging. A user might be able to replace a mechanical cooling system comprising fans or water pumps for a large pipe.

Such a large pipe might require a great amount of heat dissipation-hence the mechanical cooling system. The mechanical cooling system might take up a substantial amount of room and electricity

Another industrial application of a film would be wrapping the film around a tank or other container that holds material requiring thermal management. A holding tank would likely have very different dimensions than the pipes connecting to it, and would likely have different thermal management requirements. An advantage to providing a TMM sheet is that the sheet could be wrapped or layered multiple times around a tank no matter what the size or shape of the particular tank.

One type of industrial equipment in which overheating is of great concern is hydraulic systems. Hydraulic systems typically produce large amounts of heat, and hydraulic fluid must be maintained under a threshold temperature (e.g., 180° F), or else the liquid may damage seals and cause leaks, and the fluid itself may degrade rapidly. Often, complicated and expensive active cooling mechanisms are employed to manage overheating of hydraulic systems. Because hydraulic systems come in a wide variety of shapes, sizes, and applications, the cooling systems also come in a wide variety. Sheets of PCM-TMM film or tape could be easily customized to the various sizes and shapes of hydraulic equipment and could potentially eliminate parts of mechanical cooling systems.

Yet another application of a film would be for wrapping heat-generating wires. While plastic, rubber, and polymer materials are widely used for protecting wires from damage or from conducting electrical energy, they do not necessarily achieve thermal management. Thermal management of wires may be accomplished by wrapping them with a PCM-TMM film, which may allow the conductivity properties of the wires themselves to be managed.

Further, other heat-generating electronic components may be thermally managed by a PCM-TMM tape or film, from small items such as individual circuit boards to large ones such as servers. TMMs for thermal management of electronics are known, but many types of electronics, even fairly new ones, do not have integrated TMMs built into them. The performance and longevity of many devices could be improved by simply placing a device on a PCM-TMM sheet. For example, a laptop sitting on top of a desk could perform better if heat was dissipated away from it by a sheet of PCM-TMM placed between the laptop and the desk. Alternatively, an adhesive PCM-TMM tape could be affixed to the bottom of the laptop, creating a protective barrier from the heat and increasing the comfort for a user who used the laptop on an actual lap. Servers are well known for their heat management requirements, and mechanical cooling systems including fans and heat sinks have long been used. However, with servers, there is an ever-increasing need to improve capacity and performance and reduce physical space and energy consumption. Great efficiencies could be achieved by companies who have their own large server racks, or by service providers that host servers and data storage for other companies. Passive, effective thermal management could be realized by wrapping inner components or outer surfaces of servers themselves with PCM-TMM films.

Automotive applications may also utilize PCM-TMM films. Any number of hoses, wires, tanks, engines, batteries, pipes, or other automotive components may be wrapped. PCM-TMM film used in these applications may comprise additives to achieve other properties, such as corrosion resistance, fire resistance, and impact resistance.

Batteries and other electrochemical cells are also well known for challenges with overheating. Each cell produces its own heat through charge and discharge cycles, and both performance and longevity are adversely affected by prolonged elevated temperatures. PCM-TMM film wrapped around batteries can be especially useful because individual cells come in wide varieties of sizes and often cannot accommodate much space for temperature management in their surrounding areas. For further disclosure on how films may be constructed for the thermal management of electrochemical cells in particular, see the commonly owned and co-pending application entitled "Systems, Structures, and Materials for Electrochemical Device Thermal Management," U.S. Patent Application No. 14614223, filed on February 4, 2015 (Attorney Docket No. OUTT.048.00US), which is hereby incorporated by reference in its entirety.

Commercial and residential construction may also benefit from PCM-TMM film applications. For example, existing housing-wrap insulation, attic insulation, roofing materials, and floor underlayment may be improved by being comprised of PCM-TMM films. Commercial and residential components such as solar panels, furnaces, and water heaters may be layered or wrapped to control heat transfer or dissipation. One way homeowners can save energy is by insulating hot water pipes, especially those that run through low or no-insulation areas, such as under a house, in a garage, or near outer walls. Insulating pipes can reduce the energy used by a water heater to keep the water at a high enough temperature for the homeowner. However, insulating pipes may only elevate the temperature of the water inside a pipe a few degrees above what it would be without insulation. For a homeowner, this difference may seem negligible if it is difficult to insulate the pipes, or if the cost of the materials is high. PCM-TMM films may provide an advantage to a homeowner of being able to insulate pipes easily and inexpensively.

Temperature management of solar panels and solar cells would provide great benefits to the industry of solar energy production. Solar panels, by design, are exposed to the sun for many hours per day, and often operate at temperatures well above 130°F. Such levels of heat can reduce the level of efficiency in generating electricity and degrade the lifespan of the solar cells themselves. Active cooling processes are typically too expensive or inefficient to be used to cool solar cells, especially considering that a main purpose of using solar energy for electricity is to conserve resources, and that a barrier to widespread adoption of solar panels is cost. PCM-TMM films would be especially

advantageous in solar cell applications, because the temperature ranges are predictable and cyclical. A known property of PCMs is that they may effectively be "recharged" by dissipating stored heat when they are cooled. A PCM-TMM film may absorb and store excess heat generated by a solar cell during the day, and release it when it is cooler at night. The PCM may be recharged by this process and may undergo it repeatedly.

There are a number of industries in which films or tapes are used for purposes other than thermal management. For example, various types of tape are used for athletic purposes, such as taping joints (i.e., ankles, wrists, fingers, etc.). While the main purpose of athletic tape is typically stabilization of a joint susceptible to injury, additional benefits may be achieved by including thermal management materials in these tapes. A joint may be injured and produce excess heat as part of the inflammatory response, and TMMs could manage that excess heat. Additionally, an athlete with a taped ankle inside a shoe, or a taped wrist inside a boxing glove, could generate excess heat and moisture through physical activity, which could also be dissipated through the TMM. In gymnastics, athletes' hands are often taped to protect their skin and improve their grips. PCM-TMM tape could manage the heat generated by the athletes' hands as well as from the friction generated by gripping bars.

The appeal of temperature management materials in other areas in athletics is readily apparent, given the large industry of temperature management textiles and fabrics. Tapes for temperature management could expand the application within athletics. For example, gripping tape that is wrapped around a baseball bat or golf club could keep them from being cold to the touch in cold weather. Surfaces of metal bleachers and benches could be covered in sheets of PCM-TMM to prevent seating surfaces from being extremely cold or extremely hot.

In medical applications, there are already a variety of uses for wraps and tape, many of which could be improved by adding thermal management capabilities. For example, casts for broken bones are formed by plaster or fiberglass layers that harden. Bandages and braces of many varieties are formed by wraps. Injuries often produce excess heat due to an inflammatory response, which may be mitigated if the cast, bandage, or wrap was formed from a PCM-TMM film or tape. Additionally, many medications are delivered via adhesive transdermal patches. Some such medications may cause inflammation and heat build-up as a side effect, and an adhesive transdermal patch comprised of a PCM-TMM film could alleviate such a side effect. Some medications and even cosmetic products utilize adhesive films for the purpose of cooling the skin, and PCM-TMM films could be used in conjunction with or in place of certain chemicals used for such purposes.

One particular set of advantages attributable to manufacturing PCM-TMM films in accordance with embodiments of the disclosure is that PCM-TMM films may be applied to various substrates, allowing TMMs to be used in applications like never before. For example, in the prior art, when TMM was integrated into textiles, the manufacturing process of the textile itself would have to incorporate the TMM. With embodiments of the present disclosure, PCM-TMM films can be applied to textiles after they are manufactured, so that regular textiles may be adapted to fit purposes that require temperature management.

Another aspect of the disclosure is that some embodiments of the PCM-TMM films may be shrink-wrapped through the curing process. One advantage of shrink-wrapping is that unusually-shaped objects may be completely encased by a PCM-TMM film. It is contemplated that unusually-shaped objects may be coated in a TMM film by other methods, such as directly thermal-spraying the object and then curing it. However, shrink-wrapping provides an advantage to an end user for whom the spraying and curing is not a feasible option. An end user may receive a sheet or a roll of PCM-TMM film according to embodiments of this disclosure that is not in a particular shape, and may cut and shape it to approximately the needed size, then may apply heat sufficient to shrink-wrap the material around the object. Compositions that create shrink-wrapping properties will be described in further detail later in this disclosure.

Another aspect of the disclosure is that the film may have elastomeric properties. That is, the TMM itself and/or the PCM itself may stretch in order to ensure close contact with a wrapped object. In addition, these elastomeric properties can include thermal elasticity and rebound, or memory. That is, as the heat of the wrapped object itself changes and causes physical expansion and contraction of the object, the wrapped film can expand and contract with it in order to keep close contact. There are several advantages to of this kind of elastomeric property, especially in applications when the wrapping causes the object to be surrounded on all sides. One advantage is the reduction of air gaps between the wrapped film and the object's surface, which can be accomplished due to the elastomeric properties by maintaining very close contact. Reducing air gaps is an advantage because air is a poor conductor of heat away from a heat source. If air is trapped between an object and any surrounding material, the air gap can act as insulation and actually cause an object to get hotter than it would if there were no surrounding material. Currently, some tight-fitting materials such as plastics, PVC, foams, and fiberglass are used to protect and insulate a variety of objects described herein. Not only are these materials inferior to PCMs in terms of heat dissipating properties, but they additionally do not have elastomeric properties that minimize the effect of air gaps. As these materials expand and contract and lose contact with the object's surface, air gaps can actually increase, and the heat surrounding the object can increase. In objects that may be degraded by heat, these air gaps may further contribute to the degradation of the object over time.

Yet another aspect of the disclosure is that TMMs and/or PCMs comprising gels or greases may be used in conjunction with the solid film in order to facilitate the application of the film and to increase the contact between the film

and the object wrapped by the PCM-TMM. Because the gels and greases are in a liquid state, they may expand and contract and flow to areas between the object and the tape that may have tiny gaps. The combination of the elastomeric film and the gels or greases further diminishes the presence of air gaps. Another benefit of the tight fit accomplished by the elastomeric properties and/or gels and greases is that the contact between the outside of a wrapped object and the TMM can provide homogeneity in the overall temperature of the wrapped object. That is, some objects can normally generate hot spots, meaning one part of the object is much hotter than another. The PCM-TMM film in close contact can dissipate the heat generated by a hot spot to the PCM-TMM film that is directly in contact with the hot spot and transfer it to PCM-TMM film that is not directly in contact with the hot spot. As a result, the temperature around the entire object can remain substantially homogeneous. Many types of insulating materials that do not contain PCMs do not have this property.

Another aspect of the disclosure is that a PCM-TMM film can keep objects warm in extreme cold weather environments. One useful application would be to protect batteries from cold. It is known that extreme cold can cause battery cells to discharge more quickly and to lose capacity because the internal chemistry of the cell is disrupted. Because PCMs are latent-heat storage materials, they can insulate articles from the loss of heat and increase the temperature drop that would be necessary to produce detrimental effects. PCMs may also store heat at warmer temperatures, and as temperatures cool, they may release that heat into wrapped objects making them warmer. PCMs are also widely used to keep cold items cold, such as food, drinks or other organic substances. Various ranges of PCM properties necessary for insulating objects from extreme cold or to keep objects cold themselves will be described throughout the disclosure. Alternatively, a PCM-TMM film may primarily contain a PCM for the purposes of absorbing excess heat, but may also include one-way insulation such as a reflective layer or reflective insulation (e.g., as part of its substrate) for protection from excess cold. The industries and applications previously enumerated that may experience benefits from managing heat may have corresponding applications for managing cold.

Yet another aspect of the disclosure is that the PCM-TMM film may have corrosion resistant, waterproof, fire-retardant or impact-resistant properties. These aspects may comprise additives to the PCM, or to any TMM not comprising PCMs, or both. The advantages of such properties in various applications will be discussed in further detail in this disclosure.

Many aspects of the disclosure relate to thermal conductivity. Thermal conductivity, or the rate of heat transfer across a material, is expressed as watts per meter kelvin (W/(m·K)) or $W \cdot m^{-1} \cdot K^{-1}$, and is controlled by the flow of free electrons or crystal lattice vibrations (phonons). In metals, conductivity is primarily due to free electrons, whereas for nonmetals it is mainly due to phonon transport. Thermal conductivity can vary dependent on type of materials, temperature, material phase, impurities, etc. For instance, the change in thermal conductivity that occurs when ice (thermal conductivity of 2.18 W/(m·K) at 0 °C) melts into liquid water (thermal conductivity of 0.58 W/(m·K) at 0 °C). Pure crystalline substances can exhibit different thermal conductivities along different crystal axes, due to differences in phonon coupling along a given crystal axis. The thermal conductivity of plastics depends strongly on the degree of crystallinity in polymers (Anisotropicity of crystalline polymers). This is mostly due to phonon transport (flowing lattice vibrational energy) that is efficient along the crystalline axis but reduced substantially in amorphous areas or by various scattering processes in the other directions.

## Definitions

The following definitions apply to various elements described with respect to various aspects of the disclosure. These definitions may likewise be expanded upon herein.

As used herein, the term "monodisperse" refers to being substantially uniform with respect to a set of properties. Thus, for example, a set of microcapsules that are monodisperse can refer to such microcapsules that have a narrow distribution of sizes around a mode of the distribution of sizes, such as a mean of the distribution of sizes. A further example is a set of polymer molecules with similar molecular weights.

As used herein, the term "latent heat" refers to an amount of heat absorbed or released by a material as it undergoes a transition between two states. Thus, for example, a latent heat can refer to an amount of heat that is absorbed or released as a material undergoes a transition between a liquid state and a crystalline solid state, a liquid state and a gaseous state, a crystalline solid state and a gaseous state, two crystalline solid states or crystalline state and amorphous state or any combination thereof.

As used herein, the term "transition temperature" refers to an approximate temperature at which a material undergoes a transition between two states. Thus, for example, a transition temperature can refer to a temperature at which a material undergoes a transition between a liquid state and a crystalline solid state, a liquid state and a gaseous state, a crystalline solid state and a gaseous state, two crystalline solid states or crystalline state and amorphous state. A temperature at which an amorphous material undergoes a transition between a glassy state and a rubbery state may also be referred to as a "glass transition temperature" of the material or combination thereof.

As used herein, the term "phase change material" refers to a material that has the capability of absorbing or

releasing heat to adjust heat transfer at or within a temperature stabilizing range. A temperature stabilizing range can include a specific transition temperature or a range of transition temperatures. This allows for the adjustment of heat transfer or thermal conductivity within this transition range. In some instances, a phase change material can be capable of inhibiting heat transfer during a period of time when the phase change material is absorbing or releasing heat, typically as the phase change material undergoes a transition between two states. This action is typically transient and will occur until a latent heat of the phase change material is absorbed or released during a heating or cooling process. Heat can be stored or removed from a phase change material, and the phase change material typically can be effectively "recharged" by a source that emits or absorbs heat. For certain implementations, a phase change material can be a mixture of two or more materials. By selecting two or more different materials and forming a mixture, a temperature stabilizing range can be adjusted for any desired application. The resulting mixture can exhibit two or more different transition temperatures or a single modified transition temperature when incorporated in the articles described herein.

As used herein, the term "polymer" refers to a material that includes a set of macromolecules. Macromolecules included in a polymer can be the same or can differ from one another in some fashion. A macromolecule can have any of a variety of skeletal structures, and can include one or more types of monomeric units. In particular, a macromolecule can have a skeletal structure that is linear or non-linear. Examples of non-linear skeletal structures include branched skeletal structures, such those that are star branched, comb branched, or dendritic branched, and network skeletal structures. A macromolecule included in a homopolymer typically includes one type of monomeric unit, while a macromolecule included in a copolymer typically includes two or more types of monomeric units. Examples of copolymers include statistical copolymers, random copolymers, alternating copolymers, periodic copolymers, block copolymers, radial copolymers, and graft copolymers. In some instances, a reactivity and a functionality of a polymer can be altered by addition of a set of functional groups, such as acid anhydride groups, amino groups and their salts, N-substituted amino groups, amide groups, carbonyl groups, carboxy groups and their salts, cyclohexyl epoxy groups, epoxy groups, glycidyl groups, hydroxy groups, isocyanate groups, urea groups, aldehyde groups, ester groups, ether groups, alkenyl groups, alkynyl groups, thiol groups, disulfide groups, silyl or silane groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof. Such functional groups can be added at various places along the polymer, such as randomly or regularly dispersed along the polymer, at ends of the polymer, on the side, end or any position on the crystallizable side chains, attached as separate dangling side groups of the polymer, or attached directly to a backbone of the polymer. Also, a polymer can be capable of crosslinking, entanglement, network formation, ionic bonding, covalent bonding or hydrogen bonding in order to increase its mechanical strength or its resistance to degradation under ambient or processing conditions. As can be appreciated, a polymer can be provided in a variety of forms having different molecular weights, since a molecular weight of the polymer can be dependent upon processing conditions used for forming the polymer. Accordingly, a polymer can be referred to as having a specific molecular weight or a range of molecular weights. As used herein with reference to a polymer, the term "molecular weight" can refer to a number average molecular weight, a weight average molecular weight, or a melt index of the polymer.

Examples of polymers (including those polymers used for crosslinkers and binders) include polyhydroxyalkonates, polyamides, polyamines, polyimides, polyacrylics (e.g., polyacrylamide, polyacrylonitrile, and esters of methacrylic acid and acrylic acid), polycarbonates (e.g., polybisphenol A carbonate and polypropylene carbonate), polydienes (e.g., polybutadiene, polyisoprene, and polynorbornene), polyepoxides (e.g. bisphenol A, bisphenol F, multifunctional glycidyl based epoxies either cross-linked or uncrosslinked with amines, acids, alcohols, etc.), polyesters (e.g., polycaprolactone, polyethylene adipate, polybutylene adipate, polypropylene succinate, polyesters based on terephthalic acid, and polyesters based on phthalic acid), polyethers (e.g., polyethylene glycol or polyethylene oxide, polybutylene glycol, polypropylene oxide, polyoxymethylene or paraformaldehyde, polytetramethylene ether or polytetrahydrofuran, and polyepichlorohydrin), polyfluorocarbons, formaldehyde polymers (e.g., urea-formaldehyde, melamine-formaldehyde, and phenol formaldehyde), natural polymers (e.g., polysaccharides, such as cellulose, chitan, chitosan, and starch; lignins; proteins; and waxes), polyolefins (e.g., polyethylene, polypropylene, polybutylene, polybutene, and polyoctene), polyphenylenes, siliconcontaining polymers (e.g., polydimethyl siloxane, polyalkyl siloxanes and polycarbomethyl silane), polyurethanes, polyvinyls (e.g., polyvinyl butyral, polyvinyl alcohol, esters and ethers of polyvinyl alcohol, polyvinyl acetate, polystyrene, polymethylstyrene, polyvinyl chloride, polyvinyl pryrrolidone, polymethyl vinyl ether, polyethyl vinyl ether, and polyvinyl methyl ketone), polyacetals, polyarylates, alkyd-based polymers (e.g., polymers based on glyceride oil), copolymers (e.g., polyethylene-co-vinyl acetate and polyethylene-co-acrylic acid, styrene-butadiene, or any combination of the above), and mixtures thereof. The term polymer is meant to be construed to include any substances that become available after the filing of this application and that exhibit the general polymeric properties described above.

As used herein, the term "chemical bond" and its grammatical variations refer to a coupling of two or more atoms based on an attractive interaction, such that those atoms can form a stable structure. Examples of chemical bonds include covalent bonds and ionic bonds. Other examples of chemical bonds include hydrogen bonds and attractive

interactions between carboxy groups and amine groups.

As used herein, the term "covalent bond" means a form of chemical bonding that is characterized by the sharing of pairs of electrons between atoms, or between atoms and other covalent bonds. Attraction-to-repulsion stability that forms between atoms when they share electrons is known as covalent bonding. Covalent bonding includes many kinds of interactions, including $\sigma$-bonding, $\pi$-bonding, metalmetal bonding, agostic interactions, and three-center two-electron bonds.

As used herein, the term "ionic bond" or "electrovalent bond" means a bond formed through electrostatic attraction between oppositely charged ions. For example, between a positively charged cation and a negatively charged anion. Ionic bonds can be formed between a metal such Na, Fe, Ag, etc. and a nonmetal, or between two metals, or between two non-metals such as ammonia and acids. Ionic compounds can conduct electricity when molten, in a solid or in solution.

As used herein, the term "molecular group" and obvious variations thereof, refers to a set of atoms that form a portion of a molecule. In some instances, a group can include two or more atoms that are chemically bonded to one another to form a portion of a molecule. A group can be neutral on the one hand or charged on the other, e.g., monovalent or polyvalent (e.g., bivalent) to allow chemical bonding to a set of additional groups of a molecule. For example, a monovalent group can be envisioned as a molecule with a set of hydride groups removed to allow chemical bonding to another group of a molecule. A group can be neutral, positively charged, or negatively charged. For example, a positively charged group can be envisioned as a neutral group with one or more protons (i.e., H+) added, and a negatively charged group can be envisioned as a neutral group with one or more protons removed. A group that exhibits a characteristic reactivity or other set of properties can be referred to as a functional group, reactive function or reactive functional groups. Examples of reactive functional groups include those such as acid anhydride groups, amino groups, N-substituted amino groups and their salts, amide groups, carbonyl groups, carboxy groups and their salts, cyclohexyl epoxy groups, epoxy groups, glycidyl groups, hydroxy groups, isocyanate groups, urea groups, aldehyde groups, ester groups, ether groups, alkenyl groups, alkynyl groups, thiol groups, disulfide groups, silyl or silane groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof.

As used herein, the term "Melt flow index" or MFI is a measure of the ease of flow of the melt of a polymer. In academic terms the melt flow is defined as the mass of polymer, in grams, flowing in ten minutes through a capillary of a specific diameter and length by a pressure applied via prescribed alternative gravimetric weights for alternative prescribed temperatures. The method is described in the similar standards ASTM D1238 and ISO 1133.

As used herein, "Molecular Weight Polydispersity" (a polydispersity index (PDI)), is a measure of the distribution of molecular mass in a given polymer sample. The PDI calculated is the weight average molecular weight divided by the number average molecular weight. It indicates the distribution of individual molecular masses in a batch of polymers. The PDI has a value equal to or greater than 1, but as the polymer chains approach uniform chain length, the PDI approaches unity (1). For some natural polymers PDI is almost taken as unity. The PDI from polymerization is often denoted as:

$$PDI = M_w/M_n$$

Mn is more sensitive to molecules of low molecular mass, while Mw is more sensitive to molecules of high molecular mass. A polymer material is denoted by the term polydisperse if its chain lengths vary over a wide range of molecular masses.

As used herein, "Stereochemistry" means the study of the relative spatial arrangement of atoms within molecules. One branch of stereochemistry is the study of chiral molecules. Stereochemistry is also known as 3D chemistry. Examples, explanations, descriptions and definitions of various stereochemical nomenclature and naming regimes can be found in chapter 6 "Stereochemistry" in "Modern Physical Organic Chemistry" by Anslyn and Dougherty, ©2005, University Science Books.

Polymer stereochemistry descriptions of atactic, syndiotactic, isotactic, cis- and trans-, R- and S-, L-, D- and Meso- will be used.

As used herein, "Polymerization" is a process of reacting monomer molecules together in a chemical reaction to form three-dimensional networks or polymer chains. Many forms of polymerization and different systems exist to categorize them are known in the art.

As used herein, "Rheology" is the characterization of the flow of matter while "viscosity" is the measure of resistance to flow or deformation. Viscosity can be measured by various means and characterized as a melt flow index **(MFI)** or centipoise (cps), usually at a given temperature or shear rate.

As used herein, the term "thermal conductivity" ("k" and also denoted as λ or κ), is the property of a material's ability to conduct heat and is measured in W/m·K. Thermal conductivity is defined as the quantity of heat **(Q)** transmitted through a unit thickness **(L)** in a direction normal to a surface of unit area **(A)** due to a unit temperature gradient **(ΔT)** under steady state conditions and when the heat transfer is dependent only on the temperature gradient. In equation form this becomes the following:

$$\text{Thermal Conductivity} = \text{heat} \times \text{distance} / (\text{area} \times \text{temperature gradient})$$

$$\boldsymbol{\lambda = Q \times L / (A \times \Delta T)}$$

Generally in low conducting materials k < 0.1 W/m·K. In good conducting materials k = 0.1-10 W/m·K. In highly conducting material k>10 W/m·K. In accordance with aspects of the present disclosure the thermal management and heat dissipation material preferably has a k value of > 0.5 W/m·K. In another embodiment k >1.0 W/m·K and it yet another embodiment k is >10 W/m·K.

As used herein, the term "heat dissipation" refers to the movement or spreading of heat from a high temperature environment to a low temperature environment, e.g. moving heat from a warm object to cooler ambient air. Methods to dissipate heat can be accomplished by using high thermal conductivity materials such as metallic or ceramic heat spreaders, heat spreader plates, heat sinks, heat pipes, heat exchangers, loop pipes, liquid cold pipes, heat fins, fans, circulating coolants or a combination thereof. Further examples are products such as those supplied by Thermo Cool Corp., Thermacore Inc., etc.

As used herein, the term "interpenetrating polymer network (IPN)" refers to the standard definition according to the International Union of Pure and Applied Chemists (IUPAC), which is "a polymer comprising two or more networks which are at least partially interlaced on a molecular scale but not covalently bonded to each other and cannot be separated unless chemical bonds are broken. *Note:* A mixture of two or more pre-formed polymer networks is not an IPN."

As used herein, the term "semi-interpenetrating polymer network (SIPN)" refers to the standard IUPAC definition, which is "a polymer comprising one or more networks and one or more linear or branched polymer(s) characterized by the penetration on a molecular scale of at least one of the networks by at least some of the linear or branched macromolecules. *Note:* Semi-interpenetrating polymer networks are distinguished from interpenetrating polymer networks because the constituent linear or branched polymers can, in principle, be separated from the constituent polymer network(s) without breaking chemical bonds; they are polymer blends.

As used herein, the term "sequential interpenetrating polymer network" refers to the standard IUPAC definition, which is "interpenetrating polymer network prepared by a process in which the second component network is formed following the formation of the first component network."

As used herein, the term "sequential semi-interpenetrating polymer network" refers to the standard IUPAC definition, which is a "semi-interpenetrating polymer network prepared by a process in which the linear or branched components are formed following the completion of the reactions that lead to the formation of the network(s) or vice versa."

## Multicomponent Functional Polymeric Phase Change Material Compositions

In order to create TMMs with suitable abilities for temperature management, the ability to be coated and cured into a film, and other desirable properties, this disclosure provides several embodiments of multicomponent functional polymeric phase change material compositions. In general, a PCM-TMM in accordance with embodiments of this disclosure may comprise compositions which may include one or more of 1) monomers, polymers, or elastomers, 2) pPCMs, fpPCMs, contained PCMs, and mPCMs, and 3) additives. In order to accomplish certain aspects of the disclosure, the monomers, polymers, or elastomers may comprise low molecular weight liquid monomers or reactive species (e.g., functional polymers) to act as a solvent when the PCM-TMM composition is in a liquid state. These solvents dilute or lower the viscosity of the entire composition, such that it may be coated or cast (e.g., through spraying or spin coating). These monomers or reactive functional polymers may also be referred to as reactive diluents, especially in their liquid forms. In embodiments of the present disclosure, these reactive diluents, when cured, may form either pPCMs or fpPCMs themselves. When these reactive diluents are cured, they may be referred to as a "polymer matrix." An advantage to using a reactive diluent that transforms into pPCM or fpPCM is that no solvent needs to be driven out of the polymer matrix while curing, thus avoiding problems such as bubbling, foaming, and uneven surfaces. An additional advantage is that the reactive diluent becomes a PCM, which itself has temperature management properties.

Figure 1A is a high-level diagram of a PCM-TMM film in accordance with embodiments of the present disclosure. A section of PCM-TMM film 100a is depicted with the solid PCM-TMM composition 101a once it has been cured upon a substrate 102a. The substrate 102a may be any substrate enumerated throughout this disclosure, including an adhesive,

an energy conductive material, a textile, or the surface of an object to be thermally managed.

Figure 1B is a high-level diagram of a section of PCM-TMM film 100b, which is similar to the section of PCM-TMM film 100a depicted in Figure 1A, but is shown as flexible and pliable. The section of PCM-TMM film 100b may be produced in shapes of various sizes, including various lengths, widths, and thicknesses. The solid PCM-TMM composition 101b is disposed on a substrate 102b.

Figure 1C is a high-level diagram of a section of PCM-TMM film 100c wrapped around a cylindrical object 103. A solid PCM-TMM composition 101c is disposed on a substrate 102c. In the embodiment depicted, the substrate 102c may comprise an adhesive, or may have inherent sticky or tacky properties that allows the section of PCM-TMM film 100c to adhere to the cylindrical object 103 and to itself. An attachment gap 104 may exist where the beginning of the film is first applied to the cylindrical object 103. In many embodiments, it may be desirable to fill an attachment gap 104 with some kind of thermal management material, so as not to allow air to exist between the cylindrical object 103 and the PCM-TMM film 100c. In these embodiments, the attachment gap 104 may be filled with a tapered end of the PCM-TMM film 100c, or various thermal management gels or greases that will be described throughout this disclosure.

Figure 2 is a high-level conceptual diagram of how molecules or monomers may be polymerized during the curing process of various embodiments of the present disclosure. Liquid temperature management material (TMM) 210 is depicted containing several PCM particles 211. The PCM particles 211 may comprise any type of PCM described throughout this disclosure, including microencapsulated PCMs (mPCMs), polymeric PCMs (pPCMs), or functional polymeric PCMs (fpPCMs). The liquid TMM 210 is also depicted containing several reactive diluent PCMs 212. The reactive diluent PCMs 212 may comprise any molecules or monomers enumerated throughout the disclosure that may react upon curing to form a polymer or a polymeric PCM. It is to be understood that when the reactive diluent PCM 212 is in its liquid form, it is a molecule or monomer that is not yet a PCM, but becomes a PCM upon curing. In one embodiment, the reactive diluent PCM 212 may comprise a radiation-curable compound with at least one free-radically polymerizable group, wherein the compound can be reacted, upon the radiation curing, to form a polymeric PCM. Examples of such radiation-curable compounds will be discussed later in this disclosure, and may be referred to as a "Component A."

Many types of PCMs are described throughout this disclosure. For clarity, PCMs such as PCM particles 211 will be referred to throughout the disclosure as either "PCM particles," or "particulate PCMs," which are distinct from reactive diluent PCMs (either polymeric or functional polymeric) that exist as a liquid that start as a liquid molecule or monomer (during at least some point during the manufacturing process) and polymerize into a PCM upon curing. That is, "PCM particles" or "particulate PCMs" are, in many embodiments, the most abundant PCMs by weight in a TMM film composition. They may be specifically chosen for their transition temperatures or latent heat storage values, and may comprise over 80% by weight of the TMM film composition. In other words, their inclusion in a composition can be described as "high particulate loading" of a PCM. In contrast, the polymeric or functional polymeric PCMs formed from reactive diluents may, in many embodiments, form a relatively small percentage by weight of the composition. As stated earlier, PCM *particles* may be mPCMs, pPCMs, fpPCMs, or raw PCMs.

Although the reactive diluent PCMs referred to herein are often described as being in a liquid or viscous form, before being cured, it is contemplated that in some embodiments, reactive diluent PCMs may exist in a solid or solidified state at a certain temperature prior to becoming a liquid, which may be advantageous for purposes of mixing or handling. That is, in some embodiments of the compositions and/or methods, reactive diluents may start as solid molecules or monomers, be melted into liquid reactive diluent molecules or monomers, and then cured into solid polymers or PCMs.

Still referring to Figure 2, the liquid TMM 210 is depicted containing several additives 213. The additives 213 may comprise any additives described throughout this disclosure, including fire resistant, energy conductive, waterproofing, corrosion-resistant, or impact resistant additives. The liquid TMM 210 may comprise additional materials that form a reactive diluent/matrix 214. This reactive diluent/matrix 214 may be comprised of one or more radiation-curable compounds having at least one free-radically polymerizable group. In some embodiments, the matrix may be comprised of up to three such radiation-curable compounds, examples of which will be discussed later in this disclosure. Such compounds may be referred to as Components B, C, and D. The reactive diluent/matrix 214 is referred to as such because in its liquid form, it is more accurately characterized as a reactive diluent, but upon curing, it transforms into a solid form, which is more accurately characterized as a matrix; specifically, a polymer matrix. In the liquid TMM 210, the reactive diluent PCM 212 (alternatively referred to as Component A) is a liquid, and the matrix 214 (which may comprise Components B, C, and D), is also a liquid. As a liquid, components A, B, C, and D may be indistinguishable in implementation. However, reactive diluent PCM 212 is depicted in this drawing as a unique component to illustrate that it has the particular property of being polymerizable into a polymeric or functional polymeric PCM. Additives 213 and PCM particles 211 may themselves be in either a liquid or solid form.

Aspects of this disclosure related to methods of curing a liquid TMM such as liquid TMM 210, which will be described in further detail later in this disclosure. A curing process is represented by the arrow at 215, by which the liquid TMM 210 cures into a solid TMM 220.

When a reactive diluent polymerizes into a pPCM or fpPCM, it can form an interpenetrating polymer network (IPN) with surrounding polymers. In various embodiments of the disclosure, the reactive diluent can polymerize and

form an interpenetrating polymer network with one or more other polymers in the polymer matrix, including other PCMs. The reactive diluents (Components A, B, C, and D) may be selected for certain properties that they may have upon polymerizing. For example, a hard and soft segment IPN may be formed by diluting a "hard" or high Tg or high melting point polymer (e.g., polyester, polyurethane, polyepoxide, etc.) with a reactive diluent that forms a "soft," or low Tg or low melting point polymer. One property that may be achieved by forming such an IPN is a degree of elasticity, for example.

When the liquid TMM cures into a solid TMM 220, the reactive diluent PCM 212 cures into a solid PCM 212'. The change is represented by the change of shape from a triangle at 212 to a circle at 212', with the patterns remaining the same. Similarly, the reactive diluent/matrix 214 cures into a solid polymer matrix 214', represented by the change from an amorphous shape to a square, with the patterns remaining the same. In the embodiment depicted, the PCM particles 211 and the additives 213 may not undergo state changes when curing into 211' and 213', respectively.

Various embodiments of the present disclosure comprise elastomers and materials whose properties may be referred to as elastomeric. Figure 3 depicts a high level view of an elastomer, which may be comprised of block copolymers with "hard" (or rigid) segments 301 and "soft" (or flexible) segments 302. The hard segments can combine to provide hardness, rigidity, stiffness, toughness, stability and chemical resistance. The hard segments can be made of high glass transition temperature materials, crystallizable materials where the segments will form stable crystalline domains, or functional crosslinkable materials in which formed crosslinks will hold the domains stable and rigid. The crosslinks may be through hydrogen bonding. The soft segments are generally in an amorphous or rubbery phase and not aligned or interacting with other soft segments. This allows the segments to be flexible, free moving and have good elongation characteristics. The soft segments can be comprised of low glass transition temperature materials, amorphous (non-crystalline) materials, and can generally have low or no crosslinking. The hard or soft segments can be of different or the same chemical makeup. For example, a polypropylene elastomer can consist of syndio or isotactic polypropylene segments allowing for hard crystalline segments compared to the soft segment of randomly or atactic polymerized polypropylene. The segments can have different melting points due to the different phases or crystalline structures. Depending on the soft segment or hard segment concentration, chemistry, molecular weight, etc., the polymer can have a continuous hard phase with a dispersed soft phase, or a continuous soft phase with dispersed hard phase if the hard segments are in low concentration. The morphology developed during polymer or part production, the miscibility of the phases, the size of the dispersed phases and their characteristics strongly affect the final properties of the polymer.

In certain embodiments of the disclosure, the above-mentioned compositions can result in desirable hardness properties suitable for protecting a wrapped object from denting or puncturing by an outside force. For example, the compositions may result in a Shore A hardness of greater than 85 and a Shore D hardness of greater than 25, per ASTM D2240, when the film is wrapped around an object. In some embodiments, the film may need to be wrapped multiple times in order to achieve these hardness properties. Additionally, the above-mentioned compositions may result in desirable elasticity properties suitable for expanding and contracting with a particular object. For example, the compositions may result in a flex modulus of greater than 15 pounds per square inch and a break elongation of greater than 20%.

Figure 4 depicts how an IPN may be formed according to embodiments of the disclosure. A liquid TMM 410 is depicted containing PCM particles 411. In this case, the PCM particles 411 are polymeric PCM particles. The liquid TMM 410 also comprises matrix 414, which, similarly to the matrix 214 of Figure 2, may contain one or more of Components A, B, C, and D. When the liquid TMM 410 is cured, represented by the arrow at 415, the polymeric PCM particles 411 form an IPN with polymers 414A, which is represented by the polymeric PCM particles 411 being substantially aligned with the polymers 414A. To depict the idea that the polymers 414A are formed from the matrix 414, the polymers 414A are shaded similarly to the matrix 414.

Figure 5A depicts an IPN 500 as known in the art and described in the "Definitions" section of this disclosure. A first polymer network 501 is represented by solid lines. The first polymer network 501 is crosslinked at points 502. A second polymer network 503 is represented by dashed lines and is crosslinked at points 504. In embodiments of the present disclosure, IPNs can be formed when the first polymer network 501 is a particulate pPCM (such as particulate pPCM 411 in Figure 4) and the second polymer network is a polymer of a reactive diluent/matrix (such as reactive diluent/matrix 414A of Figure 4). The first and second polymer networks 501 and 502 are IPNs because they are at least partially interlaced in a molecular scale but not covalently bonded to each other. In the embodiment depicted in Figure 5A, the first and second polymers 501 and 502 are crosslinked, but in other embodiments, one or both may not be crosslinked. It is contemplated that additional polymers may comprise an IPN in embodiments of the disclosure. For example, if a reactive diluent/matrix is comprised of Components A, B, C, and D, the resulting IPN may contain five polymer networks-the polymeric particulate PCM and the polymer networks of Components A, B, C, and D. Additionally, if Component A is included, one of the polymer networks in the IPN may be a pPCM or fpPCM.

Figure 5B depicts a semi-interpenetrating network (SIPN) 505 as known in the art and described in the "Definitions" section of this disclosure. A polymer network 506 is represented by solid lines and cross-linked at points 507. Linear or branched polymers 508 are represented by dotted lines 508. The polymer network 506 and linear or branched polymers 508 form an SIPN because the polymer network 506 is penetrated on a molecular scale by the linear or branched polymers. In certain embodiments of the disclosure, additional linear or branched polymers may comprise the

SIPN 505. For example, if a reactive diluent/matrix is comprised of Components A, B, C, and D, the resulting SIPN may contain five polymers-the polymeric particulate PCM and the linear or branched polymers of Components A, B, C, and D. Additionally, if Component A is included, one of the linear or branched polymers in the SIPN may be a pPCM or fpPCM.

In some embodiments, the reactive diluent/matrix may, in its solid matrix form, have elastomeric properties. In such embodiments, the matrix may comprise polyolefin copolymers such as copolymers of polyethylene with any $C_3$-$C_{30}$ $\alpha$-olefin, or vinyl acetate, other vinyl monomers such as styrene or its analogues, acrylate or methacrylate monomers, vinyl ether monomers, vinyl ester monomers, acrylonitriles, rubbers and copolymers made from isoprene, butyl, etc., or combinations, blends, mixtures thereof. The polymers or copolymers can be random or block copolymers.

The elastomeric matrix can comprise a polymer elastomer made from polyesters, silicon rubbers or polyurethanes. Non-inclusive examples of these are Hytrel® polyesters from DuPont®, Kraton® SEBS, SEPS, SBS or SIS block copolymers, Septon™ and other elastomers by Kuraray Co., Spandex® type polyurethanes and RTV or LTV type silicon rubbers. To further improve the elastomeric properties of the polymer matrix, the matrix can be crosslinked for improved elastomeric properties with an average of 0.05-1.0 crosslinks per polymeric chain.

Because of the need for close contact between the film and the outer surface of the wrapped object, the elastomeric polymer matrix can have good thermal elasticity properties characterized by low coefficient of thermal expansion (CTE) and high fatigue or creep resistance. In some embodiments, these values can be a CTE of <200 ppm (per ASTM E228) and no fatigue failure (per ASTM D7791, Method A, 2 Hz, 2 mPa, Stress, sinusoidal wave.) Such properties can minimize the likelihood of air gaps forming or expanding between a film and a wrapped objects as the wrapped object expands or contracts with temperature changes.

In certain embodiments of the disclosure, a gel layer may be used to fill any air gaps between a film and a wrapped object. The gel may be characterized as a grease or a wax in certain embodiments. The gel may be a lower molecular weight version of the same polymer matrix, TMM, and/ or PCM blends that comprise embodiments of the film. In comparison to the properties of the film, the gel may have a higher percentage of conductivity additives (i.e., graphene, graphite, carbon fibers, metals, etc.) to improve the thermal transport.

In certain applications, including industrial, automotive, and medical applications discussed in this disclosure, impact resistance properties may be desirable in a film. In some embodiments, the film can have hardness and stiffness properties that enable protection from impact. Additionally, the hardness and stiffness properties can prevent damage during shipping. Therefore, certain embodiments of the film may have a Shore A hardness of >85 and a Shore D hardness >25 (per ASTM D2240). Additionally, a wrapped segment of film may have a flex modulus of >15 psi (per ASTM D790) and a break elongation >20% (per ASTM D638). The foregoing properties of the film as described herein may be accomplished by one or more of the chemical structures described throughout this disclosure, including impact-resistant additives. In some embodiments, hardness and stiffness properties described herein may be accomplished by layering the film multiple times.

Figures 6A-6C show various embodiments of a temperature management material in various arrangements with PCMs that may be used to form embodiments of the disclosure in matrix forms and layered forms. In Figure 6A shows a uniform mixture 600 of a PCM 602 and a polymeric matrix TMM 604. In the example of Figure 6A the PCM 602 is a microencapsulated PCM (mPCM) but can also be a raw or otherwise unencapsulated PCM such as a pPCM (polymeric PCM) or a fpPCM (functional polymeric PCM). However the phase change material is incorporated into the mixture 600, in the example of Figure 6A, the mixture 600 is a uniform substance with some level of homogeneity to the material. In some embodiments, the polymeric matrix TMM 604 may comprise a pPCM or fpPCM itself, dispersed throughout the polymeric TMM 604 itself. In other embodiments, the polymeric matrix TMM 604 may not comprise and pPCM or fpPCM itself. There are no specific layers within the example of Figure 6A. It is contemplated that a film made in accordance with this disclosure may be substantially homogenous as depicted in Figure 6A.

With reference to Figure 6B, a layered composition 620 is shown that includes several layers. The layered composition may form a part of a film such as the film 100 depicted in Figure 1. For example, a one of the layers depicted in Figure 6B may be the topmost or bottommost layer of a film. Alternatively, all the layers depicted in Figure 6B could be disposed completely within a film, having no contact with a wrapped object, a substrate, or the ambient environment. It is contemplated that a film may comprise more than three layers. In Figure 6B, a layer 622 may be formed from a liquid composition, cured from liquid to solid containing a particulate phase change material, an additive, and a polymer matrix. The polymer matrix may be formed from one or more of components B, C, and D as described later in this disclosure.

A layer 624 may be formed from a different liquid composition as the layer 622. It may contain a different particulate phase change material than the layer 622. The layer 624 may be formed from one or more additives and polymer matrix. The polymer matrix may be formed from different components, such as Components A and B. Component A may be a reactive diluent that polymerizes into a pPCM upon curing. Therefore, the layer 624 may contain a PCM, one or more additives, and a polymer matrix that itself comprises pPCM. That is, the pPCM polymer matrix may surround another PCM. A layer 626 may be formed in substantially the same way as layers 622 and 624, but may have a different phase change material, different additives, and a polymer matrix comprised of different components, such as Components

A, C, and D, for example.

It is contemplated that each of layer 622, 624, and 626 can utilize any of the PCM materials discussed herein in various combinations that may be necessary to fit a specific temperature control scenario. For example, certain applications may generate higher levels of heat, or have a steeper heating curve profile and thus warrant the use of PCMs that have higher latent heat values or higher PCM loading requirements in order to effectively manage the temperature changes that occur in those applications than others. Other applications may have more subtle temperature change profiles and may not demand PCMs with such large latent heat values and can thus utilize lower loading amounts. Other applications may require very low transition temperatures, such as when films are used in protecting from cold or managing cold.

With reference to Figure 6C, another layered composition 640 is shown that also includes several layers. Similarly to layered composition 630, a layer 642 may be formed from a particulate phase change material, one or more additives, and a polymer matrix. A layer 644 is formed from a second particulate phase change material, one or more additives, and a polymer matrix. The layer 644 may have the same or different additives and/or polymer matrix as the layer 642. A layer 646 may be formed in substantially the same way as layers 642 and 646, (i.e., with a particulate PCM, one or more additives, and a polymer matrix,) In the embodiment shown, at least one of the particulate PCM, additives, and polymer matrix may be different from the ones used in layers 642 and 644. As with the example of Figure 6B, the layers 642, 644, and 646 may each contain any of an mPCM, pPCM, or fpPCM as particulate PCMs, or the layers 644 and 646 may contain combinations or blends of one or more of these types of phase change materials. In addition, each of layer 644 and 646 can utilize any of the PCM materials discussed herein in various combinations that may be necessary to fit a specific temperature control scenario. In the embodiment and example of Figure 6C, layer 644 is shown utilizing microcapsules 645. These microcapsules may be within a polymeric binder 647 (with or without its own PCM and latent heat qualities).

Figures 7-15 will be described in further detail later in this disclosure.

Figures 16A-16C show other layering options that may be utilized in constructing a film for temperature management and heat dissipation. As exemplified in Figures 16A-16C, any combination of these different layers are also possible. In Figures 16A-16C each of L1 through L8 represent different layers within a film or different regions within a discrete portion of a film It should be understood that many different combinations of these layers are possible and it is not intended to limit the disclosure to any of the physical structures depicted by Figures 16A-16C. These are merely representative of several of the possibilities.

Figures 17-29 show various applications of films in accordance with the present disclosure in several contexts.

Figure 17 depicts industrial pipes and holding tanks that may be thermally managed by application of a film. A large holding tank 1701 is depicted as being wrapped by a large section of PCM-TMM film 1702. As seen in the figure, the large holding tank 1701 is shaped in a non-uniform manner, but the pliability of the large section of PCM-TMM film 1702 allows it to conform to the shape of the large holding tank 1701. Near the large holding tank 1701 is a large pipe 1703, which is wrapped by a pipe-wrapping PCM-TMM film 1704. It is contemplated that the large section of PCM-TMM film 1702 and the pipe-wrapping PCM-TMM film 1704 may have similar or different compositions. For example, if the heat ranges emitted by the large holding tank 1701 and the large pipe 1703 were very different, the PCMs used in their corresponding films (1702 and 1704) could have very different transition temperatures and/or latent heat storage values. Alternatively, if the heat ranges emitted were similar, the films 1702 and 1704 might have the same composition. If the user of the films 1702 and 1704 wanted slightly different temperature management properties for the tank 1701 and the pipe 1703 though the films 1702 and 1704 had different compositions, the user could wrap the films 1702 and 1704 in different thicknesses than each other. Also shown in Figure 17 are several smaller pipes, such as small pipe 1705. Such a pipe may require less PCM-TMM film to manage its heat dissipation. Additionally, as seen in the figure, there is very little room between the small pipe 1705 and the wall of the room. It is contemplated that users may be limited in the space they have on one or more sides of a particular object. Therefore, an advantage of a PCM-TMM film may be that one side of an object may be wrapped or layered more than another side.

Figure 18 shows one way that a pipe 1800 (in any kind of setting) may be wrapped by a PCM-TMM film 1801, which is in a partially overlapping manner as the PCM-TMM film 1801 progresses up a longitudinal axis of the pipe 1802. This method may be referred to as "progressive" wrapping. Progressive wrapping may be used instead of, or in addition to, the method as depicted in Figure 1C, wherein a section of PCM-TMM film is the entire length of the pipe to be wrapped, and the wrapping is repeated in the same position multiple times with respect to the longitudinal axis. This type of wrapping may be referred to as "stationary" wrapping. It is contemplated that many applications of PCM-TMM film in accordance with embodiments of this disclosure may use either kind of wrapping, as well as any other suitable method of wrapping or layering.

Various commercial, residential, and industrial building components may be wrapped with PCM-TMM film. Figure 19 shows a square air duct 1900 that may be wrapped with a PCM-TMM film 1901. Figure 20 shows electrical wires 2000 that may be wrapped with PCM-TMM film 2001. Figure 20A shows a water heater 2010 wrapped in a PCM-TMM film 2011. It is contemplated that many wrapped objects, such as a water heater, may have various fixtures that

extend out from the body of the object and need to be exposed, rather than wrapped. In many embodiments, PCM-TMM film may be easily cut with a knife or blade in order to accommodate fixtures of various sizes.

Figures 21A and 21B show different variations of hydraulic pistons 2100 wrapped in PCM-TMM films 2101. Figure 22 shows a hydraulic pump 2200 wrapped in a PCM-TMM film 2201. It is contemplated that for some objects, it may be beneficial to coat the object itself with a liquid PCM-TMM composition, using the object itself as the substrate, and then drying and/or curing the composition onto the object. In this manner, oddly-shaped objects, such as the hydraulic pump 2200, could be coated with a PCM-TMM film that maintains close contact with all parts of the object. Objects made out of materials that could withstand harsh temperatures or other conditions required for curing may be good candidates for being substrates themselves.

Figures 23-27 show various applications of PCM-TMM films being used in contact with human skin. An aspect of this disclosure is that PCM-TMM films may be non-toxic and easy to handle. Figure 23 shows an ankle 2300 wrapped in a PCM-TMM film 2301 implemented as athletic tape. Figure 24 shows a PCM-TMM film implemented as a cosmetic patch 2401. The cosmetic patch 2401 may provide cooling to the skin 2400, and may be infused with cosmetic or dermatological compositions for various benefits. Figure 25 shows a leg 2500 wrapped in a PCM-TMM film implemented as a bandage 2501. Figure 26 shows a PCM-TMM film 2601 implemented as a transdermal patch 2601 and applied to an arm 2600. The transdermal patch 2601 may be infused with medication or first aid treatments. Figure 27 shows a PCM-TMM implemented as a wrap 2701 that forms a cast around a broken bone 2700.

Figures 28A-28D show a variety of ways a PCM-TMM film may be used to thermally manage solar panels. Solar panels vary in composition, but most are comprised of multiple layers, each of which has a function related to one or more of conduction, electron transport, coating, cooling, or solar energy capture. Depending on the particular composition of a solar panel, a layer of PCM-TMM may be used to manage the temperature of the solar panel in various places. In the first solar panel 2800 of Figure 28A the PCM-TMM film may comprise a bottom layer 2801. In the second solar panel 2802, of Figure 28B, PCM-TMM film may comprise one of the lower layers 2803. In the third solar panel 2804, of Figure 28C, the PCM-TMM film may comprise one of the upper layers 2805. In the fourth solar panel in Figure 28D, in which the cells are comprised of a flexible film 2806 themselves, the PCM-TMM film may form a backing 2807, which may adhere to the solar panel flexible film 2806 by means of an adhesive.

Yet another embodiment of the disclosure may be achieved as depicted in Figure 29. Figure 29 shows a battery cell 2915. A battery sleeve 2910 in the embodiment depicted can be applied to the cell 2915 as a film or tape wrapped around the cell 2915 multiple times. A layer of the film or tape is depicted at 2908. An adhesive 2907 may be used to initially attach the first layer of film or tape to the outside of the cell 2905. The adhesive may comprise a substrate onto which a liquid TMM is coated and cured to form the solid film. The adhesive 2907 may be comprised of lower molecular weight versions of the TMM or PCM of the film or tape itself. The adhesive 2907 may be a backing to the film or tape itself, such that each wrapping of the film or tape around the circumference of the cell 2915 results in a layer of adhesive and the polymer matrix. The adhesive may have similar thermal management properties as the TMM film itself. It is contemplated that certain compositions of the TMM film or tape may have intrinsic "sticky" or "tacky" properties, such that no additional adhesive material is required to layer the film or tape onto itself. The film or tape may also have "shrink wrap" properties, such that the film or tape is supplied in an oriented form (polymer chains are aligned) and when heated the film or tape will shrink (due to polymer chains randomizing), adhere and fill any air gaps either within the tape/film layers or at the battery surface. An advantage to the forming a sleeve 2910 out of film is that the thickness of the sleeve 2910 can be adjusted for the particular application. That is, higher-heat generating applications can use more film to manage the thermal properties of the cells used therein. Device manufacturers or end users could customize the thermal management as required.

Figure 30 shows a computer server 3000 with PCM-TMM film 3001 attached to the side. Computer servers and other electronics are known to produce heat and require cooling for safe operation and optimal performance. Servers of various sizes and shapes may utilize one or more layers of PCM-TMM film 3001 in order to dissipate heat. The transitions temperatures and latent heat values of PCMs in various embodiments can be in ranges necessary to dissipate heat from server electronics. An advantage to utilizing a PCM-TMM film 3001 as depicted in FIG. 30 is that heat control can be achieved in tight spaces. Many computer servers are located next to multiple other servers, and there is often a need for high levels of temperature management in small spaces.

The various applications of PCM-TMM film described and depicted in Figures 17-30 and elsewhere in this disclosure illustrate that many types of objects may be thermally managed. It may be appreciated by those skilled in the art that the various objects depicted produce heat in particular ranges, and it may be understood that PCM-TMM films may have thermal management properties specifically adapted to those particular temperature ranges. However, the depictions in Figures 17-30 are exemplary only; PCM-TMM films in accordance with this disclosure may be made and utilized for temperatures far outside the ranges associated with the depicted objects.

Use of PCMs and other Thermal Management Materials

The sections above describe various compositions and other materials that may be used to create temperature control and thermal management films, tapes, sheets, and other aspects of the present disclosure. However, by referring to any of the specific structural embodiments or any of the precise and specific chemical compositions (above or further in this disclosure), it is not intended to limit the scope of the claims to any one in particular. To the contrary, the specification is arranged such that one of skill in the art could combine one or more of the films, tapes, or sheets described herein with one or more of the chemical compositions described herein in order to create a configuration that worked for a specific purpose.

While the above description of phase change technology and the different types of chemicals and other materials useful in temperature regulation is generally applicable and relevant to aspects and embodiments of the present disclosure, there are other aspects and unique features that find particular relevance in manufacturing films, tape, and sheet embodiments.

Thermal Management Materials (TMM) for Temperature Control

PCMs are very advantageous for use as thermal management materials in a wide variety of applications in that the high crystallinity allows for a combination of good thermal conductivity, high latent heat capacity and energy absorption all leading to improved heat management, lower heat buildup, and a lower rate of degradation of objects and materials to be wrapped.

Air is a very poor thermal conductor and therefore air spaces or air gaps anywhere within the TMM is not preferred. Air gaps such as between the wrapped object and the thermal management materials, cracks or voids within the TMM, voids or gaps between particles and the TMM composite or matrix material, etc. are all problematic for good thermal conductivity. For example, wrapping a film around a pipe requires that two solid surfaces be brought together into intimate contact. Unfortunately, no matter how well prepared, solid surfaces are never really flat or smooth enough to permit intimate contact. All surfaces have a certain roughness due to microscopic hills and valleys. Superimposed on this surface roughness is a macroscopic non-planarity in the form of a concave, convex or twisted shape. As two such surfaces are brought together, only the hills of the surfaces come into physical contact. The valleys are separated and form air-filled gaps. Since air is a poor conductor of heat, it should be replaced by a more conductive material to increase the joint conductivity and thus improve heat flow across the thermal interface. Adding to the difficulty of creating contact between solid surfaces, heat-generating objects often expand and contract themselves. For example, a metal pipe transporting hot water may expand slightly when the hot water is flowing through it, and may contract once it cools. This cycle of expansion and contraction will likely be repeated frequently. It follows that any material in close contact with the pipe will also go through multiple cycles of expansion and contraction, and any materials that do not have good elastomeric properties might lose contact over time. The thermal management materials should have good rheological characteristics and surface wetting to have good "gap-filling" properties, i.e. the ability to flow, wetout and fill gaps, crevices, cracks, etc. to reduce air gaps and improve thermal movement. These TMM flow properties can be formulated into the material through the use of additives or designed into the TMM molecules. TMMs can also be used in a liquid form such as a gel or a grease to maintain contact between a solid TMM and the wrapped object.

In some embodiments, the TMM film should also have good adhesive, tack or bonding properties to prevent the loosening of contact between the TMM and the wrapped object if the object is dropped, damaged, impacted or exposed to high or low temperatures.

In some embodiments, TMMs should also have good impact and puncture resistance. For example, in applications where previous wrapping, coating, or mechanical component has been used for the protection of an object, it may be desirable to incorporate impact and puncture resistance into the TMM film.

As described in general terms above and in the definition section, the term "phase change material" refers to a material that has the capability of absorbing or releasing heat to adjust heat transfer at or within a temperature stabilizing range. A temperature stabilizing range can include a specific transition temperature or a range of transition temperatures. In some instances, a phase change material can be capable of inhibiting heat transfer during a period of time when the phase change material is absorbing or releasing heat, typically as the phase change material undergoes a transition between two states. This action is typically transient and will occur until a latent heat of the phase change material is absorbed or released during a heating or cooling process. Heat can be stored or removed from a phase change material, and the phase change material typically can be effectively recharged by a source of heat or cold. For certain implementations, a phase change material can be a mixture of two or more materials. By selecting two or more different materials and forming a mixture, a temperature stabilizing range can be adjusted for any desired application. The resulting mixture can exhibit two or more different transition temperatures or a single modified transition temperature when incorporated in the articles described herein.

PCMs for Optimal Thermal Management Properties

Throughout the remainder of this disclosure, many types of PCMs will be described in great detail. The PCMs described in this section include a wide range that may be used specifically for their thermal management capabilities. Others described in this section include functional polymeric PCMs (fpPCMs) that may be used specifically for their ability to bind to a substrate. In a separate section, later in this disclosure, certain pPCMs and fpPCMs will be discussed in relation to their ability to transform from reactive monomers that form a solvent or diluent into pPCMs and fpPCMs upon curing.

PCMs for optimal thermal management properties can include various organic and inorganic substances. Organic PCMs may be preferred for the embodiments disclosed herein. Examples of phase change materials include hydrocarbons (e.g., straight-chain alkanes or paraffinic hydrocarbons, branchedchain alkanes, unsaturated hydrocarbons, halogenated hydrocarbons, and alicyclic hydrocarbons), alkanes, alkenes, alkynes, arenes, hydrated salts (e.g., calcium chloride hexahydrate, calcium bromide hexahydrate, magnesium nitrate hexahydrate, lithium nitrate trihydrate, potassium fluoride tetrahydrate, ammonium alum, magnesium chloride hexahydrate, sodium carbonate decahydrate, disodium phosphate dodecahydrate, sodium sulfate decahydrate, and sodium acetate trihydrate), waxes, oils, water, fatty acids (caproic acid, caprylic acid, lauric acid, myristic acid, palmitic acid, stearic acid, arachidic acid, behenic acid, lignoceric acid and cerotic acid, etc.), fatty acid esters (methyl caprylate, methyl caprate, methyl laurate, methyl myristate, methyl palmitate, methyl stearate, methyl arachidate, methyl behenate, methyl lignocerate, etc.), fatty alcohols (capryl alcohol, lauryl alcohol, myristyl alcohol, cetyl alcohol, stearyl alcohol, arachidyl alcohol, behenyl alcohol, lignoceryl alcohol, ceryl alcohol, montanyl alcohol, myricyl alcohol, and geddyl alcohol, etc.), dibasic acids, dibasic esters, 1-halides, primary alcohols, secondary alcohols, tertiary alcohols, aromatic compounds, clathrates, semi-clathrates, gas clathrates, anhydrides (e.g., stearic anhydride), ethylene carbonate, methyl esters, polyhydric alcohols (e.g., 2,2-dimethyl-1,3-propanediol, 2-hydroxymethyl-2-methyl-1,3-propanediol, ethylene glycol, polyethylene glycol, pentaerythritol, dipentaerythritol, pentaglycerine, tetramethylol ethane, neopentyl glycol, tetramethylol propane, 2-amino-2-methyl-1,3-propanediol, monoaminopentaerythritol, diaminopentaerythritol, and tris(hydroxymethyl)acetic acid), sugar alcohols (erythritol, D-mannitol, galactitol, xylitol, D-sorbitol), polymers (e.g., polyethylene, polyethylene glycol, polyethylene oxide, polypropylene, polypropylene glycol, polytetramethylene glycol, polypropylene malonate, polyneopentyl glycol sebacate, polypentane glutarate, polyvinyl myristate, polyvinyl stearate, polyvinyl laurate, polyhexadecyl methacrylate, polyoctadecyl methacrylate, polyesters produced by polycondensation of glycols (or their derivatives) with diacids (or their derivatives), and copolymers, such as polyacrylate or poly(meth)acrylate with alkyl hydrocarbon side chain or with polyethylene glycol side chain and copolymers including polyethylene, polyethylene glycol, polyethylene oxide, polypropylene, polypropylene glycol, or polytetramethylene glycol), metals, and mixtures thereof. Any combination of natural alcohols, natural fatty acids, sugars, celluloses and natural glycols can be combined to yield PCMs. General formulas such as the following, where m or n can be 0-100:

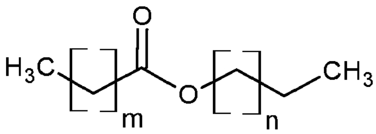

Polymerized alcohols such as polyvinyl alcohol, polyglycerols (mol. wt. of 100-10,000) or multifunctional alcohols esterified with various fatty acids.

Paraffinic PCMs may be a paraffinic hydrocarbons, that is, hydrocarbons represented by the formula $C_nH_{n+2}$, where n can range from about 10 to about 44 carbon atoms. PCMs useful in the disclosure include paraffinic hydrocarbons having 13 to 50 carbon atoms. For example, the melting point of a homologous series of paraffin hydrocarbons is directly related to the number of carbon atoms as shown in the following table:

| Paraffinic Hydrocarbon | No. of Carbon Atoms | Melting Point (° C.) |
|---|---|---|
| n-Octacosane | 28 | 61.4 |
| n-Heptacosane | 27 | 59.0 |
| n-Hexacosane | 26 | 56.4 |
| n-Pentacosane | 25 | 53.7 |
| n-Tetracosane | 24 | 50.9 |
| n-Tricosane | 23 | 47.6 |
| n-Docosane | 22 | 44.4 |

(continued)

| Paraffinic Hydrocarbon | No. of Carbon Atoms | Melting Point (° C.) |
|---|---|---|
| n-Heneicosane | 21 | 40.5 |
| n-Eicosane | 20 | 36.8 |
| n-Nonadecane | 19 | 32.1 |
| n-Octadecane | 18 | 28.2 |
| n-Heptadecane | 17 | 22.0 |
| n-Hexadecane | 16 | 18.2 |
| n-Pentadecane | 15 | 10.0 |
| n-Tetradecane | 14 | 5.9 |
| n-Tridecane | 13 | -5.5 |

**Long Chain Group Containing Monomers**

| R= | Monomers |
|---|---|
| Long chain n-alkyl crystallizible segments | $CH_2$=CR'$CO_2$($CH_2$)$_n$$CH_3$ R' = $CH_3$ or H, n =10-25<br>Long chain n-alkyl acrylates or methacrylates such as stearyl acrylate or stearyl methacrylate,<br>$CH_2$=CH-O-($CH_2$)$_n$$CH_3$, n =10-25<br>Long chain n-alkyl vinyl ethers such as stearyl vinyl ether,<br>$CH_2$=CH-O-CO-($CH_2$)$_n$$CH_3$, n =10-25,<br>Long chain n-alkyl vinyl esters such as vinyl stearate,<br>$CH_2$=C-CO-($CH_2$)$_n$$CH_3$, n =10-25,<br>Long chain n-alkyl vinyl ketoness,<br>$CH_2$=CH-($CH_2$)$_n$$CH_3$, n =4-25<br>Long chain n-alkyl olefins such as undecene,<br>or any other long chain n-alkyl containing unsaturated polvmerizable monomer |
| Long chain crystallizible glycol segments | $CH_2$=CR'$CO_2$($CH_2$$CH_2$O)$_n$OX R' = $CH_3$ or H, n = 1-1,000, X = $CH_3$ or H Glycol based acrylates or methacrylates such as polyethyleneglycol methacrylate, polyethyleneglycol acrylate,<br>$CH_2$=CH-($CH_2$)$_m$-O-(($CH_2$)$_n$O)$_2$OX m=0-4, n=1-10, z=1-1000, X = $CH_3$ or H Glycol based vinyl ethers such as polyethyleneglycol monovinyl ether<br>$CH_2$=CH-O-CO-(($CH_2$)$_n$O)$_z$OX n=1-10, z=1-1000, X = $CH_3$ or H glycol based vinyl esters such as polyethyleneglycol monovinyl ester<br>or any unsaturated polymerizable hydroxyl functional monomer |

In addition other crystallizable sections of pPCMs are contemplated, including radicals of fatty acids, radicals of long-chain dicarboxylic acids, radicals of fatty alcohols, radicals of dialcohols, polyester-polycarboxylic or as previously described.

While each of the fpPCM molecules carries at least one reactive function, large fpPCM molecules may carry multiple reactive functions. According to an embodiment an fpPCM carries at least one reactive function per 1,000,000 Daltons of the molecular weight and in some embodiments, two reactive functions.

In various embodiments, the functions are shown along the backbone, but that is only one option. As indicated above, the functions could also be placed at the end(s) of the backbone, on the side chains and any combination of those. Each fpPCM may have a single or multiple reactive functions. fpPCM may also carry multiple reactive functions of a similar chemical nature or a combination of reactive functions of different chemical nature.

As indicated, the reactive function of the fpPCM should be capable of forming covalent or electrovalent bonds with various substrates that may form a conductive or adhesive backing. Substrates can include materials that comprise the surface of an object to be thermally managed. Examples of reactive functions capable of forming covalent bonds are acid anhydride groups, amino groups, N-substituted amino groups and their salts, amide groups, imine groups, imide groups, azide groups, azo groups, amine-formaldehyde groups, carbonyl groups, carboxy groups and their salts, cyclohexyl epoxy groups, epoxy groups, glycidyl groups, hydroxy groups, isocyanate groups, cyanate groups urea groups, aldehyde groups, ketone groups, ester groups, ether groups, alkenyl groups, alkynyl groups, thiol groups, disulfide

groups, silyl or silane groups, halogenated leaving groups, peroxide groups, salt groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof. fpPCMs capable of forming covalent bonds are disclosed in commonly assigned US Patent No. 8,404,341, the teaching of which is incorporated herein by reference in its entirety. Examples of reactive functions capable of forming electrovalent bonds are acid functions, basic functions, positively charged complexes and negatively charged complexes. fpPCM capable of forming electrovalent bonds such as disclosed in commonly assigned US Patent No. 8,221,910, the teaching of which is incorporated herein by reference in its entirety. For example, the following are examples of suitable reactive functional groups:

According to one embodiment, the fpPCM may carry reactive functions as its end group or groups. Examples of such fpPCMs are $\alpha,\omega$-diglycidyl polyesters, $\alpha,\omega$-diglycidyl ethers, $\alpha,\omega$-diisocyanates, $\alpha,\omega$-diureas, $\alpha,\omega$-dialkenes, $\alpha$-glycidyl polyesters, $\alpha$-glycidyl ethers, $\alpha$-ureas and $\alpha$-isocyanates. (See e.g. the structures depicted in Figures 7-10)

Hydrocarbons--Functional groups that vary based upon the number and order of $\pi$ bonds impart different chemistry and polarity. Each listing below contains C--H bonds, but each one differs in type (and scope) of reactivity.

| Chemical class | Group | Formula | Structural Formula |
|---|---|---|---|
| Alkene | Alkenyl | $R_2C=CR_2$ | 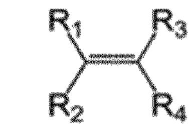 |
| Alkyne | Alkynyl | RC=CR' | 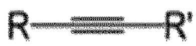 |
| Benzene derivative | Phenyl | $RC_6H_5$ RPh | 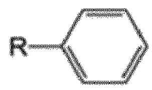 |
| Toluene derivative | Benzyl | $RCH_2C_6H_5$ RBn | 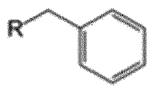 |

Groups containing halogens--Haloalkanes are a class of molecule that is defined by a carbonhalogen bond. This bond can be relatively weak (in the case of an iodoalkane) or quite stable (as in the case of a fluoroalkane). In general, with the exception of fluorinated compounds, haloalkanes readily undergo nucleophilic substitution reactions or elimination reactions. The substitution on the carbon, the acidity of an adjacent proton, the solvent conditions, etc. all can influence the outcome of the reactivity.

| Chemical class | Group | Formula | Structural Formula |
|---|---|---|---|
| haloalkane | halo | RX | R-X |
| fluoroalkane | fluoro | RF | R-F |
| chloroalkane | chloro | RCl | R-Cl |
| bromoalkane | bromo | RBr | R-Br |
| iodoalkane | iodo | RI | R-1 |

Groups containing oxygen-- Compounds that contain C-O bonds each possess differing reactivity based upon the location and hybridization of the C-O bond, owing to the electron-withdrawing effect of $sp^2$ hybridized oxygen and the donating effects of $sp^3$ hybridized oxygen.

| Chemical class | Group | Formula | Structural Formula |
|---|---|---|---|
| Acyl halide | Haloformyl | RCOX | 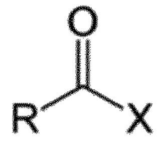 |
| Alcohol | Hydroxyl | ROH | 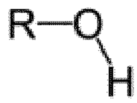 |
| Ketone | Carbonyl | RCOR' | 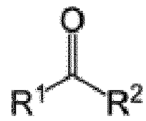 |
| Aldehyde | Aldehyde | RCHO | 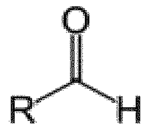 |
| Carbonate | Carbonate ester | ROCOOR | 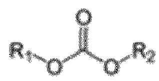 |
| Carboxylate | Carboxylate | $RCOO^-$ | 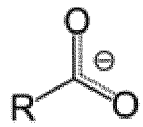 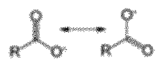 |
| Carboxylic acid | Carboxyl | RCOOH | 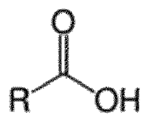 |
| Ether | Ether | ROR' | 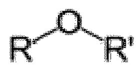 |
| Ester | Ester | RCOOR' | 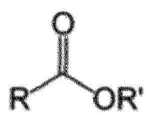 |
| Hydroperoxide | Hydroperoxy | ROOH | 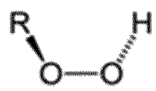 |
| Peroxide | Peroxy | ROOR | 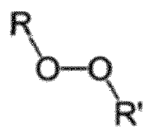 |

Groups containing nitrogen-- Compounds that contain Nitrogen in this category may contain C-O bonds, such as amides.

| Chemical class | Group | Formula | Structural Formula |
|---|---|---|---|
| Amide | Carboxamide | $RCONR_2$ | 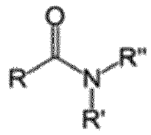 |
| Amines | Primary amine | $RNH_2$ | 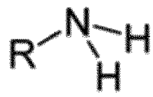 |
| | Secondary amine | $R_2NH$ | 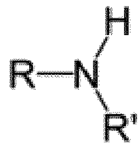 |
| | Tertiary amine | $R_3N$ | 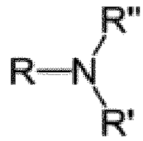 |
| | 4° ammonium ion | $R_4N^+$ | 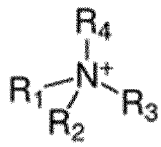 |
| Imine | Primary ketimine | RC(=NH)R’ | 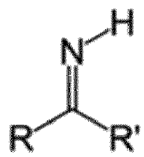 |
| | Secondary ketimine | RC(=NR)R’ | 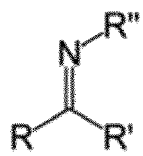 |
| | Primary aldimine | RC(=NH)H | 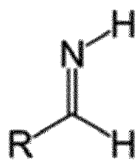 |
| | Secondary aldimine | RC(=NR’)H | 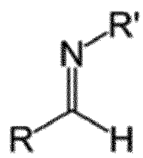 |

(continued)

| Chemical class | Group | Formula | Structural Formula |
|---|---|---|---|
| Imide | Imide | RC(=O)NC(=O)R' | 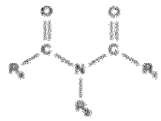 |
| Azide | Azide | $RN_3$ | 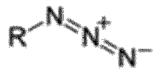 |
| Azo compound | Azo (Diimide) | $RN_2R'$ | 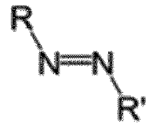 |
| Cyanates | Cyanate | ROCN | 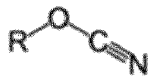 |
| | Isocyanide | RNC | 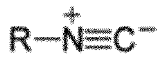 |
| Isocyanates | Isocyanate | RNCO | 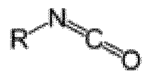 |
| | Isothiocyanate | RNCS | 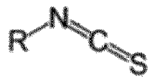 |
| Nitrate | Nitrate | $RONO_2$ | 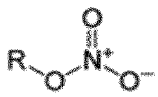 |
| Nitrile | Nitrile | RCN | 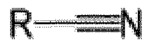 |
| Nitrite | Nitrosooxy | RONO | 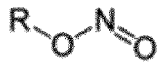 |
| Nitro compound | Nitro | $RNO_2$ | 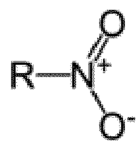 |
| Nitroso compound | Nitroso | RNO | 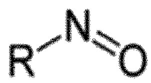 |
| Pyridine derivative | Pyridyl | $RC_5H_4N$ | 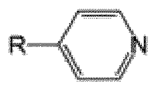 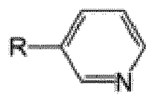 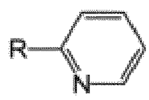 |

Groups containing phosphorus and sulfur-- Compounds that contain sulfur and phosphorus exhibit unique chemistry due to their varying polarity and ability to form more bonds than nitrogen and oxygen, their lighter analogues on the periodic table.

| Chemical class | Group | Formula | Structural Formula |
|---|---|---|---|
| Phosphine | Phosphino | $R_3P$ | 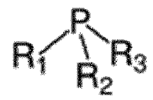 |
| Phosphodiester | Phosphate | $HOPO(OR)_2$ | 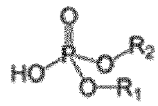 |
| Phosphonic acid | Phosphono | $RP(=O)(OH)_2$ | 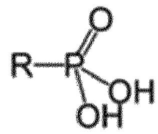 |
| Phosphate | Phosphate | $ROP(=O)(OH)_2$ | 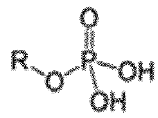 |
| Sulfide or thioether | | RSR’ | 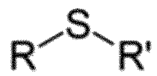 |
| Sulfone | Sulfonyl | $RSO_2R'$ | 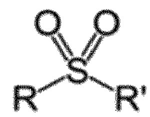 |
| Sulfonic acid | Sulfo | $RSO_3H$ | 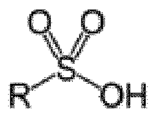 |
| Sulfoxide | Sulfinyl | RSOR’ | 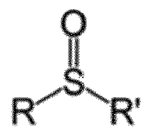 |
| Thiol | Sulfhydryl | RSH | 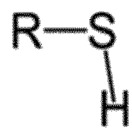 |
| Thiocyanate | Thiocyanate | RSCN | 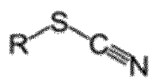 |
| Disulfide | Disulfide | RSSR’ | 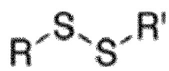 |

Other chemical classes include Organosilanes, Siloxides, Silyl halides, Silyl hydrides, Hydrosilylation, Silenes, Siloles, and Hypercoordinated silicon.

According to another embodiment, the functional polymeric phase change material may also be chemically bound to the substrate. Binding may be one of covalent binding, electrovalent binding, direct binding, or binding via a

connecting compound. According to another embodiment, binding is such as the one resulting from a reaction between a reactive function of the fpPCM and a reactive function of the substrate, preferably the binding is a result of such reaction. The substrate can be selected from the group comprising plastic or polymeric layers such as plastic films, plastic sheets, laminates or combinations of above, metals, composites, other polymers, carbons, ceramics, glass, fiberglass or other known materials used in the construction of outer surfaces of objects described throughout this disclosure.

The fpPCM can be implemented as a coating, laminate, infusion, treatment or ingredient in a coating, laminate, infusion, treatment that is formed adjacent to, on or within the substrate using any suitable coating, laminating, infusion, etc. technique. During use, the fpPCM can be positioned so that it is adjacent to the outer surface of a wrapped object, thus serving as an inner coating. It is also contemplated that the fpPCM can be positioned so that it is exposed to an outside environment, thus serving as an outer coating. The fpPCM may cover at least a portion of the substrate. Depending on characteristics of the substrate or a specific coating technique that is used, the fpPCM can penetrate below the top surface and permeate at least a portion of the substrate. While two layers are described, it is contemplated that the article can include more or less layers for other implementations. In particular, it is contemplated that a third layer can be included so as to cover at least a portion of a bottom surface of the substrate. Such a third layer can be implemented in a similar fashion as the fpPCM or can be implemented in another fashion to provide different functionality, such as water repellency, stain resistance, stiffness, impact resistance, etc.

In one embodiment, the fpPCM may be blended with a binder which may also contain a set of microcapsules that are dispersed in the binder. The binder can be any suitable material that serves as a matrix within which the fpPCM and possibly also the microcapsules are dispersed, thus offering a degree of protection to the fpPCM and microcapsules against ambient or processing conditions or against abrasion or wear during use. For example, the binder can be a polymer or any other suitable medium used in certain coating, laminating, or adhesion techniques. For certain implementations, the binder may be a polymer having a glass transition temperature ranging from about -110° C to about 100° C, and in some embodiments specifically from about -110° C to about 40° C. While a polymer that is water soluble or water dispersible can be advantageous, a polymer that is water insoluble or slightly water soluble can also be used as the binder for certain implementations.

The selection of the binder can be dependent upon various considerations, such as its affinity for the fpPCM, PCM and/or microcapsules or the substrate, its ability to modify heat transfer, its breathability, its flexibility, its elasticity, its softness, its water absorbency, its coating-forming ability, its resistance to degradation under ambient or processing conditions, and its mechanical strength. For example, the selection of a binder can influence the hardness, stiffness, break elongation, and flex modulus of the film As discussed previously, it may be desirable in certain embodiments that a wrapped sleeve structure have a Shore A hardness of >85 and a Shore D hardness of >25 (per ASTM D2240). Additionally, it may be desirable that a wrapped sleeve structure have a Flex Modulus of >15 psi (per ASTM D790) and a break elongation of >20% (per ASTM D638). The selection of a binder may be one way to achieve these properties. In particular, for certain implementations, the binder can be selected so as to include a set of functional groups, acid anhydride groups, amino groups, N-substituted amino groups and their salts, amide groups, imine groups, imide groups, azide groups, azo groups, amine-formaldehyde groups, carbonyl groups, carboxy groups and their salts, cyclohexyl epoxy groups, epoxy groups, glycidyl groups, hydroxy groups, isocyanate groups, cyanate groups urea groups, aldehyde groups, ketone groups, ester groups, ether groups, alkenyl groups, alkynyl groups, thiol groups, disulfide groups, silyl or silane groups, halogenated leaving groups, peroxide groups, salt groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof.

These functional groups can allow chemical bonding to a complementary set of functional groups included in either of, or any of, the fpPCM, the PCM, the thermal conductive particles, the microcapsules and the substrate, thereby enhancing durability of the article during processing or during use. Thus, for example, the binder can be a polymer that includes a set of epoxy groups, which can chemically bond to a set of carboxy groups included in the fpPCM, PCM, the thermal conductive particles, and/or the microcapsules. As another example, the binder can be a polymer that includes a set of isocyanate groups or a set of amino groups, which can chemically bond with those carboxy groups included in the fpPCM, PCM, the thermal conductive particles, microcapsules, or substrate.

In some instances, a set of catalysts can be added when forming the coating composition. Such catalysts can facilitate chemical bonding between complementary functional groups, such as between those included in the binder and those included in the microcapsules. Examples of materials that can be used as catalysts include boron salts, hypophosphite salts (e.g., ammonium hypophosphite and sodium hypophosphite), phosphate salts, tin salts (e.g., salts of $Sn^{+2}$ or $Sn^{+4}$, such as dibutyl tin dilaurate and dibutyl tin diacetate), and zinc salts (e.g., salts of $Zn^{+2}$). A desirable amount of a tin salt or a zinc salt that is added to the coating composition can range from about 0.001 to about 1.0 percent by dry weight, such as from about 0.01 to about 0.1 percent by dry weight. A desirable amount of a boron salt or a phosphate salt that is added to the coating composition can range from about 0.1 to about 5 percent by dry weight,

such as from about 1 to about 3 percent by dry weight. Other examples of materials that can be used as catalysts include alkylated metals, metal salts, metal halides, and metal oxides, where suitable metals include Li, Be, Na, Mg, K, Ca, Rb, Sr, Ti, V, Mn, Fe, Co Ni, Cu Zn Ga, Ge As, Se, Al, Y, Zr, Nb, Mo, Ru, Rh, Pd, Ag, Cd, In, Sn, Sb, Te, Cs, Ba, La, Hf, Ta, W, Re, Os, Ir, Pt, Au, Hg, Tl, Pb, Bi, Po, A Ra, Ac, Ce, Nd, Pm, Sm, Eu, Gd, Tb, Dy, Ho, Er, Tm, Yb, Lu, Th, Pa, U, Pu, etc.. These metals and their compounds can be used singly or blended. Organic acids and bases, such as those based on sulfur (e.g., sulfuric), nitrogen (e.g., nitric), phosphorous (e.g., phosphoric), or halides (e.g., F, Cl, Br, and I), can also be used as catalyst. Further examples of materials that can be used as catalysts include acids such as citric acid, itaconic acid, lactic acid, fumaric acid, and formic acid.

Bonds between substrate, functional phase change material, binder, PCM, the thermal conductive particles, and/or microcapsules are, according to various embodiments, covalent, electrovalent or various combinations of those. Binding could be direct or indirect, e.g. via a connecting compound. According to some embodiments, the connecting compound is selected from a group consisting of functional polymeric phase change material and microcapsules. According to another or the same embodiment, the functional polymeric phase change material may form a binder for at least a portion of a second PCM.

According to another embodiment, the reactive function of the fpPCM can be converted into another reactive function, which is more suitable for reacting with particular substrates.

According to another embodiment, the reactive function of the fpPCM could be of various chemical nature. For example, reactive functions capable of reacting and forming covalent or electrovalent bonds with reactive functions of various substrates, e.g. metals, plastics, components of an external wall of an electrochemical cell, external components of an electrochemical pack, and thermal interface materials.

According to another embodiment of the disclosure, the reactive function can be any of the following: 1) glycidyl or epoxy such as from glycidyl methacrylate or glycidyl vinyl ether; 2) anhydride such as from maleic anhydride or itaconic anhydride; 3) isocyanate such as from isocyanato methacrylate, TMI® from Cytec Ind. or blocked isocyanates such as 2-(0-[1'-methylproplyideneamino]carboxyamino)ethyl methacrylate; 4) amino or amine-formaldehyde such as from N-methylolacrylamide; and 5) silane such as from methacryloxypropyltriethoxysilane. Such reactive functions can react with OH functional groups of cellulosic based materials; with hydroxyl or carboxyl groups of polyester based materials and with amide functional groups of nylon functional resins.

According to still another embodiment of the disclosure, the reactive function may be a double bond, capable of binding to another double bond, providing a cross-linking point, a polymerization point, etc. The above described reactions, linking or crosslinking reactions can be triggered by any energy such as light, UV, IR, heat, thermal, plasma, sound, microwave, radiowave, pressure, x-ray, gamma, or any form of radiation or energy. They can be triggered by chemical reactions such as by the use of free radical, anionic or cationic catalysts or initiators.

The reactive function of the fpPCM can assume a positive charge and bind electrovalently with a negative charge on the substrate. According to another embodiment, the reactive function can assume a negative charge and bind electrovalently with a positive charge on the substrate. According to another embodiment, the reactive functions of both the substrate and the fpPCM and/or microcapsule may be negatively charged and binding is via a multivalent cation, which acts as a cross-linker. According to still another embodiment, the reactive functions of both the substrate and the fpPCM and/or microcapsule may be positively charged and binding is via a multivalent anion, which acts as a cross-linker. The crosslinking multivalent cation, anion or both could be organic or inorganic.

Examples of fpPCM Structures

Figures 7-10D are schematic drawings of fpPCMs used in accordance with various aspects of the present disclosure. Both are composed of a backbone chain and side chains. The fpPCM in Figure 7 represent long chain alkyl polyacrylate or polymethacrylate, and Figures 7A-7C where Figure 7A is long chain alkyl vinyl esters, Figure 7B is long chain vinyl ethers and Figure 7C is long chain alkyl olefins.

Figures 8A and 8B represent long chain glycol polyacrylates or polymethacrylates, where Figure 8A is long chain glycol vinyl esters and Figure 8B is long chain glycol vinyl ethers.

In Figures 7 and 8, R represents one or more of the reactive functions(s) described above. In those figures, the functions are drawn along the backbone, but that is only one option. As indicated above, the functions could also be placed at the end(s) of the backbone, on the side chains and any combination of those. Each fpPCM may have a single or multiple reactive functions. fpPCM may also carry multiple reactive functions of a similar chemical nature or a combination of reactive functions of different chemical nature. The length of the side chains may vary in different embodiments as shown by the variable chain length n.

With reference to Figures 9A-9F, Figure 9A depicts an acidic or low pH carboxyl functional fpPCM ionically interacting with a basic or high pH amino functional substrate. Figure 9B depicts basic or high pH amino functional fpPCM ionically interacting with an acidic or low pH carboxyl functional substrate. Figure 9C depicts basic or high pH amino functional fpPCM and a basic or high pH amino functional substrate being neutralized and ionically bound or "crosslinked"

with an anion such as an amine. Figure 9D depicts an acidic or low pH carboxyl functional fpPCM and an acidic or low pH carboxyl functional substrate being neutralized and ionically bound or "crosslinked" with a cation such as a metal salt. Figure 9E depicts basic or high pH amino functional fpPCM and a basic or high pH amino functional substrate being neutralized and ionically bound or "crosslinked" with negatively charged organic compound such as dicarboxy functional polymer or dicarboxy functional fpPCM. Figure 9F depicts an acidic or low pH carboxyl functional fpPCM and an acidic or low pH carboxyl functional substrate being neutralized and ionically bound or "crosslinked" with positively charged organic compound such as diamine functional polymer or diamine functional fpPCM.

With reference to Figures 10A-10D, Figure 10A depicts a covalent ether bond from the reaction of an fpPCM epoxy and hydroxyl on a cellulose substrate. Figure 10B depicts a covalent urea bond from the reaction of an fpPCM isocyanate and amine from another material. Figure 10C depicts a covalent urethane bond from the reaction of an fpPCM isocyanate on the end of a side chain and hydroxyl from a cellulose substrate. Figure 10D depicts a covalent urea and urethane bonds from the reaction of amine function, fpPCMs, multifunctional isocyanate crosslinker/binder, and hydroxyl from a cellulose substrate.

<u>Use of Precisely Branched fpPCMs</u>

In addition to the above disclosure concerning functional polymeric phase change material application and the use with various substrates, the disclosure below focuses on how using a more precisely branched polymer can give more precise and repeatable control of a copolymer addition. It is contemplated that each of the embodiments shown in Figures 7-10 may be modified to utilize the precisely branched polymers disclosed and discussed below. One of skill in the art would readily recognize how to incorporate the precisely branched polymers into the examples above and into variations of the above examples.

Standard polymerizations do not give precise and repeatable control of the comonomer addition because the catalysts generally add co-monomers in a random fashion. As discussed above, many polymers currently available commercially have random monomer incorporation. Current catalysts and polymerization technologies produce polymers with "drifting copolymer" structures where polymer chains in the product have a wide range of compositions of the comonomers. Due to the randomness of this comonomer addition, less control can be exerted over the thermal properties of the copolymer such as melt/crystallization temperature and the amount of crystallization. The amount of crystallization can be expressed as a percentage crystallinity, latent heat, heat of fusion or Joules per gram.

Precise branching control as discussed below allows for greater latent heat content at a given melt temperature versus random comonomer incorporation which requires more comonomer to give the same melt temperature, leading to lower percentage crystallinity and lower latent heat.

With general reference to Figures 11-13, various graphs are shown that illustrate the difference in both melt temperature and latent heat. In Figures 11-13 the precisely branched polymers are labeled as ADMET. In the various embodiments, figures and examples, ADMET stands for Acyclic Diene Metathesis Polymerization and generally represents polymers with precisely placed methyl branches.

It is well known in the industry, that for polyolefin polymers the melting temperature and latent heat are controlled by the longest carbon segments in the polyolefin backbone. For instance in polyethylene random copolymers, the distribution of ethylene sequences vary. In the case of polyethylene copolymers, thick crystal lamella can be formed from the longer ethylene sequences which will result in higher melting temperatures. The shorter ethylene sequences and/or branches will not be incorporated into these crystallites or lamella resulting in lowered heats of fusion or Joules per gram.

In accordance with one embodiment and example, a precisely branched polyethylene material having equal ethylene sequences yields the following features: a faster decrease in melting temperature at lower branch content; better control of the melting temperature; and an overall higher amount of ethylene sequences incorporated into the lamella or crystallite, yielding higher latent heats.

Therefore, aspects of the disclosure and the use of these precisely controlled copolymers yields materials with phase change materials exhibiting enhanced temperature regulating, heat absorbing, and heat releasing properties. In order to obtain an embodiment of a PCM with melting temperatures suitable for use in electrochemical device applications and as described below, many embodiments have PCMs with a latent heat of fusion of >10 Joules per gram and other specifically of >60 Joules per gram. PCMs have a latent heat of fusion of >25 Joules per gram in one embodiment, > 5 Joules per gram in another embodiment, and between 5 and 150 Joules per gram in yet another embodiment, a PCM in one example has a structure with one or more of the following features:

- The overall polymer molecular weight (n) is between 100 - 10,000,000, between 1000-1,000,000 or between 10,000 - 500,000 in various embodiments.
- With the molecular weight distribution expressed as polydispersity or Pd=Mn/Mw, it is between 1-100, between 1-10, or between 1-5 in various embodiments.

- The ethylene length between branches is between 5-500,000, between 10-400,000, or between 10-20 in various embodiments.

The length described above can be expressed in a number of ways such as number of m units in diagram 1 below, branches per 1000 carbons (branches/1000 C), mole % branches or weight % branches. The PCM structure also has the following characteristics in one or more embodiments:

- The number of m units is between 0-500,000, between 0-200,000 or between 5-15,000 in different embodiments.
- There are between 200-0, between 100-5 or between 100-0 branches per 1000 carbons (branches/1000 C) in different embodiments.
- The mole % of the branches is 0-50% or between -30% in different embodiments.
- The weight percentage of the branches is between 0-50% or between 10-30% in different embodiments.

In some embodiments the branches may be methyl branches from propylene as the comonomer and propylene may be 0-30 mole % of the polymer. In another embodiment, propylene may be 0-39 weight % of the polymer

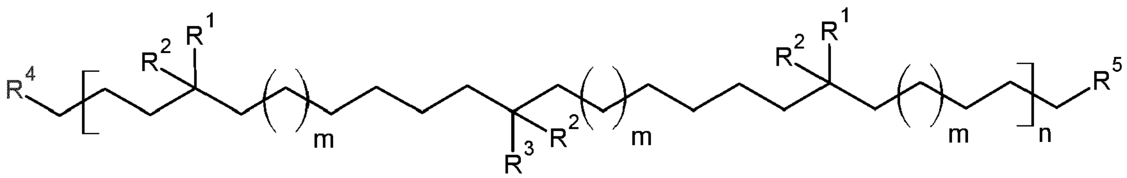

Diagram 1

In diagram 1 above, illustrating one embodiment, R1 may be any of CH3, C2H5, CnH2n+1, OCH3, OC2H5, or any functional group, polar group, halogen group, their normal, branched or mixed group. In certain embodiments R1 is selected from either CH3 or OCH3. In diagram 1, R2= H, CH3 and in certain specific embodiments, R2 is limited to H.
In diagram 1, R3= H, CH3 or some concentration of a selected functional group, polar group or halogen group. In certain embodiments, R3 is limited to CH3 or to one or more concentrations of a functional group
In diagram 1, R4 and R5 are polymer end groups and can be H, CH3 or any functional group, polar group or halogen group, salt, metal, catalyst end, crosslinking group, another polymer chain to form copolymers (block, graft, etc.). In some embodiments, R4, R5 can be the same or different. In certain embodiments, R4 and R5 are limited to H, CH3 or any functional group.
In accordance with other aspects R1, R2, R3, R4, and R5 can have some degree of stereocontrol or stereochemistry associated with them. For example:

- R1, R2, R3 may be atactic, isotactic or syndiotactic.
- R1, R2, R3 may also be controlled for chirality to yield L-and D-chiral homo and copolymers (or another way of stating is R- and S- polymers).

Different polymer architectures based on the stereochemistry of the polymer chain can lead to various crystalline complexes. For example, syndiotactic homo- and copolymers can complex with isotactic homo- and copolymers. Polymers of opposite chirality can be mixed to form a racemic stereocomplex. In one embodiment, a certain amount of isotactic L-polymer can be mixed with an amount of isotactic D-polymer to yield a racemic stereocomplex with thermal and physical properties different than the L- or D-chiral homopolymers. (A polymer stereocomplex is defined as a stereoselective interaction between two complementing stereoregular polymers, that interlock and form a new composite, demonstrating altered physical and thermal properties different than the parent polymers.)
These syndiotactic, isotactic or L- and D-chiral segments can be in different polymer chains (homopolymer segments) or in the same polymer (stereoblock copolymers segments). The syndiotactic, isotactic or L- and D-chiral segments can make up anywhere between 1-100% of the polymer or copolymer as well as various intermediate percentage ranges.
The syndiotactic, isotactic or L- and D-chiral segments can be mixed in a ratio between 9:1 and 1:9 to give complete or partial stereocomplex formation and subsequent thermal and physical property adjustment. Other crystalline complexes may also be utilized such as complexes between different polymers such as polyamides and polyesters, cationic/anionic, polyethers/polyacids, and triple helixes.
R3, R4, R5 can be any functional group, polar group or halogen group, salt, metal, catalyst end, crosslinking group, or any of the following functional groups: acid anhydride groups, amino groups, N-substituted amino groups and their salts, amide groups, imine groups, imide groups, azide groups, azo groups, amine-formaldehyde groups, carbonyl

groups, carboxy groups and their salts, cyclohexyl epoxy groups, epoxy groups, glycidyl groups, hydroxy groups, isocyanate groups, cyanate groups urea groups, aldehyde groups, ketone groups, ester groups, ether groups, alkenyl groups, alkynyl groups, thiol groups, disulfide groups, silyl or silane groups, halogenated leaving groups, peroxide groups, salt groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof.

The crosslinking groups discussed above may also include the ability to reversibly or irreversibly change the crosslink by thermal, sonic, photon, pressure, or chemical (water, solvents, ions, etc.) energy.

Various other methods of functionalization are described shown in the following references, the details of which are incorporated by reference into the present application: Synthesis of Functional Polylefins using Metallocenes: A Comprehensive Review; Atiqullah M., et.al.; Polymer Reviews, 50:178-230, 2010; Comprehensive Organometallic Chemistry III: 11.20-Polymerization of Alkenes; Elsevier Ltd.; Fujita T., Makio H.; Vol. 11, 691-734, 2007; Functionalized Ethylene Copolymers and Materials via Olefin Metathesis Polymerization; Baughman, T., Univ. of Florida Dissertation, 2006.

In accordance with another aspect, the polymer may have a certain degree of unsaturation or include double and triple bonded atoms in the polymer. For instance, alkene or alkyne bonds. This unsaturation (not all valence electrons are "saturated" or reacted to other atoms) can be incorporated and controlled by the monomers used in the polymerization (isoprene, butadiene, α-olefins, etc.), hydride elimination (β-hydride elimination, etc.), various polymerization mechanisms (ring-opening metathesis polymerization (ROMP), acyclic diene metathesis (ADMET described herein, etc.) or controlled hydrogenation/dehydrogenation. For instance, as shown in Diagram 2 and Figure 14, examples of making the disclosure by ADMET polymerization and subsequent hydrogenation are shown. Figure 14 shows the effect of unsaturation on the thermal properties.

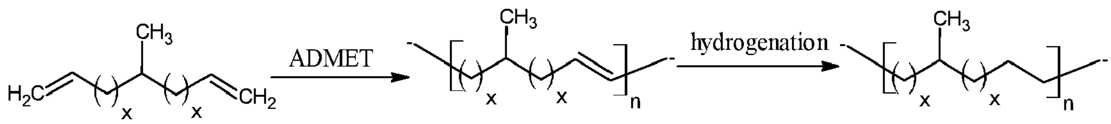


Diagram 2

As can be seen, unsaturation causes a lower melting temperature and also slighly lower latent heat of fusion, but by controlling the level of unsaturation, one can also control the thermal properties. In some embodiments the unsaturation is 0-99 mole %. In other embodiments the unsaturation is 0-15 mole %. In various other embodiments, various intermediate ranges of mole % are utilized.

The isomeric orientation of the double bond can also have an effect on the properties of the unsaturated polymer. The cis/trans orientation of the double bond can also be controlled by the hydrogenation catalyst or the hydrogenation process conditions such as time, temperature, pressure, etc.

The isomer ratio, unsaturation and double bond orientation will vary depending on the comonomer composition, comonomer distribution and polymerization conditions. One goal of a composition in accordance with an aspect of the present disclosure is to maximize latent heat in the preferred temperature range.

Copolymer segments used in connection with aspects of the present disclosure can be copolymers or blended with any of the other polymers/copolymers disclosed herein. In addition, copolymer segments used in connection with aspects of the present disclosure can be high melt or low melt.

Use of Nucleating Agents

In order to improve the crystallinity of the PCM, pPCM and/or fpPCM and therefore the latent heat and thermal conductivity of these materials, nucleating agents can also be added. Nucleating agents can effect improved crystal growth, crystallization speed, crystal shape and size, amount of crystallization and number of crystallites of the materials.

Furthermore, the particle size of the nucleating agent and the mixing of these are important factors in ensuring optimal performance of the additives. Features of a good melt insensitive nucleating agent may be selected from one or more of the following:

- It contains both an organic group and a polar group
- It is well dispersed
- It is insoluble or can become insoluble in the polymer
- It has an epitaxial match with the PCM crystal

- The physical or chemical nature of nucleating agents can be diverse. A nucleating agent can be:

    o An impurity, i.e. catalyst residue
    ◦ An organic compound like benzoic acid, hydrocarbons or fatty acid based materials
    ◦ An inorganic compound such as mineral or pigment
    ◦ A foreign polymer crystal, i.e. polyethylene for octadecane, etc.
    ◦ A diluent, i.e. a material that lowers the Tg or improves the chain mobility leading to improved chain packing.

Additional nucleating agents that may be utilized include those described in US Patent No. 7569630 β-Crystalline Polypropylene to Chemtura Corp., US Patent No. 7879933 Blending Nucleating Agents Composition and Methods to Milliken & Company, and Hyperform® products from Milliken & Company. Other examples are aliphatic hydrocarbons, aromatic hydrocarbons, alcohols, fatty acids, fatty acid salts, fatty acid esters, fatty amides, fatty amines, fatty alcohols, N-Phenyl-N'-stearylurea, zinc glycerolate, benzoic acid, salts and esters of benzoic acid, benzoic acid derivatives, Na benzoate, Li benzoate, Al hydroxy -4-tert.- butyl benzoate, phosphate esters, phosphate ester salts, sodium 2,2'-methylene-bis-(4,6-di-tert-butylphyenyl)phosphate, dibenzylidene sorbitol, derivatives of dibenzylidene sorbitol, linear or branched polyphenyls, salts of hydrogen phthalate, 1, 2-cyclohexanedicarboxylic acid and the mono or di neutralized salt (i.e. Na, K, Al, B, Ti, Zn, Ca, Li, Mg, etc.), cetyl alcohol, stearyl alcohol, eicosanol, myristic acid, palmitic acid, behenic acid, stearic acid amide, ethylenebisoleic acid amide, methylolbehenic acid amide, 1-octacosanol, 1-heptacosanol, 1-hexacosanol, 1-pentacosanol, 1-tetracosanol, 1-tricosanol, 1-docosanol, 1-heneicosanol, 1-eicosanol, 1-nonadecanol, 1-octadecanol, 1-heptadecanol, 1-hexadecanol, 1-pentadecanol, 1-tetradecanol, 1-tridecanol, tridecylamine, tetradecylamine, pentadecylamine, hexadecylamine, heptadecylamine, octadecylamine, nonadecylamine, eicocylamine, heneicocylamine, dococylamine, tricocylamine, tetracocylamine, pentacocylamine, hexacocylamine, heptacocylamine and octacocylamine, etc. Minerals such as talc, kaolin, calcium carbonate, $TiO_2$, salts, NaCl, and Na carbonate may also be used as nucleating agents. These compounds may be used singly or in combination of two or more thereof.

Microcapsules and Other Containment and/or Absorbing Structures for Phase Change Materials

PCM can be contained in microcapsules or some other containment structure or particulate. Containment structures other than microcapsules are capable of carrying functional groups, either as a natural part of their structure or from their modified manufacturing. For instance, PCMs can be absorbed and stabilized into any number of particles including silica (fumed or precipitated), graphite, graphene, carbon nanoparticles, carbon or activated carbon, zeolites, organoclays, montmorillonite, bentonite, clay, talc, and vermiculite. Paraffin or hydrophobic PCMs can also be absorbed in any number of polymers, especially crosslinked polymers, similar to how a plasticizer will absorb into plastics. For instance PCM can be absorbed into any polyolefin and polyolefin copolymer such as polyethylene, polypropylene, salts of polyolefins and their copolymers, poly(meth)acrylates, salts of poly(meth)acrylates, polyvinyls, aliphatic polyesters, rubbers, copolymers (i.e. Kraton® copolymers, Elvaloy®) and mixtures, etc. PCMs based on glycols can be absorbed into any hydrophilic polymers such as polyvinyl alcohol, substituted cellulose (CMC, HMC, etc.) etc.

PCMs, pPCMs, fpPCMs can also be contained, absorbed or infused on any particle or fiber including the below described thermal conductive fillers, reinforcing materials, fire retardant additives, etc. These containment or absorbing particle can be any shape such as round, spherical, cylindrical, fiber, sheet, flake, powder, whiskers, tubular, platelets, foams, meshes, agglomerates of these shapes or any other irregular shape. Depending on the application, a single shape or mixture of shapes may be advantageous to provide the optimum packing and arrangement for particle contact. These containment or absorbing particles can be any size, but in certain embodiments of the present disclosure, may be 0.1nm to 100mm.

Other materials which can absorb or contain PCMs such as standard superabsorbent polymers based on cross-linked sodium polyacrylate. Other materials are also used to make a superabsorbent polymer, such as polyacrylamide copolymer, ethylene maleic anhydride copolymer, cross-linked carboxymethyl-cellulose, polyvinyl alcohol copolymers, cross-linked polyethylene oxide, and starch grafted copolymer of polyacrylonitrile to name a few.

Microcapsules are implemented to contain a phase change material, which serves to absorb or release heat to reduce or eliminate heat transfer. In particular, the microcapsules are formed as shells that define internal compartments within which the phase change material is positioned. The shells can be formed of any suitable material that serves to contain the phase change material, thus offering a degree of protection to the phase change material against ambient or processing conditions or against loss or leakage during use. For example, the shells can be formed of a polymer or any other suitable encapsulation material.

The selection of a material forming the microcapsule shells can be dependent upon various considerations, such as its affinity for other materials in the TMM, affinity or adhesion to batteries, its reactivity or lack of reactivity with the phase change material, TMM materials or batteries its resistance to degradation under ambient or processing conditions, and its mechanical strength. In particular, for certain implementations, a material forming the shells can be

selected so as to include a set of functional groups, acid anhydride groups, amino groups, N-substituted amino groups and their salts, amide groups, imine groups, imide groups, azide groups, azo groups, amine-formaldehyde groups, carbonyl groups, carboxy groups and their salts, cyclohexyl epoxy groups, epoxy groups, glycidyl groups, hydroxy groups, isocyanate groups, cyanate groups urea groups, aldehyde groups, ketone groups, ester groups, ether groups, alkenyl groups, alkynyl groups, thiol groups, disulfide groups, silyl or silane groups, halogenated leaving groups, peroxide groups, salt groups, groups based on glyoxals, groups based on aziridines, groups based on active methylene compounds or other b-dicarbonyl compounds (e.g., 2,4-pentandione, malonic acid, acetylacetone, ethylacetone acetate, malonamide, acetoacetamide and its methyl analogues, ethyl acetoacetate, and isopropyl acetoacetate), halo groups, hydrides, or other polar or H bonding groups and combinations thereof. At least some of these functional groups can be exposed on outer surfaces of the shells and can allow chemical bonding to a complementary set of functional groups included in either of, or both, the substrates or other TMM materials, thereby enhancing durability or adhesion. In such fashion, at least some of the microcapsules can be chemically bonded to other PCMs, pPCMs, fpPCMs or fillers. Thus, for example, a material forming the shells can include a set of carboxy groups, which can chemically bond to a set of hydroxy groups. As another example, those carboxy groups included in the shells can chemically bond to a set of amino groups.

Examples of polymers that can be used to form the shells include those listed under the "polymers" definition, including those with carboxy groups, such as polymers including monomeric units based on acrylic acid or methacrylic acid. For certain implementations, the shells can be formed of a polymer that includes from about 1 to about 100 molar percent of monomeric units that include carboxy groups, such as from about 20 to about 80 molar percent, from about 25 to about 60 molar percent, or from about 40 to about 50 molar percent of the monomeric units. In some instances, it can be desirable to adjust a molar percentage of the monomeric units based on sizes of the microcapsules. For example, as a size of an individual one of the microcapsules decreases, an outer surface area of that microcapsule also typically decreases. Thus, to maintain a desired amount of exposed functional groups for chemical bonding, it can be desirable to increase the molar percentage of the monomeric units as the size of that microcapsule decreases. As another example, as a size of an individual one of the microcapsules increases, a weight of that microcapsule also typically increases. Thus, to account for the increasing weight, it can be desirable to increase the molar percentage of the monomeric units as the size of that microcapsule increases. Table 1 provides examples of ranges of the molar percentages as a function of the sizes of the microcapsules. Referring to Table 1, the microcapsules are assumed to be spherical for ease of presentation. Similar considerations and molar percentages can also apply to polymers with other types of functional groups.

TABLE 1

| Radius - r (μm) | Surface area - $4\pi r^2$ ($\mu m^2$) | Molar percent of monomeric units with carboxy groups |
|---|---|---|
| 0.5 | 3 | 50-60 |
| 1 | 13 | 45-55 |
| 2 | 50 | 40-50 |
| 3 | 113 | 40-50 |
| 4 | 201 | 35-45 |
| 5 | 314 | 35-45 |
| 6 | 452 | 30-40 |
| 7 | 616 | 30-40 |
| 8 | 804 | 25-35 |

Other examples of polymers that can be used to form the shells include those formed of monomers using any suitable polymerization technique. Table 2 below sets forth examples of such monomers that include different types of functional groups.

TABLE 2

| Functional Group | Monomers |
|---|---|
| Carboxy Group | acrylic acid, methacrylic acid, maleic acid, itaconic acid, citraconic acid, vinylacetic acid, p-vinylbenzoic acid, 2-acryloyloxyethylacidphosphate, β-acrytoytoxyethyt hydrogen succinnate (or any other anhydride reacted or modified hydroxy group-containing monomer), and any other unsaturated polymerizable carboxylic acid |

(continued)

| Functional Group | Monomers |
|---|---|
| Isocyanate Group | isocyanato methacrylate, monomer supplied as TMI by Cytec Industries, 2-methacryloyloxyethyl isocyanate, acryloyloxyethyl isocyanate, blocked isocyanates such as 2-(0-[1'-methylproplyideneamino]carboxyamino)ethyl methacrylate, and any other unsaturated polymerizable isocyanate |
| Anhydride Group Hydroxy Group | maleic anhydride, itaconic anhydride, citraconic anhydride, and any other unsaturated polymerizable anhydride $CH_2$=CR'COO($CH_2$)$_n$OH, where R' = $CH_3$ or H, n = 2-4 (e.g., hydroxyethyl methacrylate, hydroxyethyl acrylate, hydroxypropyl methacrylate, hydroxypropyl acrylate, hydroxybutyl methacrylate, and hydroxybutyl acrylate); $CH_2$=CR'COO(($CH_2$)$_n$O)$_z$OH, where R' = $CH_3$ or H, n = 1-10, z = 1-1,000 (e.g., glycol based acrylates or methacrylates, such as ethyleneglycol methacrylate, ethyleneglycol acrylate, polyethyleneglycol methacrylate, and polyethyleneglycol acrylate); allyl alcohol; $\alpha$-ethylallyl alcohol; allylcarbinol; $CH_2$=CH-($CH_2$)n-O-($CH_2$)$_n$O$_z$OH, where m = 0-4, n = 1-10, z = 1-1000 (e.g., glycol based vinyl ethers, such as ethyleneglycol monovinyl ether and polyethyleneglycol monovinyl ether) ; $CH_2$=CH-O-CO-(($CH_2$))$_n$O)$_z$OH, where n = 1-10, z = 1-1000 (e.g., glycol based vinyl esters, such as ethyleneglycol monovinyl ester and polyethyleneglycol monovinyl ester); and any other unsaturated polymerizable hydroxy group-containing monomer |
| Epoxy Group Amino or N-Substituted Amino Group Silyl Group | glycidyl methacrylate, glycidyl acrylate, allyl glycidyl ether, 2-vinyloxyethyl glycidyl ether, and any other unsaturated polymerizable epoxy-group containing monomer acrylamide; methacrylamide; $CH_2$=CR'CONHCH$_2$OX, where R' = $CH_3$ or H, X = H, methoxy, ethoxy, propoxy, isopropoxy, butoxy, or isobutoxy; and vinylamine; and any other unsaturated polymerizable amino group-containing monomer methacryloxypropyltrimethoxysilane, methacryloxypropyltriethoxysilane, methacryloxypropyltributoxysilane, triethoxyvinylsilane, trimethoxyvinylsilane, triacetoxyvinylsitane, triisopropoxyvinylsilane, tris(methoxyethoxy)vinylsilane, and any other unsaturated polymerizable silane |

Microcapsules can also be multi-walled or have multiple shell layers. The inner layer should provide the required strength, durability and containment while the outer layers provide compatibility or adhesive properties. The outerwall of the microcapsule can be a thermoplastic polymer which can melt or soften at desired temperatures to flow, act like a viscous liquid or fuse the microcapsules to substrates, other microcapsules or other TMM materials. The thermoplastic outer wall should have a melting or glass transition temperature (Tg) below the temperature which will damage the wall of an electrochemical cell, but above normal use temperatures. The outerwall can be made of any polymer that melts, softens or flows such as polyolefins, rubbers, polyvinyls, poly(meth)acrylates, polyurethanes, etc. or copolymers.

Microcapsules can have the same shape or different shapes, and can have the same size or different sizes. In some instances, the microcapsules can be substantially spheroidal or spherical, and can have sizes ranging from about 0.1nm to about 1,000 microns, such as from about 0.1nm to about 500 microns, from about 0.1nm to about 100 microns, from about 1 to about 15 microns. For certain implementations, it can be desirable that a substantial fraction, such as at least 50 percent, or at least 80 percent of the microcapsules have sizes within a specified range, such as from about 1 to about 15 microns. It can also be desirable that the microcapsules are monodisperse with respect to either of, both, their shapes and sizes.

Microcapsules can be of a durable, breakable, or controlled release type. It can be advantageous to have the microcapsule core material permanently contained to prevent leakage or loss of core material during use or processing. It may also be advantageous to have the core material released thru either fast (breakable) or slow (controlled release) means. For instance, when microcapsules are added to composites, and the composites are cured at higher temperatures, small voids can be formed between the composite and microcapsule shell wall due to variables such as differential expansion between microcapsule and composite, incompatibility between microcapsule and composite or shape distortion, etc. Release of core material and allowing it to flow into and fill these voids allows for greatly improved thermal conductivity. This is shown in Figures 15A-15B.

Selection of a PCM Transition Temp.

The selection of a PCM is typically dependent upon the transition temperature that is desired for a particular application that is going to include the PCM. The transition temperature is the temperature or range of temperatures at which the PCM experiences a phase change from solid to liquid or liquid to solid. For example, a PCM having a transition

temperature near the normal range of temperatures that batteries reach during multiple charge and discharge cycles can be desirable for battery applications to prevent overheating, thermal runaway, and fires. A phase change material according to some embodiments of the disclosure can have a transition temperature in the range of about 5 °C. to about 120 °C. In one embodiment, the transition temperature is about 10°C. to about 80°C. In another embodiment, the transition temperature is about 30 °C. to about 70 °C. In another embodiment, the PCM has a transition temperature of about 40 °C. to about 60 °C.

In applications for keeping cold materials cold or for protecting objects from the effects of extreme ambient cold temperatures, the transition temperatures may be in the range of -60°C to 5°C.

In certain industrial applications, temperatures of transported and stored materials within pipes or holding tanks, for example, may reach extremely high temperatures. In such cases, the phase change material may have wide ranges, such as above 500°C.

In applications for contact with human skin, transition temperatures may be within a certain range of body temperatures, such as 90°-100°F.

In applications for commercial or residential building purposes, transition temperatures may be in the range of room temperature, for example, if the film is used for insulation of living areas. The ranges may be wider than around room temperature to accommodate for extreme cold or hot weather. Alternatively, some transition temperatures for building purposes may be suited to common temperatures associated with water heaters, electrical wires, and other heat-generating building components.

In applications for thermally managing solar power, transition temperatures may be in the range of operating temperatures for solar panels, such as between 100°-150° C

The transition temperature can be expanded or narrowed by modifying the purity of the PCM, molecular structure, blending of PCMs, blending of mPCM, blending of polymeric PCMs and any mixtures thereof.

In addition, the temperature control is dependent on the absorption and release of energy by changing phases, preferably by absorbing energy and melting or releasing/dissipating heat by solidifying or crystallization. These melting (Tm) and crystallization (Tp) temperatures for a single PCM material in embodiments of this disclosure is not widely spread ("supercooling"). This difference can be defined as the difference between peak melting temperature and peak crystallization temperature as measured by a DSC., i.e. $\Delta T$ (°C) = Tm-Tc. In some embodiments this $\Delta T$ is <15 °C, in others <10 °C, and in yet others <5° C.

Mixing and Layering of PCM Transition Temperatures

In some embodiments, it may be advantageous to have multiple or broad transition temperatures. If a single narrow transition temperature is used, this can cause thermal/energy buildup before the transition temperature is reached. Once the transition temperature is reached, then energy will be absorbed until the latent energy is consumed and the temperature will then continue to increase. Broad or multiple transition temperatures allow for temperature regulation and thermal absorption as soon the temperature starts to increase, thereby alleviating any thermal/energy buildup. Multiple or broad transition temperatures can also more efficiently help "bleed-off" or conduct heat away from an object by overlapping or staggering thermal absorptions. For instance a TMM contains PCM1 which absorbs at 35-40°C and PCM2 which absorbs at 38-45°C. PCM1 will start absorbing and controlling temperature until a majority of the latent heat is used, at which time PCM2 will start to absorb and conduct energy from PCM1 thereby rejuvenating PCM1 and allowing it to keep functioning. This broadening or mixing of transition temps can be done by combining all materials or layering them as depicted in the various figures.

Selection of Latent Heat Values

The selection of the phase change material can be dependent upon a latent heat of the phase change material. A latent heat of the phase change material typically correlates with its ability to absorb and release energy/heat or modify the heat transfer properties of the article. In some instances, the phase change material can have a latent heat that is at least about 10 J/g, such as at least 20 J/g, as at least about 40 J/g, at least about 50 J/g, at least about 70 J/g, at least about 80 J/g, at least about 90 J/g, or at least about 100 J/g. Thus, for example, the phase change material can have a latent heat ranging from about 10 J/g to about 60 J/g, from about 20 J/g to about 400 J/g, such as from about 60 J/g to about 400 J/g, from about 80 J/g to about 400 J/g, or from about 100 J/g to about 400 J/g.

TMM Rheology. Viscosity. Elasticity, Crosslinking and Physical Properties of the PCM

The rheology (i.e. viscosity or flow properties) of the TMM is important for many different processes of the application to electrochemical cells and packs. Variables such as general application of the TMM to the cells or packs either by inserting the cell into a sleeve or casing, wrapping a cell with tape or film, or otherwise applying with spray,

flow, coating, pressure adhesive or extrusion is controlled by the rheology. The application must balance rheology to achieve sufficient gap fill and reduction of air spaces for good thermal conductivity versus maintaining the structure and stability of the TMM on a cell or pack. That is, the material should not allow a tube, sleeve, casing, film, tape, or other surrounding material to slide off the cell.

TMM with phase change and/or polymeric properties are advantageous in solving the above problems since they can be formulated to be solid at low temperatures and deformable or flowable at phase change or higher temperatures. The phase change temperature can be lower, equal to or higher than the normal operating temperature of the battery cell or battery pack. These TMM embodiments of tubes, sleeves, and wrapped film and tape are also advantageous over greases or waxes alone in that improved physical properties such as tensile strength, elongation, flexibility, toughness can be improved.

Rheology, viscosity, elasticity, and flow of the TMM can be controlled by many different variables. These can include the characteristics of the pPCM/fpPCM such as type of polymer, the polymer structure, the polymer mol. wt., polymer branching (i.e. number of branches, length of branches, types of branches, etc.), polymer functionality (i.e. type and amount), copolymers, compatibility with other polymers, crosslinking of the polymer, type of crosslinking, entanglement of the polymer, compatibility with additives, etc.

One way in which the elasticity of the TMM can be controlled is through the section of polymer materials with elastomeric properties. Such elastomeric materials include polyolefin copolymers such as copolymers of polyethylene with any $C_3$-$C_{30}$ $\alpha$-olefin, or vinyl acetate, other vinyl monomers such as styrene or its analogues, acrylate or methacrylate monomers, vinyl ether monomers, vinyl ester monomers, acrylonitriles, rubbers and copolymers made from isoprene, butyl, etc., or combinations, blends, mixtures thereof. Additionally, the TMM may be made of polymer elastomers such as polyesters, silicon rubbers or polyurethanes. Non-inclusive examples of these are Hytrel® polyesters from DuPont®, Spandex®- type polyurethanes and RTV or LTV type silicon rubbers.

An additional way that the elastomeric properties may be influenced is by crosslinking. In certain embodiments, the TMM and the PCM may be crosslinked with an average of 0.05-1 crosslinks per polymeric chain.

The polymer glass transition temperature (Tg, temperature at which a polymer changes from a glassy state to a rubbery state) can be controlled to allow for flow at or below operating temperatures. The Tg can be between -20°C and 150°C, preferably between 15°C and 90°C.

The polymer mol. wt. can be controlled to provide low or high viscosity with higher mol. wt. polymers providing low viscosity or no flow. The molecular weight of pPCM/fpPCM in some embodiments can be at least 500 Daltons, and in other embodiments can be at least 2000 Daltons. It is possible that in some embodiments, Mol. wts. can range from 500 Daltons to millions of Daltons, as exemplified by the use of ultra high mol. wt. polyethelene (UHMWPE)

The polymer branching can be controlled to provide for chain entanglement and rheology control. The polymer chain can have any number and length of chains. The polymer may have 0-1000 branches. The branches are at least 50 daltons. The branches can be in any configuration such as random, comb, etc. The branches can be in a stereospecific configuration.

The polymer can be a copolymer or block copolymer in which there are "hard" segments and "soft" segments. The hard segments can be due to high Tg moieties or crystallizible moieties, whereas the soft segments can be due to low Tg moieties or amorphous segments. The hard segments can interact and create a crosslink, entanglement or interaction that creates a degree of fixation or high viscosity. The soft segment can be free to flow or rearrange due to heat or pressure.

In one embodiment, the weight of the crystallizable section forms at least 20%, in other embodiments at least 50%, and in yet other embodiments at least 70% of the total weight of the pPCM/fpPCM.

They type and amount of functionality can also effect the rheology, flow properties and setting of the TMM. For instance, the TMM can be applied to the electrochemical cell or pack, then fully cured and crosslinked through any of the previously described functionality, reactions or crosslinking mechanisms.

The functionality can be also be mixed to give different crosslinking reactions and different kinetics of the crosslinking reactions. As example, epoxy and amines can quickly cure at room temperature to provide for a fast set, tack free time, and use. This partial cure allows for slight flow or gap fill either over time or as the TMM warms up from battery cell charge and discharge cycles - at this time, a second set of crosslinking reactions can occur to fully crosslink and fully set the TMM. These second set of crosslinking reactions can be triggered either by oxidation, heat, UV etc. such as oxygen radical crosslinking of unsaturated groups, N-methylol ether crosslinking with hydroxyl or carboxyl, or UV initiated free radical reaction with unsaturated moieties.

The different crosslinking reactions and kinetics can be controlled by the use of different catalysts or initiators that react at different temperatures. For instance, two different free radical (FR) initiators (peroxide, azo, etc.) with different decomposition temperatures can be combined with a polymer containing various unsaturated groups. The first FR initiator can decompose at application temperatures creating a partial cure, crosslinking or set while allowing for some flow and gap fill. The second FR initiator can then decompose and react to further react at higher temperatures.

These various temperatures of reaction, application or use can also be controlled by external environments.

For instance, room temperature crosslinking reactions can be controlled by preparing, shipping/storing and applying the TMM at lower than room temperature or at freezing temperatures, then allowing the reaction to proceed as batteries are installed in their devices or as battery packs are assembled. Likewise, crosslinking reactions can be controlled by the addition of heat, light or other forms of energy.

Rheology, viscosity, flow and gap fill can also be controlled by the use of reversible reactions or crosslinks. These are sometimes referred to as "self-healing" systems. For instance, the TMM can contain ionic crosslinks based $Zn^{+2}$ carboxyl salts or S-S disulfide bonds that can easily break, allow flow and recombine.

Examples of reversible reactions are hydrolysis of ester bonds, breaking and recombination of the urethane bond into hydroxyl and isocyanate, breaking and recombination of the urea bond into amine and isocyanate, Diels-Alder reaction based crosslinks, ionic bonds based on metals, metal coordination or organic salts, hydrogen bonding, metal coordination, hydrophobic forces, van der Waals forces, pi-pi interactions and electrostatic effects.

The above described phase changes or chemical associations can also be used as reversible crosslinks, i.e. the melting and recrystallization of polymer crystalline segments. Associations of hydrophobic/hydrophilic sections of copolymers or block copolymers. Copolymers or block copolymers with different Tg segments, hard/soft segments, etc.

Rheology, viscosity, flow and gap fill can also be controlled by the use or addition of rheology control agents, thickeners, etc. These materials are such things as silica, carbon particles, talc, ZnO, Calcium carbonate, partially crosslinked or gelled polymers, etc. The following described particles and fillers can also be used a rheology control or viscosity agents.

## Fillers and Additives - Thermal Conductive Fillers

Aspects of the present disclosure pertain to using a thermal conductive filler in combination with the various phase change materials described herein. As described above, Thermal Conductivity, k (also denoted as $\lambda$ or $\kappa$), is the property of a material's ability to conduct heat is measured in W/m·K.

Thermal conductivity is defined as the quantity of heat (Q) transmitted through a unit thickness (L) in a direction normal to a surface of unit area (A) due to a unit temperature gradient ($\Delta$T) under steady state conditions and when the heat transfer is dependent only on the temperature gradient.

The TMM described herein can also have various conductive additives and fillers to improve the various properties. Materials to enhance the thermal conductivity can be added such as forms of carbon (graphene, graphite (synthetic or natural, expanded or exfoliated, etc), graphite oxide, diamond (synthetic or natural), diamond aerogel, carbon fibers, carbon nanotubes (CNT or single walled (SWCNT)), multiwalled (MWCNT)), fullerenes (buckyballs), loonsdaleite, carbine, glassy carbon, amorphous carbon and their various forms of oxides or other functionalized forms. Other thermal conductive particles can include highly crystalline or highly oriented organic materials and polymers such as high density polyethylene (low mol. wt. or UHMWPE, such as Dyneema® fibers or Spectra® fibers), polybenzobisoxazole (PBO or Zylon®), poly(p-phenylene benzobisthiazole) (PBT), and any other liquid crystalline polymer (LCPs), metals, metal salts, metal carbides, metal oxides, metal nitrides, metal sulfate derivatives, metal phosphate derivatives, boron nitride (cubic and hexagonal), boron carbide, boron oxide, alumina, Al, Al oxide, $Al_2O_3$, Al nitride, Ag, Au, metallized glass, Sn, Zn, ZnO, Cu, Mg, MgO, wollastonite, silica (fused, fumed, precipitated, coated, colloidal, agglomerated),silicon carbide (SiC), silicone, silica coated particles and alloys (silica coated Al nitride), sapphire, quartz, polyhedral oligomeric silsesquioxane (POSS), Fe, Fe oxides, Pt, Pb, Ni, Ti, Ti oxides, Cr, Bi, In, Ga, Ge, Ce, La, W, WC, Li, Be, Be oxides, Ca, Rb, Sr, Cs, Ba, Fr, Ra, Sc, V, Mn, Co, As, Se, Y, Zr, Zr oxides Nb, Mo, Tc, Ru, Rh, Cd, In, Sb, Te, Hf, Ta, Re, Os, Ir, Hg, Tl, Po, Rf, Db, Sg, Bh, Hs, Mt, Ds, Rg, Pr, Nd, Pm, Sm, Eu, Gd, Tb, Dy, Ho, Er, Tm, Yb, Lu, Ac, Th, Pa, U, Np, Pu, Am, Cm, Bk, Cf, Es, Fm, Md, No, Lr, etc. and their blends, solders, eutectics and alloys.

Low melting or fusible materials and fillers can also be included for enhancement of rheology, gap fill, and conductivity (electrical and thermal). These may comprise Ga, In, Sn, or any alloy and oxide thereof. The low melting metal may optionally further comprise Ag, Bi, Cd, Cu, Pb, Zn, or a combination thereof. Examples of suitable low melting metals, alloys and their oxides include Ga, In-Ga alloys, In-Ag alloys, In-Bi alloys, In-Sn alloys, Bi-Sn alloys, Pb-Sn alloys, In-Pb alloys, In-Pb-Ag alloys, In-Bi-Sn alloys, Sn-In-Zn alloys, Sn-Bi-Zn alloys, Sn-In-Ag alloys, Sn-Ag-Bi alloys, Sn-Bi-Cu-Ag alloys, Sn-Ag-Cu-Sb alloys, Sn-Ag-Cu alloys, Sn-Ag alloys, Sn-Ag-Cu-Zn alloys, and combinations thereof. The low melting metal can have a melting point of up to 250°C, alternatively up to 225 °C. The low melting metal can have a melting point of at least 50 °C., alternatively at least 150 °C. The low melting metal can be a eutectic alloy, a non-eutectic alloy, an oxide, or a pure metal. Low melting metals are commercially available. The low melting metal may comprise at least about 0.1 up to about 20% of the weight of the TMM. These alloys, oxides or metals can also be separate layers or segregated portions of the TMM.

The thermal conductive fillers work most efficiently if they are in close contact or touching other thermally conductive or crystalline materials in order to facilitate a direct conductive pathway. Therefore the concentration, impurities, crystallinity, shape, size, compatibility and distribution of the fillers and other thermal materials is important.

The fillers can be 2-95% of the TMM. The amount of filler will depend on many factors such as compatibility,

effect on rheology, etc. The particle can be any shape such as round, spherical, cylindrical, fiber, sheet, flake, powder, whiskers, tubular, platelets, foams, meshes, agglomerates of these shapes or any other irregular shape. Depending on the application, a single shape or mixture of shapes may be advantageous to provide the optimum packing and arrangement for particle contact.

The particle can have any aspect ratio, from 1 for perfectly spherical particles to an infinite aspect ratio for nanomaterials. The particle size can be from 0.1 nanometer to 1 mm. The size and distribution of the particle will depend on the application such as nanometer sized particles allow for good dispersion, compatibility with good gap fill whereas large particles allow for lower concentration but provide for higher occurrence of contact between the particles for good thermal conductivity. Depending on the application, a single particle size or narrow particle size distribution may be advantageous for rheology optimization, whereas a mixture of particle sizes allows for most efficient packing distribution curve. For instance smaller average particles can fill the interstitial spaces between larger average particles to produce the optimum packing theory distribution curve which provides for efficient thermal transfer.

The particles can be of a monomolecular thickness, layer or size, such as the individual platelets or layers which make up bigger particles. As an example, graphene is one-atom-thick planar sheets of $sp^2$-bonded carbon atoms that are densely packed in a honeycomb crystal lattice. Graphene sheets stack to form graphite.

Thermal conductivity can be improved by using materials that will align or orient themselves to connect and provide a pathway from the electrochemical cell or pack to a cooler surface. For instance the use of fibers, whiskers, flakes, foams, or sheets can be used such that the materials when applied will align radially with respect to each cylindrical cell. Further examples are using fibers, flakes, foams, or sheets that have a length equal or greater than the TMM thickness so that the one end of the fiber, flake, foam, or sheet is touching the cell and the other end reaches to the surface or contacts the cooler surface, heat sink or heat spreader providing a constant unbroken single particle pathway. Alternatively, fibers, flakes, foams, or sheets could align longitudinally with multiple cells within a pack to move heat away from areas in between multiple cells. These types of fillers can also provide strength and reinforcement to the TMM to improve the tensile, adhesive or cohesive properties.

In order to provide optimum thermal conductivity, the fillers should have maximum purity with reduced included elemental materials. Purities greater than 95%, preferably >99% and most preferably >99.99999%. Higher purities also lead to higher material crystallinity with >50% crystallinity, preferably >90% and most preferably >99% crystalline. Reduced elemental impurities also means lower radioactive emissions with radioactive particle emissions lower than 0.001 counts/$cm^2 \cdot hr$, (i.e. positively charged alpha particle ($\alpha$), a positively or negatively charged beta particle ($\beta$), a photon or gamma particle, ($\gamma$), or a neutron and neutrinos).

In order to improve filler or particle compatibility with the TMM, electrochemical cells, or matrix material, it can be advantageous to surface treat the particle. This can also improve the rheology, viscosity and flow characteristics of the TMM. Surface treated particles can have improved compatibility with the binder, polymers or matrix material of the TMM which allows for complete coverage or surface wetting of the particle and therefore less air space or voids around the particle providing for improved thermal conductivity. Particle surface treatments can be by reaction with coupling agents, oxidation, acid, base, flame, functionalizing, etc. plasma oxidation, plasma sulfonation, ozone treatments in the presence or absence of ultraviolet light, alkaline surface hydrolysis, silanation, silanization and plasma ammonia treatment.

Coupling or surface treating agents can be any functional material such as fpPCMs, fpPCM with salt functionality, nonionic molecules with hydrophilic or hydrophobic moieties (i.e. aliphatic ethoxylates, alkyl ethoxylates, alkylphenoxy ethoxylates and their branched analogues), anionic or cationic molecules or polymers such as those containing carboxyl groups, containing amine neutralized carboxy groups, containing amine groups, acid neutralized amine groups etc. anhydrides and unsaturated polymeric acids or their salts and analogues (i.e. maleinized polybutadiene, maleinized or acid functional polyolefins, maleinized or acid functional poly (meth)acrylates, maleinized or acid functional polyesters, including metal or amine salts), alkoxysilanes, alkoxy-functional oligosiloxanes, alkylthiols, fatty acids, fatty acid metal salts, fatty acid amine salts, fatty amines, fatty amine salts, titanates, titanate coupling agents, zirconates, zirconate coupling agents, aluminates, aluminate coupling agents or mixtures thereof. Coupling agents such as those supplied by Kenrich Petrochemicals, Inc., Capaute Chemical, or Tyzor™ products from Dupont Inc., Dynasylan® silanes and organofunctional silanes from Evonik Degussa GmbH Germany, Dow Corning® "Z" silanes or Xiameter® "OFS" silanes from Dow Corning Corp.

Treating agents and treating methods are known in the art, see for example, U.S. Pat. No. 6,169,142 (col. 4, line 42 to col. 5, line 2). The TMM may comprise at least about 0.05% of a treating agent. The TIM may comprise up to about 10%, alternatively up to about 5%, alternatively up to about 0.5%, of a treating agent. The treating agent can be an alkoxysilane having the formula: $R^5_xSi(OR^6)_{(4-x)}$, where x is 1, 2, or 3; alternatively x is 3. $R^5$ is a substituted or unsubstituted monovalent hydrocarbon group of at least about 1 carbon atom, alternatively at least about 8 carbon atoms. $R^5$ has up to about 50 carbon atoms, alternatively up to about 30 carbon atoms, alternatively up to about 18 carbon atoms. $R^5$ is exemplified by alkyl groups such as hexyl, octyl, dodecyl, tetradecyl, hexadecyl, and octadecyl; and aromatic groups such as benzyl and phenylethyl. $R^5$ can be saturated or unsaturated, branched or unbranched, and

unsubstituted. $R^5$ can be saturated, unbranched, and unsubstituted.

$R^6$ is an unsubstituted, saturated hydrocarbon group of at least about 1 carbon atom. $R^6$ may have up to about 4 carbon atoms, alternatively up to about 2 carbon atoms. The treating agent is exemplified by hexyltrimethoxysilane, octyltriethoxysilane, decyltrimethoxysilane, dodecyltrimethyoxysilane, tetradecyltrimethoxysilane, phenylethyltrimethoxysilane, octadecyltrimethoxysilane, octadecyltriethoxysilane, a combination thereof, and others.

Alkoxy-functional oligosiloxanes can also be used as treatment agents. Alkoxy-functional oligosiloxanes and methods for their preparation are known in the art, see for example, EP 1 101 167 A2. For example, suitable alkoxy-functional oligosiloxanes include those of the formula $(R^6\ O)_aSi(OSi\ R^7{}_2R^8)$.sub.4-a. In this formula, a is 1, 2 or 3, alternatively a is 3. Each $R^6$ can be an alkyl group. Each $R^7$ is can be independently selected from unsaturated monovalent hydrocarbon groups of about 1 to about 10 carbon atoms. Each $R^8$ can be an unsaturated monovalent hydrocarbon group having at least about 11 carbon atoms.

Treatment agents for alumina or passivated aluminum nitride could include alkoxysilyl functional alkylmethyl polysiloxanes (e.g., partial hydrolysis condensate of $R^9{}_bR^{10}{}_cSi(O^{11})_{(4-b-c)}$ or cohydrolysis condensates or mixtures), similar materials where the hydrolyzable group would be silazane, acyloxy or oximo. In all of these, a group tethered to Si, such as $R^9$ in the formula above, is a long chain unsaturated monovalent hydrocarbon or monovalent aromatic-functional hydrocarbon. $R^{10}$ is a monovalent hydrocarbon group, and $R^{11}$ is a monovalent hydrocarbon group of about 1 to about 4 carbon atoms. In the formula above, b is 1, 2, or 3 and c is 0, 1, or 2, with the proviso that b+c is 1, 2, or 3. One skilled in the art could optimize a specific treatment to aid dispersion of the filler without undue experimentation.

Other specific examples of treating agents such as organo-functional silanes have the following typical molecular structure: $X\text{-}CH_2CH_2CH_2Si(OR)_{3-n}R'_n$ where n = 0, 1, 2. Many combinations are possible, but these are characterized by the fact that they contain two different types of reactive groups. The OR groups are hydrolyzable groups such as methoxy, ethoxy or acetoxy groups. The group X is an organo-functional group, such as epoxy, amino, methacryloxy (shown below), or sulfido. The presence of some Si-alkyl groups ensures low surface tension and good wetting properties. A typical example for sulfido-silanes: $(OR)_3Si\text{-}(CH_2)_3\text{-}S_x\text{-}(CH_2)_3Si(OR)_3$ where x = 2 to 8.

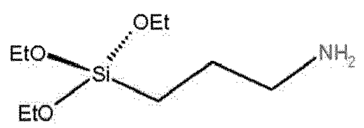

***Amino-silane***
γ-Aminopropyltriethoxysilane

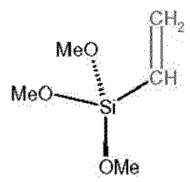

***Vinyl-silane***
Vinyltrimethoxysilane

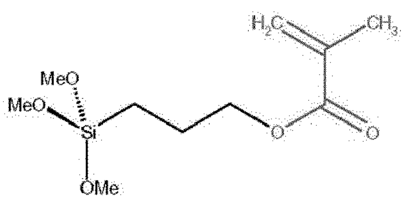

***Methacryloxy-silane***
γ-Methacryloxypropyltrimethoxysilane

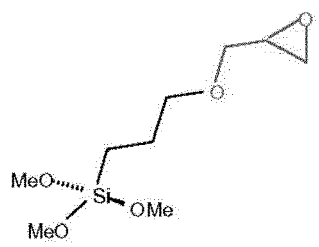

***Epoxy-silane***
γ-Glycidoxypropyltrimethoxysilane

Use of Fire Resistant Additives

In order to reduce the likelihood of fire spreading from an electrochemical cell to the components of a device it is powering, or to prevent fire spreading within an electrochemical pack, or to prevent an external fire from igniting an electrochemical pack, a fire retardant can be added to prevent combustion. Materials such as Magnesium hydroxide, Al hydroxide, expanded graphite, ammonium polyphosphate, phosphate salts, polyphosphate salts, wherein the polyphosphate can be any mol. wt. or degree of polymerization, pentaerythritol, treated montmorillonite, halogenated compounds, ammonium bromide, chlorinated or brominated molecules, alkanes and polymers, antimony oxide, antimony trioxide, red phosphorous, magnesia cements, magnesium oxysulphate, magnesium phosphate, magnesium sulphate, boron compounds, borates, boric acid, silicon and silica compounds, melamine and melamine compounds, sol-gels, sodium carbonate, sodium silicate, tetrakis(hydroxymethyl)phosphonium salts, halocarbons, including chlorendic acid

derivates, halogenated phosphorus compounds including tri-o-cresyl phosphate, tris(2,3-dibromopropyl)phosphate (TRIS), bis(2,3-dibromopropyl)phosphate, tris(1-aziridinyl)-phosphine oxide (TEPA), phosphoramides, triphenyl phosphate (TPP), resorcinol diphosphate (RDP), bisphenol-a-disphosphate (BPA-DP), organic phosphine oxides, halogenated epoxy resin (tetrabromobisphenol A), etc. including their blends or mixtures.

Additionally, fire retardant additives can include ceramic type treatments and coatings based on silica, quartz, or other ceramics, as disclosed in U.S. Patent No. 6,921,431 to Evans, "Thermal Protective Coating for Ceramic Surfaces," U.S. Patent No. 7,105047, to Simmons, "Thermal Protective Coating," and non-patent publications by Rakotomalala et. al., entitled "Recent Developments in Halogen Free Flame Retardants for Epoxy Resins for Electrical and Electronic Applications," Materials 2010, 3, 4300-4327, the non-patent publication by the Phosphorous, Inorganic and Nitrogen Flame Retardants Association entitled "Innovative Flame Retardants in E&E Applications," June 2009, and the non-patent publication by Feldman, entitled "Polymer Nanocomposites: Flammability," Journal of Macromolecular Science, Part A: Pure and Applied Chemistry, 2013, which are incorporated herein by reference. They can also include ionic materials as disclosed in US Patent Application Nos. 12/947,377 and 12/806,267 to Xu, both entitled "Ionic Liquid Flame Retardants," and which are incorporated herein by reference.

Another example is to have the fire retardant material as part of the microcapsule shell. For instance, melamine or melamine compounds provide excellent microcapsule shells and also provide good fire retardant properties. Sol-gels can also be used as mPCM shells or secondary shells to give additional fire retardant protection. Any shell material, shell polymer or secondary shell layer can also incorporate any of the above fire retardant additives to infer improved fire retardant properties to the TMM.

Use of Electrically Conducting and Static Charging/Discharging Materials and Polymers.

In certain embodiments of the disclosure, it may also be advantageous to include materials designed to control electrical energy or static energy. This electrical energy can either be conducted, discharged, dissipated, stored or other means of moving this energy. As discussed earlier in this disclosure, electrical shorts can damage various components of a battery cell, and in some circumstances it may be desirable to keep electrical energy away from the walls of the cell. Metallic materials, such as previously described above, can include any metal, alloy, oxide, etc. These can also be in any shape or size as previously outlined. Many of the previously described thermal conductors can also be used as electrical conductors. Organic or organic doped materials can also be included in the TMM package. These materials can include the general polymer structures shown below:

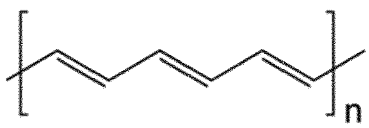

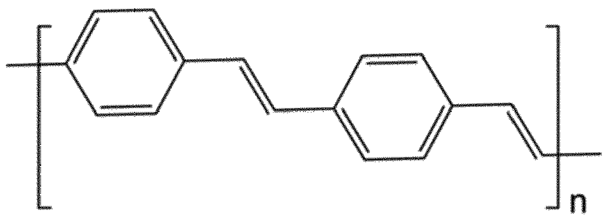

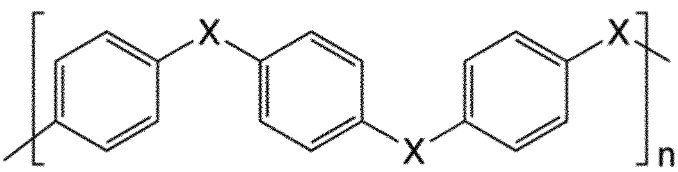

(X = NH/N, S)

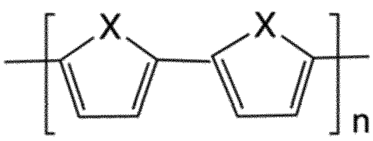

(X = NH, S)

Chemical structures of some conductive polymers.

From top left clockwise: polyacetylene; polyphenylene vinylene;
polypyrrole (X = NH) and polythiophene (X = S);
and polyaniline (X = NH/N) and polyphenylene sulfide (X = S)

These and other general classes of conducting polymers can be broken down as in the below table:

| | No heteroatom | Nitrogen-containing | Sulfur -containing |
|---|---|---|---|
| Aromatic main chain | Poly(fluorene)s<br>Polyphenylenes<br>Polypyrenes<br>Polyazulenes<br>polynaphthalenes | Poly(pyrroles)s (PPY)<br><br>Poly(*o*-aminophenols) (POAP)<br><br>Polycarbazoles<br>Polyindoles<br>Polyazepines<br>Polypyridines<br>Polyanilines (PANI) | Poly(thiophene)s (PT)<br>Poly(3,4-ethylenedioxythiophene) (PEDOT)<br><br>Poly(p-phenylene sulfide) (PPS) |
| Double bonds in main chain | Poly(acetylene)s (PAC) | | |
| Aromatic and Double Bonds | Poly(p-phenylene vinylene) (PPV) | | |

More specifically, various materials, polymers and monomers can be further broken down into more specific charge or radiation conducting materials. The various combinations and mixtures of materials using below example materials is infinite and the below examples are not complete or inclusive. Infinite combinations can produce various polymers, copolymers, mixtures, etc. that can have electrically conducting and static dissipating properties. These polymers can be doped with any combination of the infinite number of salts or metallic salts to enhance the conductivity:

Photosensitizing and charge transport compounds such as 1,1,4,4-Tetraphenyl-1,3 butadiene, 4-[2-[5-[4-(Diethylamino)phenyl]-4,5-dihydro-1-phenyl-1*H*-pyrazol-3-yl]ethenyl]-*N*,*N*-diethylaniline, 5,12-Bis(phenylethynyl)naphthacene, 9,10-Bis(phenylethynyl)anthracene, 9,10-Di-*p*-tolylanthracene, 9,10-Phenanthrenequinone, Benzo[*ghi*]perylene, Coronene, Julolidine, Pentaphene-78, Perylene-66, Phenanthrene, Phenanthridine, Phenazine, Phenothiazine, Pyrazole-72, Quinacridonequinone, Quinolin-65, Thioxanthone-64, Triphenylene, Violanthrone-79, [4-[Bis(2-hydroxyethyl)amino]phenyl]-1,1,2-ethylenetricarbonitrile.

Light Emitters, Dopants, electron & hole transporting materials such as: 5,12-Dihydro-5,12-dimethylquino[2,3-b]acridine-7,14-dione, 8-Hydroxyquinoline zinc, Anthracene, Benz[b]anthracene, Coumarin 6, Dichlorotris(1,10-phenanthroline)ruthenium(II) hydrate, Lithium tetra(2-methyl-8-hydroxyquinolinato)boron, Perylene, Platinum octaethylporphyrin, Rubrene, Tris(2,2'-bipyridyl)dichlororuthenium(II) hexahydrate, Tris(2,2'-bipyridyl-d8)ruthenium(II) hexafluorophosphate, Tris(benzoylacetonato) mono(phenanthroline)europium(III), Tris(dibenzoylmethane) mono(1,10-phenanthroline)europium(lll), Tris(dibenzoylmethane) mono(5-amino-1,10-phenanthroline)europium (111), Tris-(8-hydroxyquinoline)aluminum, Tris[1-phenylisoquinoline-C2,N]iridium(III), Tris[2-(4,6-difluorophenyl)pyridinato-C2,N]iridium(III), Tris[2-(benzo[b]thiophen-2-yl)pyridinato-C3,N]iridium(III), Tris[2-phenylpyridinato-C2,N]iridium(III), Metal and salt dopants.

Light Emitting Polymers such as: Cyano-Polyphenylene vinylene (CN-PPV) Polymers including: Poly(2,5-di(hexyloxy)cyanoterephthalylidene), Poly(2,5-di(octyloxy)cyanoterephthalylidene), Poly(2,5-di(3,7-dimethyloctyloxy)cyanoterephthalylidene), Poly(5-(2-ethylhexyloxy)-2-methoxy-cyanoterephthalylidene), Poly(5-(3,7-dimethyloctyloxy)-2-methoxy-cyanoterephthalylidene).

Nitrogen-Containing Polymers including: Poly(2,5 pyridine) and Poly(3,5 pyridine).

Poly(fluorenylene ethynylene) (PFE) Polymers including: Poly(9,9-dioctylfluorenyl-2,7-yleneethynylene), Poly[9,9-di(3',7'-dimethyloctyl)fluoren-2,7-yleneethynylene], Poly[9,9-di(2'-ethylhexyl)fluoren-2,7-yleneethynylene], Poly[9,9-didodecylfluroenyl-2,7-yleneethylnylene].

Poly(phenylene ethynylene) (PPE) Polymers including: Poly(2,5-di(3',7'-dimethyloctyl)phenylene-1,4-ethynylene), Poly(2,5-dicyclohexylphenylene-1,4-ethynylene), Poly(2,5-di(2'-ethylhexyl)-1,4-ethynylene), Poly(2,5-didodecylphenylene-1,4-ethynylene), and Poly(2,5-dioctylphenylene-1,4-ethynylene).

Polyfluorene (PFO) Polymers and Co-Polymers including: Poly(9,9-di-n-dodecylfluorenyl-2,7-diyl), Poly(9,9-di-n-hexylfluorenyl-2,7-diyl), Poly(9,9-di-n-octylfluorenyl-2,7-diyl), Poly(9,9-n-dihexyl-2,7-fluorene-alt-9-phenyl-3,6-carbazole), Poly[(9,9-di-n-octylfluorenyl-2,7-diyl)-alt-(benzo[2,1,3]thiadiazol-4,8-diyl)], Poly[(9,9-dihexylfluoren-2,7-diyl)-alt-(2,5-dimethyl-1,4-phenylene)], Poly[(9,9-dihexylfluoren-2,7-diyl)-co-(9-ethylcarbazol-2,7-diyl)], Poly[(9,9-dihexylfluoren-2,7-diyl)-co-(anthracen-9,10-diyl)], Poly[(9,9-dioctylfluorenyl-2,7-diyl)-co-bithiophene], Poly[9,9-bis-(2-ethylhexyl)-9H-fluorene-2,7-diyl].

Polyfluorene-Vinylene (PFV) Co-Polymers including: Poly((9,9-dihexyl-9H-fluorene-2,7-vinylene)-co-(1-methoxy-4-(2-ethylhexyloxy)-2,5-phenylenevinylene)), Poly(9,9-di-(2-ethylhexyl)-9H-fluorene-2,7-vinylene), Poly(9,9-di-n-hexylfluorenyl-2,7-vinylene), Poly[(9,9-di-(2-ethylhexyl)-9H-fluorene-2,7-vinylene)-co-(1-methoxy-4-(2-ethylhexyloxy)-2,5-phenylenevinylene)], and Poly[9-(2-ethylhexyl)-3,6-carbazolevinylene-alt-2,6-naphthalenevinylene].

Polyphenylene Vinylene (PPV) Polymers and Co-Polymers including: Poly(1-methoxy-4-(3-propyloxy-heptaisobutyl-PSS)-2,5-phenylenevinylene)-co-(1-methoxy-4-(2-ethylhexyloxy)-2,5-phenylenevinylene) (60:40), Poly(1-methoxy-4-(O-disperse Red 1))-2,5-phenylenevinylene, Poly(2,5-bis(1,4,7,10-tetraoxaundecyl)-1,4-phenylenevinylene), Poly(2,5-dihexyloxy-1,4-phenylenevinylene), Poly(2,5-dioctyl-1,4-phenylenevinylene), Poly(2,6-naphthalenevinylene), Poly(p-xylene tetrahydrothiophenium chloride), Poly[(m-phenylenevinylene)-alt-(2,5-dibutoxy-p-phenylenevinylene)], Poly[(m-phenylenevinylene)-alt-(2,5-dihexyloxy-p-phenylenevinylene)], Poly[(m-phenylenevinylene)-alt-(2-methoxy-5-(2-ethylhexyloxy)-p-phenylenevinylene)], Poly[(m-phenylenevinylene)-alt-(2-methoxy-5-octyloxy-p-phenylenevinylene)], Poly[(m-phenylenevinylene)-co-(2,5-dioctoxy-p-phenylenevinylene)], Poly[(o-phenylenevinylene)-alt-(2-methoxy-5-(2-ethylhexyloxy)-p-phenylenevinylene)], Poly[(p-phenylenevinylene)-alt-(2-methoxy-5-(2-ethylhexyloxy)-p-phenylenevinylene)], Poly[1-methoxy-4-(3-propyloxy-heptaisobutyl-PSS)-2,5-phenylenevinylene], Poly[1-methoxy-4-(3-propyloxy-heptaisobutyl-PSS)-2,5-phenylenevinylene]-co-[1-methoxy-4-(2-ethylhexyloxy)-2, 5-phenylenevinylene] (30:70), Poly[2,5-bis(3',7'-dimethyloctyloxy)-1,4-phenylenevinylene], Poly[2,5-bisoctyloxy)-1,4-phenylenevinylene], Poly[2-(2',5'-bis(2"-ethylhexyloxy)phenyl)-1,4-phenylenevinylene], Poly[2-methoxy-5-(2-ethylhexyloxy)-1,4-phenylenevinylene], Poly[2-methoxy-5-(3',7'-dimethyloctyloxy)-1,4-phenylenevinylene], Poly[5-methoxy-2-(3-sulfopropoxy)-1,4-phenylenevinylene] potassium salt, Poly[tris(2,5-bis(hexyloxy)-1,4-phenylenevinylene)-alt-(1,3-phenylenevinylene)], and Poly{[2-[2',5'-bis(2"-ethylhexyloxy )phenyl]-1,4-phenylenevinylene]-co-[2-methoxy-5-(2'-ethylhexyloxy)-1,4-phenylenevinylene]}.

Polythiophene Polymers and Co-Polymers (regioregular or regiorandom stearic configurations) including: Poly(3-(2-methoxyethoxy)ethoxymethylthiophene-2,5-diyl), Poly(3,4-ethylenedioxythiophene), Poly(3-butylthiophene-2,5-diyl), Poly(3-cyclohexyl-4-methylthiophene-2,5-diyl), Poly(3-cyclohexylthiophene-2,5-diyl), Poly(3-decyloxythiophene-2,5-diyl), Poly(3-decylthiophene-2,5-diyl), Poly(3-dodecylthiophene-2,5-diyl), Poly(3-hexylthiophene-2,5-diyl), Poly(3-octylthiophene-2,5-diyl), Poly(3-octylthiophene-2,5-diyl-co-3-decyloxythiophene-2,5-diyl), Poly(thiophene-2,5-diyl), bromine terminated, Poly[(2,5-didecyloxy-1,4-phenylene)-alt-(2,5-thienylene)].

Water-Soluble light emitting polymers including: Poly(2,5-bis(3-sulfonatopropoxy)-1,4-phenylene, disodium salt-alt-1,4-phenylene), Poly[(2,5-bis(2-(N,N-diethylammonium bromide)ethoxy)-1,4-phenylene)-alt-1,4-phenylene], Poly[5-methoxy-2-(3-sulfopropoxy)-1,4-phenylenevinylene] potassium salt, Poly{[2,5-bis(2-(N,N-diethylamino)ethoxy)-1,4-phenylene]-alt-1,4-phenylene}

Polymer Hole Transport and Host Materials including Polyvinyl polymers such as: Poly(1-vinylnaphthalene), Poly(2-vinylcarbazole), Poly(2-vinylnaphthalene), Poly(9-vinylcarbazole), and Poly(N-ethyl-2-vinylcarbazole).

Conducting polymers, copolymers and monomers including Polyacetylenes such as Poly[1,2-bis(ethylthio)acetylene], Poly(phenylene sulfides), Polyanilines, copolymers and Polyaniline Dopants including Camphor-10-sulfonic acid (β), Dinonylnaphthalenesulfonic acid, Dodecylbenzenesulfonic acid, Polyaniline (emeraldine base, emeraldine salt, leucoemeraldine base, nigraniline, or pernigraniline), *o*-ethoxyaniline, *ortho* & *meta* mono- and di-substituted anilines, *ortho or meta*-aminoacetophenone, and *m*-toluidine.

Polypyrroles and Pyrrole Monomers including: 1H-Pyrrole-1-propanoic acid, 3,4-Ethylenedioxypyrrole-2,5-dicarboxylic acid, 3,4-Ethylenedioxypyrrole, 3,4-Propylenedioxypyrrole, 4-(3-Pyrrolyl)butyric acid, Diethyl 1-benzyl-3,4-ethylenedioxypyrrole-2,5-dicarboxylate, and Polypyrrole (conductive, doped or undoped).

Polythiophenes and Thiophene Monomers including: 3,4-Ethylenedioxythiophene, Poly(3,4-ethylenedioxythiophene), bis-poly(ethyleneglycol), lauryl terminated, Poly(3,4-ethylenedioxythiophene), tetramethacrylate end-capped, Poly(3,4-ethylenedioxythiophene)-block-poly(ethylene glycol), Poly(3,4-ethylenedioxythiophene)-poly(styrenesulfonate), Poly(3,4-ethylenedioxythiophene), Poly(thiophene-3-[2-(2-methoxyethoxy)ethoxy] -2,5 -diyl).

Sulfonated versions of Polyfluorenes, Poly(*o*-aminophenols) (POAP), Polytetrathiafulvalenes, Polynapththalenes, Poly (para-phenylene vinylene)s, natural or Biological Melanin pigment polymers.

Organic Photovoltaic materials including: Conducting Materials such as 5,5‴″-Dihexyl-2,2′:5′,2″:5″,2‴:5‴,2″″:5″″,2‴″-sexithiophene, Copper(II) 1,2,3,4,8,9,10,11,15,16,17,18,22,23,24,25-hexadecafluoro-29H,31H-phthalocyanine, Copper(II) phthalocyanine, Fullerene-$C_{60}$, Fullerene-$C_{84}$, Pentacene, Perylene-3,4,9,10-tetracarboxylic dianhydride, Perylene, Poly(3-dodecylthiophene-2,5-diyl), Poly(3-hexylthiophene-2,5-diyl) regioregular, Poly(3-octylthiophene-2,5-diyl) regioregular, Poly[2-methoxy-5-(3',7'-dimethyloctyloxy)-1,4-phenylenevinylene], Tris[4-(5-dicyanomethylidenemethyl-2-thienyl)phenyl]amine, [5,6]-Fullerene-$C_{70}$, [6,6]-Pentadeuterophenyl $C_{61}$ butyric acid methyl ester, [6,6]-Phenyl $C_{61}$ butyric acid methyl ester, [6,6]-Phenyl $C_{71}$ butyric acid methyl ester, [6,6]-Phenyl $C_{85}$ butyric acid methyl ester, [6,6]-Thienyl $C_{61}$ butyric acid methyl ester, [6.6] Diphenyl $C_{62}$ bis(butyric acid methyl ester), and α-Sexithiophene.

Dyes including 1,3-Bis[4-(dimethylamino)phenyl]-2,4-dihydroxycyclobutenediylium dihydroxide, bis(inner salt), 7-Methylbenzo[a]pyrene, 9,10-Dihydrobenzo[a]pyrene-7(8H)-one, Benzo[e]pyrene, Coumarin 102, Coumarin 153, Coumarin 30, Coumarin 480 D, Coumarin 6, Merocyanine 540 and Pyrene.

Particles and their doped, undoped, various crystal forms, and mixtures with metals including: Titanium oxide, Titanium dioxide, $Titanium^{+4}$ oxides and dioxides, and Zinc oxide.

Organic Semi-conductors including n-Type Oligomers and Polymers such as 1,4,5,8-Naphthalenetetracarboxylic dianhydride , 2,3,5,6-Tetrafluoro-7,7,8,8-tetracyanoquinodimethane, 5,10,15,20-Tetrakis(pentafluorophenyl)-21H,23H-porphine palladium(II), 7,7,8,8-Tetracyanoquinodimethane, Copper(II) 1,2,3,4,8,9,10,11,15,16,17,18,22,23,24,25-hexadecafluoro-29H,3 1H-phthalocyanine, Fullerene-$C_{60}$, Fullerene-$C_{84}$, N,N'-Dioctyl-3,4,9,10-perylenedicarboximide, N,N'-Dipentyl-3,4,9,10-perylenedicarboximide, N,N'-Diphenyl-3,4,9,10-perylenedicarboximide, N,N'-Bis(2,5-di-tert-butylphenyl)-3,4,9,10-perylenedicarboximide, Perylene-3,4,9,10-tetracarboxylic dianhydride, [5,6]-Fullerene-$C_{70}$, [6,6]-Phenyl $C_{61}$ butyric acid methyl ester, [6,6]-Phenyl $C_{71}$ butyric acid methyl ester, [6,6]-Phenyl $C_{85}$ butyric acid methyl ester, [6,6]-Phenyl-$C_{61}$ butyric acid butyl ester, [6,6]-Phenyl-$C_{61}$ butyric acid octyl ester, [6,6]-Thienyl $C_{61}$ butyric acid methyl ester, [6.6] Diphenyl $C_{62}$ bis(butyric acid methyl ester), Poly(2,5-di(hexyloxy)cyanoterephthalylidene), Poly(2,5-di(octyloxy)cyanoterephthalylidene), Poly(2,5-di(3,7-dimethyloctyloxy)cyanoterephthalylidene), Poly(5-(2-ethylhexyloxy)-2-methoxy-cyanoterephthalylidene), Poly(5-(3,7-dimethyloctyloxy)-2-methoxy-cyanoterephthalylidene), Poly(benzimidazobenzophenanthroline), Poly[(1,4-divinylenephenylene)(2,4,6-triisopropylphenylborane)], and Poly[(2,5-didecyloxy-1,4-phenylene) (2,4,6-triisopropylphenylborane)], diphenyl terminated.

p-Type Oligomers and Polymers including: 13,6-N-Sulfinylacetamidopentacene, 2,2′:5′,2″:5″,2‴-Quaterthiophene, 3,3‴-Didodecyl-2,2′:5′,2″:5″,2‴-quaterthiophene, 3,3‴-Dihexyl-2,2′:5′,2″:5″,2‴-quaterthiophene,5,5⁗′-Dihexyl-2,2′:5′,2″:5″,2‴:5‴,2⁗:5⁗,2⁗′-sexithiophene,5,5'-Di(4-biphenylyl)-2,2'-bithiophene, 5,5'-Dihexyl-2,2'-bithiophene, 6,13-Dihydro-6,13-methanopentacene-15-one, Benz[b]anthracene, Benz[b]anthracene, Bis(ethylenedithio)tetrathiafulvalene, Copper(II) phthalocyanine, Coronene purified by sublimation, Dibenzotetrathiafulvalene, Pentacene, Pentacene-N-sulfinyl-n-butylcarbamate adduct, Pentacene-N-sulfinyl-tert-butylcarbamate, Platinum octaethylporphyrin, Rubrene, Tetrathiafulvalene, Tris[4-(5-dicyanomethylidenemethyl-2-thienyl)phenyl]amine, $\alpha$-Sexithiophene, Poly(3-dodecylthiophene-2,5-diyl) regiorandom or regioregular, Poly(3-hexylthiophene-2,5-diyl) regiorandom or regioregular, Poly(3-octylthiophene-2,5-diyl) regiorandom or regioregular, Poly[(9,9-din-octylfluorenyl-2,7-diyl)-alt-(benzo[2,1,3]thiadiazol-4,8-diyl)],Poly[(9,9-dioctylfluorenyl-2,7-diyl)-co-bithiophene], Poly[2-methoxy-5-(2-ethylhexyloxy)-l,4-phenylenevinylene], Poly[2-methoxy-5-(3′,7′-dimethyloctyloxy)-1,4-phenylenevinylene]

Use of Other Additives

Suitable antioxidants are known in the art and are commercially available. Suitable antioxidants include phenolic antioxidants and combinations of phenolic antioxidants with stabilizers. Phenolic antioxidants include fully sterically hindered phenols and partially hindered phenols. Stabilizers include organophosphorous derivatives such as trivalent organophosphorous compound, phosphites, phosphonates, and a combination thereof; thiosynergists such as organosulfur compounds including sulfides, dialkyldithiocarbamate, dithiodipropionates, and a combination thereof; and sterically hindered amines such as tetramethyl-piperidine derivatives. Suitable antioxidants and stabilizers are disclosed in Zweifel, Hans, "Effect of Stabilization of Polypropylene During Processing and Its Influence on Long-Term Behavior under Thermal Stress," Polymer Durability, Ciba-Geigy AG, Additives Division, CH-4002, Basel, Switzerland, American Chemical Society, Vol. 25, pp. 375-396, 1996. Suitable antioxidants are supplied by BASF Corp. under the Irgafos® and Irganox® tradenames. Antioxidants supplied by Chemtura Corp. under the Naugalube® and Naugard® tradenames. Stabilizers and antioxidants supplied by Nanjing Union Rubber and Chemicals Co., Ltd.

Suitable phenolic antioxidants include vitamin E, IRGANOX B225 and IRGANOX 1010 from BASF Corp., IRGANOX 1010 comprises pentaerythriol tetrakis(3-(3,5-di-t-butyl-4-hydroxyphenyl)propionate).

Reaction catalyst inhibitors are known in the art and commercially available, see for example, U.S. Pat. No. 5,929,164 (col. 1, line 65 to col. 3, line 65). Inhibitors can be a phosphine, a diphosphine, an amine, a diamine, a triamine, an organic sulfide, an alkenyl-functional compound, an alkynyl-functional compound, a hydroxy-functional compound, a combination thereof, or any other transition metal catalyst poison.

Suitable phosphines include trialkyl phosphines and triaryl phosphines such as triphenyl phosphine. Suitable diphosphines include tetraphenylethylene diphosphine. Suitable amines include n-butyl amine and triethanolamine. Suitable diamines include tetramethylenediamine. Suitable organic sulfides include ethyl phenyl sulfide. Suitable alkenyl-functional compounds can be organic, organosilicones, or organosilanes. Suitable alkenyl-functional compounds include vinylsiloxanes and vinylsilanes. Suitable alkynyl functional compounds can be organic, such as acetylene, propyne, 1-butyne, 1-pentyne, 4,4-dimethyl-1-pentyne, 1-hexyne, 5-methyl-1-hexyne, and 1-decyne.

Monomers and Polymers Suitable for Forming Reactive Diluents that are Curable into pPCMs and fpPCMs

Certain monomers and polymers can form liquid reactive diluents at low mol. wt. versions, and can form pPCMs or fpPCMs upon drying and/or curing. Examples of these are listed in the following table:

TABLE 4

Long Chain Group Containing Monomers

| R = | Monomers |
|---|---|
| Long chain n-alkyl crystallizible segments | $CH_2{=}CR'CO_2(CH_2)_nCH_3$ R' = $CH_3$ or H, n = 10-25<br>Long chain n-alkyl acrylates or methacrylates such as stearyl acrylate or stearyl methacrylate,<br>$CH_2{=}CH{-}O{-}(CH_2)_nCH_3$, n = 10-25<br>Long chain n-alkyl vinyl ethers such as stearyl vinyl ether,<br>$CH_2{=}CH{-}O{-}CO{-}(CH_2)_nCH_3$, n = 10-25,<br>Long chain n-alkyl vinyl esters such as vinyl stearate,<br>$CH_2{=}CH{-}(CH_2)_nCH_3$, n = 4-25<br>Long chain n-alkyl olefins such as undecene,<br>or any other long chain n-alkyl containing unsaturated polymerizable monomer |
| Long chain crystallizible glycol segments | $CH_2{=}CR'CO_2(CH_2CH_2O)_nOX$ R' = $CH_3$ or H, n = 1-1,000, X = $CH_3$ or H<br>Glycol based acrylates or methacrylates such as polyethyleneglycol methacrylate, polyethyleneglycol acrylate,<br>$CH_2{=}CH{-}(CH_2)_m{-}O{-}((CH_2)_nO)_zOX$ m = 0-4, n = 1-10, z = 1-1000, X = $CH_3$ or H<br>Glycol based vinyl ethers such as polyethyleneglycol monovinyl ether<br>$CH_2{=}CH{-}O{-}CO{-}((CH_2)_nO)_xOX$ n = 1-10, z = 1-1000, X = $CH_3$ or H<br>glycol based vinyl esters such as polyethyleneglycol monovinyl ester<br>or any unsaturated polymerizable hydroxyl functional monomer |

Additionally, certain pPCMs and fpPCMS can be made into curable diluents themselves. Examples of these include the homopolymers and polyester monomers listed in the following table:

TABLE 1

| Class of Monomer Unit | Homopolymer |
|---|---|
| Alkanes and Alkenes | Poly-1-decene |
| | Poly-1-heptene |
| | cis-polyoctenamer (Vestenamer ® 6213, available from Degussa AG, Frankfurt, Germany) |
| | Poly-1-octene |
| | Poly-1-nonene |
| | trans-polypentemer |
| | Poly-1-undecene |
| | cis-polyisoprene |
| | syndiotactic 1,2-poly(1,3-pentadiene) |
| | 1-methyl-polydodecamethylene |
| Ethers | Polymethyleneoxytetramethylene oxide (Poly-1,3-dioxepane) |
| | Polyhexamethyleneoxymethylene oxide |
| | Polyoxacyclobutane (POX) |
| | n-octadecyl polyacetaldehyde |
| Sulfur Containing Compounds | 3,3-dimethyl-polytrimethylene sulfide |
| | Polymethylene sulfide |
| | Polytetramethylene disulfide |
| | Sulfurtrioxide |
| | 1-methyl-trimethylene-poly-sulfonyldivalerate |
| Silicon Containing Compounds | beta-2-polydiethyl siloxane |
| | Nonamethylene-poly-disiloxanylene dipropionamide-diethyl, dimethyl (Si) |
| | Nonamethylene-poly-disiloxanylene dipropionamide-tetraethyl (Si) |
| | Polymethyl hexadecyl siloxane |
| Amides and Nitrogen Containing Compounds | Poly-(hexamethylene)cyclopropylene dicarboxamide-cis-N,N'-dibutyl |
| | Poly-(hexamethylene)cyclopropylene dicarboxamide-cis-N,N'-diethyl |
| | Poly-(hexamethylene)cyclopropylene dicarboxamide-cis-N,N'-diisopropyl |
| | Poly-(hexamethylene)cyclopropylene dicarboxamide-cis-N,N'-dimethyl |
| | Polypentamethylene adipamide-2,2,3,3,4,4 hexafluoro (diamine)-cis-N,N'-dibutyl |
| | Polypentamethylene adipamide-2,2,3,3,4,4 hexafluoro (diamine)-cis-N,N'-diethyl |
| | Polypentamethylene adipamide-2,2,3,3,4,4 hexafluoro (diamine)-cis-N,N'-diisopropyl |
| | Polypentamethylene adipamide-2,2,3,3,4,4 hexafluoro (diamine)-cis N,N'-dimethyl |
| | Poly-(4,4'-methylene diphenylene sebacamide)-N,N'-diethyl |
| | Polypentamethylene (hexamethylene disulfonyl)-dicaproamide |
| Esters | Poly-[ethylene 4,4'-oxydimethylene-di-2-(1,3-dioxolane)-caprylate] |
| | Polypentamethylene adipate-2,2,3,3,4,4 hexa fluoro |
| | (4-methyl-(R+)-7-polyhydroxyenanthic acid) |
| | Poly-[4-hydroxy tetramethylene-2-(1,3-dioxolane) caprylic acid] (cis or trans) |
| | Polypentamethylene 2,2'-dibenzoate |
| | Polytetramethylene 2,2'-dibenzoate |
| | Poly-1-methyl-trimethylene 2,2' dibenzoate |
| | Polycaprolactone glycol (Molecular weight = 830) |

| Glycol | Diacid |
|---|---|
| Ethylene glycol | Carbonic |
| Ethylene glycol | Pimelic |
| Ethylene glycol | Diglycolic |
| Ethylene glycol | Thiodivaleric |
| 1,2-Propylene glycol | Diglycolic |
| Propylene glycol | Malonic |
| Propylene glycol | Glutaric |
| Propylene glycol | Diglycolic |
| Propylene glycol | Pimelic |
| 1,3-butanediol | Sulphenyl divaleric |
| 1,3-butanediol | Diphenic |
| 1,3-butanediol | Diphenyl methane-m,m'-diacid |
| 1,3-butanediol | trans-H,H-terephthalic acid |
| Butanediol | Glutaric |
| Butanediol | Pimelic |
| Butanediol | Azelaic |
| Butanediol | Thiodivaleric |
| Butanediol | Phthalic |
| Butanediol | Diphenic |
| Neopentyl glycol | Adipic |
| Neopentyl glycol | Suberic |
| Neopentyl glycol | Sebacic |
| Pentanediol | Succinic |
| Pentanediol | Glutaric |
| Pentanediol | Adipic |
| Pentanediol | Pimelic |
| Pentanediol | para-phenyl diacetic acid |
| Pentanediol | Diglycolic |
| Hexanediol | Glutaric |
| Hexanediol | 4-Octenedioate |
| Heptanediol | Oxalic |
| Octanediol | 4-Octenedioate |
| Nonanediol | meta-phenylene diglycolic |
| Decanediol | Malonic |
| Decanediol | Isophthalic |
| Decanediol | meso-tartaric |
| Diethylene glycol | Oxalic |
| Diethylene glycol | Suberic |
| Diethylene glycol | Sebacic |
| Diethylene glycol | Phthalic |
| Diethylene glycol | trans-H,H-terephthalic acid |
| Triethylene glycol | Sebacic |
| Triethylene glycol | Sulphonyl divaleric |
| Triethylene glycol | Phthalic |
| Triethylene glycol | Diphenic |
| para-dihydroxy-methyl benzene | Malonic |
| meta-dihydroxy-methyl benzene | Sebacic |
| meta-dihydroxy-methyl benzene | Diglycolic |

Compositions Containing Multiple Radiation-Curable Monomers and Radiation-Curable Compounds

Radiation curable monomers for use in reaction mixtures can be obtained by a reaction of acrylic acid with polyols, alkoxylated polyols or polyesterols in a molar ratio of (hydroxy groups : acrylic acid in the esterification). For example, ratios in accordance with embodiments of the present disclosure could be [1 : 0.75 - 2.5], [1 : 0.8 - 2], [1 : 0.9 - 1 .5], or [1 : 1 - 1 .2].

Polyols may include trimethylolbutane, trimethylolpropane, trimethylolethane, neopentylglycol, pentaerythritol, ethylene glycol, 1,2-propanediol, 1,3-propanediol, 1,1-dimethylethane-1,2-diol, 2-butyl-2-ethyl-1,3-propanediol, 2-ethyl-1,3-propanediol, 2-methyl- 1,3-propanediol, neopentyl glycol, neopentyl glycol hydroxypivalate, 1,2-,1,3- or 1,4-butanediol, 1,6-hexanediol, 1,8-octanediol, 1,10-decanediol, 2-ethyl-1,3-hexanediol, 2-propyl-1,3-heptane-diol, and 2,4-diethyloctane-1,3-diol diethylene glycol, triethylene glycol, dipropylene glycol, tripropylene glycol.

Alkoxylated polyols include, for example, the above-mentioned polyols, such as pentaerythritol, trimethylolethane or trimethylolpropane with from single to 20-fold ethoxylation, propoxylation or mixed ethoxylation and propoxylation.

Polyesterols may have a molar mass Mn of 1000 to 4000 g/mol and may be synthesized from the above-recited polyols or alkoxylated polyols with di- or higher carboxylic acids.

Polyesters are synthesized from aliphatic diols such as ethylene glycol, 1,2-propanediol, 1,3-propanediol, 1,1-dimethylethane-1,2-diol, 2-butyl-2-ethyl-1,3-propanediol, 2-ethyl-1,3-propanediol, 2-methyl-1,3-propanediol, neopentyl glycol, neopentyl glycol hydroxypivalate, 1,2-,1,3- or 1,4-butanediol, 1,6-hexanediol, 1,10-decanediol, 2-ethyl-1,3-hexanediol, 2-propyl-1,3-heptanediol, and 2,4-diethyloctane-1,3-diol and dicarboxylic acids, such as oxalic acid, malonic acid, succinic acid, glutaric acid, adipic acid, pimelic acid, suberic acid, azelaic acid, sebacic acid, undecane-a,co-dicarboxylic acid, dodecane-a,co-dicarboxylic acid, phthalic acid, isophthalic acid, terephthalic acid, 1,2-cyclohexanedicarboxylic acid, 1,4-cyclohexanedicarboxylic acid, 1,3-cyclohexanedicarboxylic acid, and 4-methyl-1,3-cyclohexanedicarboxylic acid.

Radiation curable monomers for use in reaction mixtures can also be obtained by reaction of glycidyl ethers with carboxylic acids such as acrylic acid, or with hydroxy compounds that may be present. The glycidyl ethers can be of a pure form or optionally as a mixture of oligomers bearing glycidyl ether groups.

The reaction with epoxide compounds may take place in some embodiments between 90 and 130°C, and in some other embodiments specifically between 100 to 110°C. The reaction may be continued until the reaction mixture has an acid number to DIN EN 3682 of less than 20, and preferably less than 5 mg KOH/g (excluding solvent).

Catalysts which can be used for the reaction with the epoxide compounds include, for example, quaternary ammonium compounds and phosphonium compounds, tertiary amines, phosphines such as triphenylphosphine, and Lewis bases.

Compositions in accordance with aspects of the disclosure may be comprised of one to four components. For ease of reference, four of these components will be referred to as Components A, B, C, and D. As will be discussed later in the disclosure, other embodiments may also comprise components E, F, G, and additional components.

Component (A) is at least one to four radiation-curable compounds having at least one free-radically polymerizable group, where component A can be reacted to form a polymeric PCM or functional polymeric PCM.

Component (B) is at least one to four and in some embodiments precisely one radiation-curable compound having precisely one free-radically polymerizable group.

Component (C) may be at least one to four, and in some embodiments precisely one radiation-curable compound having precisely two free-radically polymerizable groups.

Component (D) may be at least one to four, and in some embodiments precisely one radiation-curable compound having more than 2, in some embodiments 3-10, and in other embodiments specifically 3 free-radically polymerizable groups.

Examples of free-radically polymerizable groups are vinyl ether or $\alpha,\beta$-ethylenically unsaturated carboxylic acids, which in some embodiments are (meth)acrylate groups.

Monofunctional free-radically polymerizable compounds that make up Component (B) may be, for example, esters of $\alpha,\beta$-ethylenically unsaturated carboxylic acids, such as (meth)acrylic acid, with alcohols containing 1 to 40 carbon atoms, e.g., methyl (meth)acrylate, ethyl (meth)acrylate, n-butyl (meth)acrylate, 2-ethyl-hexyl (meth)acrylate, 2-hydroxyethyl (meth)acrylate, 2-hydroxypropyl (meth)acrylate or 4-hydroxybutyl (meth)acrylate.

Component (B), which comprises a monoethylenically unsaturated reactive species (B) may be sub-categorized into compound (B1), embodiments of which comprise least one cycloaliphatic group, or a compound (B2), embodiments of which comprise at least one heterocyclic group. Compounds (B1) are esters of (meth)acrylic acid with cycloalkanols or bicycloalkanols, the cycloalkanol or bicycloalkanol containing from 3 to 20 carbon atom and being optionally substituted by C1 to C4 alkyl.

Examples of cycloalkanol and bicycloalkanol are cyclopentanol, cyclohexanol, cyclooctanol, cyclododecanol, 4-methylcyclohexanol, 4-isopropylcyclohexanol, 4-tert-butylcyclohexanol (preferably cis-configured), dihydrodicyclopentadienyl alcohol, and norbornyl alcohol.

When a compound (B2) is utilized in embodiments of the disclosure, it is possible to use all monofunctional esters of α,β-ethylenically unsaturated carboxylic acids with a monofunctional alkanol that has as a structural element at least one saturated 5- or 6-membered heterocycle having one or two oxygen atoms in the ring. Component (B) may be derived from acrylic acid or methacrylic acid. Examples of suitable compounds (B2) of Component (B) comprise compounds of the general formula:

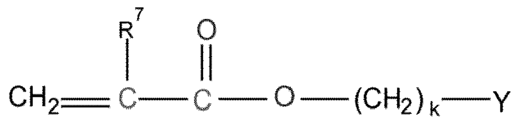

(III)

in which $R^7$ is selected from H and $CH_3$, k is a number from 0 to 4, and Y is a 5- or 6-membered, saturated heterocycle having one or two oxygen atoms, the heterocycle being optionally substituted by C1-C4 alkyl, e.g., methyl, ethyl, n-propyl, isopropyl, n-butyl, 2-butyl, isobutyl or tert-butyl.

The 5- or 6-membered saturated heterocycle derives preferably from tetrahydrofuran, tetrahydropyran, 1,3-dioxolane or 1,3- or 1,4-dioxane.

In many embodiments, compound (B2) is selected from trimethylolpropane monoformal acrylate, glycerol monoformal acrylate, 4-tetrahydropyranyl acrylate, 2-tetrahydropyranyl methylacrylate, tetrahydrofurfuryl acrylate, and mixtures of these.

Also conceivable for compound (B2) in certain embodiments are vinyl aromatic compounds, e.g., styrene, divinylbenzene, α,β-unsaturated nitriles, e.g., acrylonitrile, methacrylonitrile, α,β-unsaturated aldehydes, e.g., acrolein, methacrolein, vinyl esters, e.g., vinyl acetate, vinyl propionate, halogenated ethylenically unsaturated compounds, e.g., vinyl chloride, vinylidene chloride, conjugated unsaturated compounds, e.g., butadiene, isoprene, chloroprene, monounsaturated compounds, e.g., ethylene, propylene, 1-butene, 2-butene, isobutene, cyclic monounsaturated compounds, e.g., cyclopentene, cyclohexene, cyclododecene, N-vinylformamide, allylacetic acid, vinylacetic acid, monoethylenically unsaturated carboxylic acids having 3 to 8 carbon atoms and also their water-soluble alkali metal, alkaline earth metal or ammonium salts, such as, for example: acrylic acid, methacrylic acid, dimethylacrylic acid, ethylacrylic acid, maleic acid, citraconic acid, methylenemalonic acid, crotonic acid, fumaric acid, mesaconic acid, and itaconic acid, maleic acid, N-vinylpyrrolidone, N-vinyl lactams, such as N-vinylcaprolactom, N-vinyl-N-alkylcarboxamides or N-vinylcarboxamides, such as N-vinylacetamide, N-vinyl-N-methylformamide, and N-vinyl-N-methylacetamide, or vinyl ethers, examples being methyl vinyl ether, ethyl vinyl ether, n-propyl vinyl ether, isopropyl vinyl ether, n-butyl vinyl ether, sec-butyl vinyl ether, isobutyl vinyl ether, tert-butyl vinyl ether, 4-hydroxybutyl vinyl ether, and also mixtures of these.

Components (C) and (D) may be selected independently of one another. Component (C) may be sub-categorized into compounds (C1), (C2), (C3), (C4), (C5), (C6), and (C7). Component (D) may also be similarly sub-categorized into compounds (D1), (D2), (D3), (D4), (D5), (D6), and (D7). Embodiments of compounds (C1) or (D1) may comprise polyfunctional (meth)acrylic esters. Embodiments of compounds (C2) or (D2) may comprise polyester (meth)acrylates. Embodiments of compounds (C3) or (D3) may comprise polyether (meth)acrylates. Embodiments of compounds (C4) or (D4) may comprise urethane (meth)acrylate Embodiments of compounds (C5) or (D5) may comprise epoxy (meth)acrylates. Embodiments of compounds (C6) or (D6) may comprise (meth)acrylated polyacrylates. Embodiments of compounds (C7) or (D7) may comprise carbonate (meth)acrylates. In embodiments where both a Component (C) and a Component (D) are selected, they may comprise any combination of the compounds listed above. For example, a compound (C1), which comprises a polyfunctional (meth)acrylic ester, may be combined with a compound (D4), which comprises a urethane (meth)acrylate.

Any compounds comprising Components (C) and/or (D) may be, for example, esters of α,β-ethylenically unsaturated carboxylic acids, which in some embodiments may be of (meth)acrylic acid, and in other embodiments may be of acrylic acid with polyalcohols having a corresponding functionality of at least two. Suitable examples of polyalcohols of this kind are at least dihydric polyols, polyetherols or polyesterols or polyacrylatepolyols having an average OH functionality of at least 2, and in some embodiments specifically 3 to 10.

Examples of polyfunctional polymerizable compounds (C1) are ethylene glycol diacrylate, 1,2-propanediol diacrylate, 1,3-propanediol diacrylate, 1,4-butanediol diacrylate, 1,3-butanediol diacrylate, 1,5-pentanediol diacrylate, 1,6-hexanediol diacrylate, 1,8-octanediol diacrylate, neopentyl glycol diacrylate, 1,1-, 1,2-, 1,3-, and 1,4-cyclohexanedimethanol diacrylate, and 1,2-, 1,3- or 1,4-cyclohexandiol diacrylate.

Examples of polyfunctional polymerizable compounds (D1) are trimethylolpropane triacrylate, ditrimethylolpropane pentaacrylate or hexaacrylate, pentaerythritol triacrylate or tetraacrylate, glycerol diacrylate or triacrylate, and also diacrylates and polyacrylates of sugar alcohols, such as of sorbitol, mannitol, diglycerol, threitol, erythritol, adonitol (ribitol), arabitol (lyxitol), xylitol, dulcitol (galactitol), maltitol or isomalt, for example.

Further examples of (C1) and/or (D1) are (meth)acrylates of compounds of the formula shown below in (IIa) to (IId),

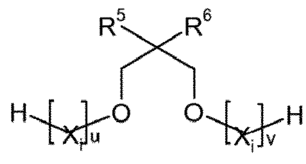
(IIa)

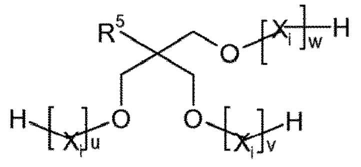
(IIb)

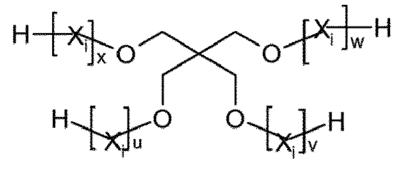
(IIc)

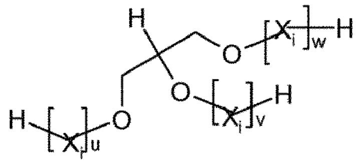
(IId)

In example (IIa) and (IIb) above, $R^5$ and $R^6$ independently of one another are hydrogen or are C1-C40 alkyl optionally substituted by aryl, alkyl, aryloxy, alkyloxy, heteroatoms and/or heterocycles. U, v, w, and x independently of one another are each an integer from 1 to 40, and each $X_i$, for i = 1 to u, 1 to v, 1 to w, and 1 to x, can be selected independently of the others from the group $-CH_2-CH_2-O-$, $-CH_2-CH(CH_3)-O-$, $-CH(CH_3)-CH_2-O-$, $-CH_2-C(CH_3)_2-O-$, $-C(CH_3)_2-CH_2-O-$, $-CH_2$-CHVin-O-, -CHVin-$CH_2$-O-, $-CH_2$-CHPh-O-, and -CHPh-$CH_2$-O-.

In these definitions, C1-C40 alkyl optionally substituted by aryl, alkyl, aryloxy, alkyloxy, heteroatoms and/or heterocycles may be, for example, methyl, ethyl, propyl, isopropyl, n-butyl, sec-butyl, tert- butyl, pentyl, hexyl, heptyl, octyl, 2-ethylhexyl, 2,4,4-trimethylpentyl, decyl, dodecyl, tetradecyl, hexadecyl, octadecyl, 1,1-dimethylpropyl, 1,1-dimethylbutyl, 1,1,3,3-tetramethylbutyl.

Polyester (meth)acrylates comprising compounds (C2) and/or (D2) are the corresponding esters of$\alpha$,$\beta$-ethylenically unsaturated carboxylic acids, preferably of (meth)acrylic acid with polyesterpolyols.

Polyesterpolyols are known in the art and disclosed in, for example, Ullmanns Encyklopadie der technischen Chemie, 4th Edition, Volume 19, pp. 62 to 65. In some embodiments of the disclosure, polyesterpolyols may be obtained by reacting dihydric alcohols with dibasic carboxylic acids. In place of the free polycarboxylic acids it is also possible to use the corresponding polycarboxylic anhydrides or corresponding polycarboxylic esters of lower alcohols, or mixtures thereof, to prepare the polyesterpolyols. The polycarboxylic acids may be aliphatic, cycloaliphatic, araliphatic, aromatic or heterocyclic and may, if desired, be substituted, by halogen atoms for example, and/or unsaturated. Examples thereof that may be mentioned include the following: oxalic acid, maleic acid, fumaric acid, succinic acid, glutaric acid, adipic acid, sebacic acid, dodecanedioic acid, o-phthalic acid, isophthalic acid, terephthalic acid, trimellitic acid, azelaic acid, 1,4-cyclohexanedicarboxylic acid or tetrahydrophthalic acid, suberic acid, azelaic acid, phthalic anhydride, tetrahydrophthalic anhydride, hexahydrophthalic anhydride, tetrachlorophthalic anhydride, endomethylenetetrahydrophthalic anhydride, glutaric anhydride, maleic anhydride, dimeric fatty acids, their isomers and hydrogenation products, and also esterifiable derivatives, such as anhydrides or dialkyl esters, such as C1-C4 alkyl esters, preferably methyl, ethyl or n-butyl esters, of the stated acids are employed. Some embodiments specifically include dicarboxylic acids of the general formula HOOC-$(CH_2)$y-COOH, y being a number from 1 to 40. Polyhydric alcohols contemplated for the preparation of the polyesterols include 1,2-propane-diol, ethylene glycol, 2,2-dimethyl-1,2-ethanediol, 1,3-propanediol, 1,2-butanediol, 1,3-butanediol, 1,4-butanediol, 3-methylpentane-1,5-diol, 2-ethylhexane-1,3-diol, 2,4-diethyloctane-1,3-diol, 1,6-hexanediol, polyTHF with a molar mass between 162 and 2000, poly-1,3-propanediol with a molar mass between 134 and 1178, poly-1,2-propanediol with a molar mass between 134 and 898, polyethylene glycol with a molar mass between 106 and 458, neopentyl glycol, neopentyl glycol hydroxypivalate, 2-ethyl-1,3-propanediol, 2-methyl-1,3-propanediol, 2,2-bis(4-hydroxycyclohexyl)propane, 1,1-, 1,2-, 1,3-, and 1,4-cyclohexanedimethanol, 1,2-, 1,3- or 1,4- cyclohexanediol, trimethylolbutane, trimethylolpropane, trimethylolethane, neopentyl glycol, pentaerythritol, glycerol, ditrimethylolpropane, dipentaerythritol, sorbitol, mannitol, diglycerol, threitol, erythritol, adonitol (ribitol), arabitol (lyxitol), xylitol, dulcitol (galactitol), maltitol or isomalt, which, if desired, may be alkoxylated as described above.

Certain embodiments utilize alcohols of the general formula HO-$(CH_2)$x-OH, x being a number from 1 to 40. Also contemplated, furthermore, are polycarbonatediols of the kind obtainable, for example, by reacting phosgene with an excess of the low molecular mass alcohols specified as synthesis components for the polyesterpolyols.

Also suitable are lactone-based polyesterdiols, which are homopolymers or copolymers of lactones, preferably hydroxyl-terminated adducts of lactones with suitable difunctional starter molecules. Lactones contemplated for some

embodiments include those deriving from the compounds of the general formula HO-$(CH_2)$z-COOH, z being a number from 1 to 40, and it also being possible for one H atom of a methylene unit to have been substituted by a C1 to C4 alkyl radical. Examples are ε-caprolactone, β-propiolactone, gamma-butyrolactone and/or methyl-ε-caprolactone, 4-hydroxybenzoic acid, 6-hydroxy-2-naphthoic acid or pivalolactone, and also their mixtures. Suitable starter components are, for example, the low molecular mass dihydric alcohols specified above as a synthesis component for the polyesterpolyols. The corresponding polymers of ε-caprolactone may be especially suitable for certain embodiments. Lower polyesterdiols or polyetherdiols can also be used as starters for preparing the lactone polymers. In place of the polymers of lactones it is also possible to use the corresponding, chemically equivalent polycondensates of the hydroxyl- carboxylic acids corresponding to the lactones.

Polyether (meth)acrylates comprising compounds (C3) and/or (D3) are the corresponding esters of α,β-ethylenically unsaturated carboxylic acids, in many embodiments (meth)acrylic acid, with polyetherols. The polyetherols may include polyethylene glycol with a molar mass between 106 and 20000, poly-1,2-propanediol with a molar mass between 134 and 12178, poly-1,3-propanediol with a molar mass between 134 and 12178, and polytetrahydrofurandiol having a number-average molecular weight Mn in the range from about 500 to 40000.

Urethane (meth)acrylates comprising compounds (C4) and/or (D4) are obtainable, for example, by reacting polyisocyanates with hydroxyalkyl (meth)acrylates or hydroxyalkyl vinyl ethers and, if desired, chain extenders such as diols, polyols, diamines, polyamines or dithiols or polythiols. Urethane (meth)acrylates dispersible in water without addition of emulsifiers additionally comprise ionic and/or nonionic hydrophilic groups, which are introduced into the urethane through synthesis components, for example, such as hydroxycarboxylic acids.

Such urethane (meth)acrylates substantially comprise as synthesis components: (a) at least one organic aliphatic, aromatic or cycloaliphatic di- or polyisocyanate, (b) at least one compound having at least one isocyanate-reactive group and at least one free-radically polymerizable unsaturated group, and (c) if desired, at least one compound having at least two isocyanate-reactive groups.

The urethane (meth)acrylates in certain embodiments have a number-average molar weight Mn of 500 to 20000 g/mol (as determined by gel permeation chromatography using tetrahydrofuran and polystyrene as standard). The urethane (meth)acrylates in certain embodiments have a (meth)acrylic group content of 1 to 5 per 1000 g of urethane (meth)acrylate.

Epoxide (meth)acrylates comprising compounds (C5) and/or (D5) are obtainable by reacting epoxides with (meth)acrylic acid. Examples of epoxides include epoxidized olefins, aromatic glycidyl ethers or aliphatic glycidyl ethers.

Epoxidized olefins may be, for example, ethylene oxide, propylene oxide, isobutylene oxide, 1 - butene oxide, 2-butene oxide, vinyloxirane, styrene oxide or epichlorohydrin. Aromatic glycidyl ethers are, for example, bisphenol A diglycidyl ether, bisphenol F diglycidyl ether, bisphenol B diglycidyl ether, bisphenol S diglycidyl ether, hydroquinone diglycidyl ether, alkylation products of phenol/dicyclopentadiene, e.g., 2,5-bis[(2,3-epoxypropoxy)phenyl]octa-hydro-4,7-methano-5H-indene) (CAS No. [13446-85-0]), tris[4-(2,3-epoxypropoxy)phenyl]- methane isomers) (CAS No. [66072-39-7]), phenol-based epoxy novolacs, (CAS No. [9003-35-4]), and cresol-based epoxy novolacs (CAS No. [37382-79-9]).

Aliphatic glycidyl ethers are, for example, 1,4-butanediol diglycidyl ether, 1,6-hexanediol diglycidyl ether, trimethylolpropane triglycidyl ether, pentaerythritol tetraglycidyl ether, 1,1,2,2-tetra-kis[4-(2,3-epoxypropoxy)phenyl]ethane (CAS No. [27043-37-4]), diglycidyl ethers of polypropylene glycol (a,ω-bis(2,3-epoxypropoxy)poly(oxypropylene) (CAS No. [16096-30-3]) and of hydrogenated bisphenol A (2,2-bis[4-(2,3-epoxypropoxy)cyclohexyl]propane, CAS No. [13410-58-7]).

The epoxide (meth)acrylates and epoxide vinyl ethers in some embodiments have a number-average molar weight Mn of 200 to 20000. The (meth)acrylic group or vinyl ether group content may be between 1 to 5 per 1000 g of epoxide (meth)acrylate or vinyl ether epoxide (as determined by gel permeation chromatography using polystyrene as standard and tetrahydrofuran as eluent).

(Meth)acrylated polyacrylates comprising compounds (C6) and/or (D6) are the corresponding esters ofα,β-ethylenically unsaturated carboxylic acids with polyacrylate polyols. Polyacrylate polyols of this kind in some embodiments have molecular weight Mn of at least 1000-80000. Some embodiments contain OH numbers of polyacrylate polyols, measured in accordance with DIN 53240-2, between 15-250 mg KOH/g.

Additionally, the polyacrylate polyols may have an acid number in accordance with DIN EN ISO 3682 of up to 200 mg KOH/g. The polyacrylate polyols are copolymers of at least one (meth)acrylic ester with at least one compound having at least one, and in some embodiments precisely one hydroxyl group, and at least one, in some embodiments precisely one (meth)acrylate group. The latter may be, for example, monoesters of α,β-unsaturated carboxylic acids, such as acrylic acid, methacrylic acid (referred to in this disclosure for ease of reference as "(meth)acrylic acid"), with diols or polyols, which in some embodiments have 2 to 40 carbon atoms and at least two hydroxyl groups, such as ethylene glycol, diethylene glycol, triethylene glycol, 1,2-propylene glycol, 1,3-propylene glycol, 1,1-dimethyl-1,2-ethanediol, dipropylene glycol, triethylene glycol, tetraethylene glycol, pentaethylene glycol, tripropylene glycol, 1,4-butanediol, 1,5-pentanediol, neopentyl glycol, neopentyl glycol hydroxypivalate, 2-ethyl-1,3-propanediol, 2-methyl-1,3-propanediol,

2-butyl-2-ethyl-1,3- propanediol, 1,6-hexanediol, 2-methyl-1,5-pentanediol, 2-ethyl-1,4-butanediol, 2-ethyl-1,3-hexanediol, 2,4-diethyloctane-1,3-diol, 2,2-bis(4-hydroxycyclohexyl)propane, 1,1-, 1,2-, 1,3-, and 1,4-bis(hydroxymethyl)cyclohexane, 1,2-, 1,3- or 1,4-cyclohexanediol, glycerol, trimethylolethane, trimethylolpropane, trimethylolbutane, pentaerythritol, ditrimethylolpropane, dipentarythritol, sorbitol, mannitol, diglycerol, threitol, erythritol, adonitol (ribitol), arabitol (lyxitol), xylitol, dulcitol (galactitol), maltitol, isomalt, polyTHF with a molar weight between 162 and 4500, poly-1,3-propanediol or polypropylene glycol with a molar weight between 134 and 2000, or polyethylene glycol with a molar weight between 238 and 2000.

In many embodiments, 2-hydroxyethyl acrylate, 2-hydroxyethyl methacrylate, 2- or 3-hydroxypropylacrylate, 1,4-butanediol monoacrylate or 3-(acryloyloxy)-2-hydroxypropyl acrylate are used. The monomers which carry hydroxyl groups are used in the copolymerization in a mixture with other polymerizable monomers, which may be free-radically polymerizable monomers, some of which may be those composed to an extent of more than 50% by weight of C1-C40 alkyl (meth)acrylate, (meth)acrylic acid, vinylaromatics having up to 40 carbon atoms, vinyl esters of carboxylic acids comprising up to 40 carbon atoms, vinyl halides, nonaromatic hydrocarbons having 4 to 8 carbon atoms and 1 or 2 double bonds, unsaturated nitriles, and mixtures of these. Many embodiments specifically include polymers composed to an extent of more than 60% by weight of C1-C40 alkyl (meth)acrylates, styrene, vinylimidazole or mixtures of these.

The polymers may further comprise hydroxyl-functional monomers corresponding to the above hydroxyl group content, and, if desired, further monomers, examples being glycidyl epoxy esters of (meth)acrylic acid, or ethylenically unsaturated acids, more particularly carboxylic acids, acid anhydrides or acid amides.

Carbonate (meth)acrylates comprising compounds (C7) and/or (D7) are obtainable with different functionalities, in a similar manner to how methacrylated epoxides such as (C5)/(D5) and methacrylated urethanes such as (C4)/(D4) are obtainable with different functionalities. The number-average molecular weight Mn of the carbonate (meth)acrylates in many embodiments is less than 3000 g/mol (as determined by gel permeation chromatography using polystyrene as standard; solvent: tetrahydrofuran).

The carbonate (meth)acrylates are obtainable in a simple way by transesterification of carbonic esters with polyhydric, preferably dihydric alcohols (diols, e.g., hexanediol) and subsequent esterification of the free OH groups with (meth)acrylic acid, or else transesterification with (meth)acrylic esters, as described in EP-A 92 269, for example. They are also obtainable by reaction of phosgene, urea derivatives with polyhydric alcohols, dihydric alcohols for example.

Obtainable in a similar way are vinyl ether carbonates, by reaction of a hydroxyalkyl vinyl ether with carbonic esters and also, if desired, dihydric alcohols. Also conceivable are (meth)acrylates or vinyl ethers of polycarbonate polyols, such as the reaction product of one of the stated diols or polyols and a carbonic ester and also a hydroxyl-containing (meth)acrylate or vinyl ether. Examples of suitable carbonic esters are ethylene carbonate, 1,2- or 1,3-propylene carbonate, dimethyl carbonate, diethyl carbonate or dibutyl carbonate.

Examples of suitable hydroxyl-containing (meth)acrylates are 2-hydroxyethyl (meth)acrylate, 2- or 3-hydroxypropyl (meth)acrylate, 1,4-butanediol mono(meth)acrylate, neopentyl glycol mono(meth)acrylate, glycerol mono(meth)acrylate and di(meth)acrylate, trimethylolpropane mono(meth)acrylate and di(meth)acrylate, and also pentaerythritol mono(meth)acrylate, di(meth)acrylate, and tri(meth)acrylate. Examples of suitable hydroxyl-containing vinyl ethers are 2-hydroxyethyl vinyl ether and 4-hydroxybutyl vinyl ether.

Many embodiments may contain carbonate (meth)acrylates of the formula:

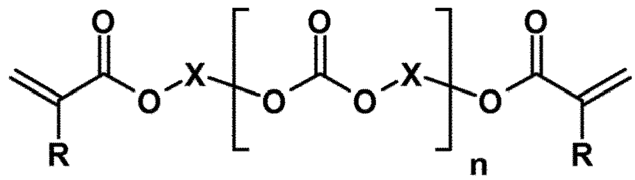

in which R is H or $CH_3$, X is a $C_2$-$C_{40}$ alkylene group, and n is an integer from 1 to 5. R is preferably H, and X is preferably $C_2$ to $C_{40}$ alkylene, exemplified by 1,2-ethylene, 1,2-propylene, 1,3-propylene, 1,4-butylene or 1,6-hexylene.

The carbonate (meth)acrylates in many embodiments may be aliphatic carbonate (meth)acrylates. Among the polyfunctional polymerizable compounds, many embodiments will specifically include urethane (meth)acrylates (C4) and/or (D4).

In certain embodiments of the present disclosure, at least one of the components, (C) or (D), has a diol as a synthesis component, selected from the group consisting of: polytetrahydrofurandiol ($H-[-O-CH_2-CH_2-CH_2-CH_2-]_k-OH$), polyethylene glycol ($H-[-O-CH_2-CH_2-]k-OH$), polypropylene glycol, $H-[-O-CH_2-CH(CH_3)-]_k-OH$ polycaprolactonediol ($-[-O-CH_2-CH_2-CH_2-CH_2-CH_2-(CO)-]_k-R^8-OH$), and polyesterdiol ($HO-[R^8-O-(CO)-R^9-(CO)-O-R^8-]_k$ -OH).

In the above formulas, $R^8$ and $R^9$ independently of one another are a divalent aliphatic or cycloaliphatic radical having at least one carbon atom, and k is a positive integer with a value sufficient to obtain the molar mass in question.

In certain embodiments, radicals $R^8$ and $R^9$ are, independently of one another, methylene, 1,2-ethylene, 1,2-propylene, 1,3-propylene, 1,2-, 1,3- or 1,4-butylene, 1,1-dimethyl-1,2-ethylene or 1,2-dimethyl-1,2-ethylene, 1,5-pentylene, 1,6-hexylene, 1,8-octylene, 1,10-decylene or 1,12-dodecylene.

In the present disclosure, components can also contain, cure or crosslink by the thiol-ene reaction as shown below. The thiol-ene reaction is an addition reaction between a thiol and alkene to form a thioether. Any of the above compounds can be polyfunctional and polymerizable. Thiols can also react with vinyl ethers, propenyl ethers, allyl ethers, (meth)acrylates, vinyls, and or conjugated dienes.

In addition to components (A), (B), (C), and (D), various embodiments may also further comprise a component (E), which may be a photoinitiator. Based on the sum of the components (A), (B), (C), and (D), the compositions of the disclosure may comprise 0% to 10% by weight of at least one photoinitiator (E). Photoinitiators (E) may be, for example, photoinitiators known to the skilled worker, examples being those specified in "Advances in Polymer Science", Volume 14, Springer Berlin 1974 or in K. K. Dietliker, Chemistry and Technology of UV- and EB-Formulation for Coatings, Inks and Paints, Volume 3; Photoinitiators for Free Radical and Cationic Polymerization, P. K. T. Oldring (ed.), SUA Technology Ltd, London.

Suitable photoinitiators are those of the kind described in WO 2006/005491 A1 , page 21 , line 18 to page 22, line 2 (corresponding to US 2006/0009589 A1 , paragraph [0150]), which is hereby incorporated by reference as part of the present disclosure.

Also suitable are non-yellowing or low-yellowing photoinitiators of the phenylglyoxalic ester type, as described in DE-A 198 26 712, DE-A 199 13 353 or WO 98/33761. Particular photoinitiators selected for embodiments of the present disclosure include 2,4,6-trimethylbenzoyldiphenylphosphine oxide, ethyl 2,4,6-trimethylbenzoylphenylphosphinate, bis(2,4,6-trimethylbenzoyl)phenylphosphine oxide, benzophenone, 1-benzoylcyclohexan-1-ol, 2-hydroxy-2,2-dimethylacetophenone, and 2,2-dimethoxy-2-phenylacetophenone.

In addition to components (A), (B), (C), and (D), various embodiments may also further comprise a component (F), which may be an additive. It is contemplated that embodiments of the disclosure may include components (A), (B), (C), (D), (E), and one or more components (F). Based on the sum of the components (A), (B), (C), (D), and sometimes (E), the compositions of the disclosure may further comprise 0% to 10% by weight of typical additives (F).

Examples of further typical additives (F) that can be used include antioxidants, stabilizers, activators (accelerants), fillers, pigments, dyes, antistats, flame retardants, thickeners, thixotropic agents, surface-active agents, viscosity modifiers, plasticizers or chelating agents.

It is further possible to add one or more thermally activable initiators, examples being potassium peroxodisulfate, dibenzoyl peroxide, cyclohexanone peroxide, di-tert-butyl peroxide, azobisisobutyronitrile, cyclohexylsulfonyl acetyl peroxide, diisopropyl percarbonate, tert-butyl peroctoate or benzpinacol, and also, for example, those thermally activable initiators which have a half-life at 80°C of more than 100 hours, such as di-tert-butyl peroxide, cumene hydroperoxide, dicumyl peroxide, tert-butyl perbenzoate, silylated pinacols, which are available commercially under the trade name ADDID 600 from Wacker, for example, or hydroxyl-containing amine N-oxides, such as 2,2,6,6-tetramethylpiperidine-N-oxyl, 4-hydroxy-2,2,6,6-tetramethylpiperidine-N-oxyl, etc. Further examples of suitable initiators are described in "Polymer Handbook", 2nd Ed., Wiley & Sons, New York.

Thickeners contemplated include not only free-radically (co)polymerized (co)polymers but also typical organic and inorganic thickeners such as hydroxymethylcellulose or bentonite.

Examples of chelating agents which can be used include ethylenediamineacetic acid and the salts thereof, and also β-diketones. Suitable fillers comprise silicates, examples being silicates obtainable by silicon tetrachloride hydrolysis, such as Aerosil® from Degussa, silicious earth, talc, aluminum silicates, magnesium silicates, and calcium carbonates, etc. Suitable stabilizers comprise typical UV absorbers such as oxanilides, triazines, and benzotria-zole (the latter available as Tinuvin® products), and benzophenones. They can be used alone or together with suitable free-radical scavengers, examples being sterically hindered amines such as 2,2,6,6-tetramethylpiperidine, 2,6-di-tert-butylpiperidine or derivatives thereof, such as bis(2,2,6,6-tetramethyl-4-piperidyl) sebacate, for example. Stabilizers are used typically in amounts from 0.1 % to 5.0% by weight, based on the solid components comprised in the preparation.

The compositions may further comprise a solvent, examples being water, butyl acetate, ethyl acetate, methoxypropyl acetate, toluene, xylene, fluorinated aromatics, and aliphatic and aromatic hydrocarbon mixtures.

Methods of Manufacture of the PCM-TMM Film for Temperature Management and Heat Dissipation

The TMM composition that is prepared for application onto a substrate, including liquid, powder, particulate, or solid compositions, can be prepared by any convenient means such as mixing all components together at higher temperatures. In many embodiments, this temperature is higher than the phase change temperature of the PCM/pPCM/fp-PCM. The TMM can be manufactured by addition of ingredients in a certain order to facilitate specific reactions or interactions such as the pretreatment of the fillers and particles with coupling agents or silanes. The TMM can be mixed at low, room temperature or higher temperatures. The TMM can be prepared in mixing vessels, reactors, extruders or the like.

Methods of Casting, Coating, and Other Application Forms

The PCM-TMM can be implemented as a tape, film casing, coating, laminate, infusion, treatment, gums, gels, film, sheets, greases, waxes or ingredient in a coating, laminate, infusion, treatment, caulks, gums, gels, film, sheets, tapes, greases, waxes that is formed adjacent to, on or within objects requiring thermal management using any suitable coating, laminating, layering, infusion, etc. technique. Application techniques and forms can include manually or mechanically wrapping a film or tape one or more times, spray coating, air atomized spraying, airless atomized spraying, electrostatic spraying, slot die coating, contact slot coating, curtain coating, knife coating, roller coating, kiss coating, transfer coating, foam coating, brushing, screenprinting, padding, dipping or immersion, saturating, printing, pressure or gravity feed nozzles/guns, hot melt applicators, pump guns, manually operated guns, syringes, needle guns, various shape and size nozzles, molding, overmolding, injection molding, RIM, prepeg, Resin infusion process such as resin transfer molding (RTM), vacuum infusion process (VIP) and vacuum assisted RTM (VARTM), pultrusion, extrusion, plasma, etc. The TMM may be manufactured as rolls or individually cut sheets for shipment to and subsequent transfer by an end user. Such sheets of tape or film may be manufactured in standard sizes that anticipate the number of times the sheet may be wrapped around a particular object, and the standard sizes and shapes can vary depending on anticipated applications.

The compositions of the present disclosure are suitable as molding compounds, as for example in films, tapes, or sheets which may optionally be reinforced with fibers or for coating substrates such as wood, paper, textile, leather, nonwoven, plastics surfaces, glass, ceramic, mineral building materials, such as molded cement bricks and fiber cement slabs, or coated or uncoated metals. In many embodiments substrates may be plastics or metals, which themselves may exists in the form of films or foils. Many embodiments may utilize metal substrates.

The materials may be applied more than one time by any of a very wide variety of spraying methods, such as compressed-air, airless or electrostatic spraying methods, using one- or two-component spraying units, or else by injecting, troweling, knifecoating, brushing, rolling, roller coating, pouring, laminating, injection backmolding or coextruding multiple layers.

The application process of the liquid composition can be run cold, warm, hot or at room temperature, i.e. above or below room temperature, i.e. -100°C to 400°C, depending on the temperature conditions required for optimal application of the particular composition.

Compositions of the present disclosure can be applied to a substrate. Treatment of substrates with the compositions of the disclosure takes place in accordance with typical processes known to those skilled in the art. A composition can be applied in the desired thickness to the target substrate and optionally dried. This operation may be repeated one or more times. Application to the substrate may take place in a known way, similar to methods previously described for application in general. For example, application to a substrate may occur by spraying, troweling, knife- coating, brushing, rolling, roller coating, pouring, laminating, injection backmolding or coextruding. The material may also be applied electrostatically in the form of powder (powder coating materials). In many embodiments, the compositions can be up to 200 μm in thickness. However, it is contemplated that other embodiments may be of less or greater thickness. The film thickness in many embodiments may be in a range from about 3 to 1000 g/m2. In other embodiments it may be more specifically between 10 to 200 g/$m^2$.

Methods of Curing

Once liquid or powder compositions of the present disclosure are coated or cast, they may be cured in a number of different ways. In some embodiments of the method, liquid compositions may be simply dried to cure, and in other embodiments they may be dried first and then cured by a separate curing process.

One way that liquid or powder compositions may be curable is by actinic radiation. Actinic radiation having a wavelength range from 200 nm to 700 nm is useful for example. Actinic radiation having an energy in the range from 30 mJ/cm2 to 2000 mJ/cm2 is useful for example. Actinic radiation may advantageously be applied continuously, or alternatively, in the form of flashes.

Another method of curing the compositions of this disclosure is electron radiation though suitable electron flash devices. This electron radiation can occur, in some embodiments, with energy in the range from 50 to 300 keV, and in other embodiments, specifically between 90 to 200 keV.

When curing is effected by means of electron radiation, the mixture of the present disclosure may not comprise any photoinitiator (E). This has the advantage that no migratable photoinitiator constituents remain in the coating which has been formed by irradiation. This is particularly of advantage when the coatings are intended for food contact, because of concerns of food contamination related to photoinitators.

In certain embodiments of the curing method of this disclosure, the distance of the electron flash devices to the printing surface may be between 1 and 100 cm, and more specifically, between 2 to 50 cm.

Radiation curing may take place with high-energy light, UV light for example, or electron beams. Radiation

curing may take place at relatively high temperatures. When a radiation-curable binder is used, the radiation curing may take place at a temperature above the Tg of the radiation-curable binder.

Besides radiation curing, there may also be further curing mechanisms involved. Some examples include thermal curing (e.g. polyaddition/condensation reactions such as isocyanate-alcohol or humidity, melamine-alcohol, epoxy-epoxy, epoxy-acid, epoxy-amine, cationic cure, etc.), moisture curing (e.g. siloxane condensation, isocyanate, etc.), chemical curing (free radical curing such as with peroxides, etc.) and/or oxidative curing. In some embodiments, a combination of both thermal and radiation curing may be used, and in other embodiments, curing may be accomplished by radiation curing alone.

Drying and curing of the coatings take place generally under standard temperature conditions, i.e., without the coating being heated. Alternatively the mixtures of the invention can be used to produce coatings which, following application, are dried and cured at an elevated temperature, e.g., at 40-250°C. This is limited by the thermal stability of the substrate.

In embodiments where the composition is admixed with thermally curable formulations (which may also be known as resins), the composition may be applied to the substrate, dried, and then cured with electron beams or UV exposure under an oxygen-containing atmosphere or, preferably, under inert gas, optionally at temperatures up to the level of the drying temperature.

If a plurality of layers of the coating material are applied one on top of another, drying and/or radiation curing may optionally take place after each coating operation.

Examples of suitable radiation sources for the radiation cure are low-pressure, medium- pressure, and high-pressure mercury lamps, and also fluorescent tubes, pulsed lamps, metal halide lamps, electronic flash devices, which allow radiation curing without a photoinitiator, or excimer lamps. The radiation cure is accomplished by exposure to high-energy radiation, i.e., UV radiation or daylight, preferably light emitted in the wavelength range of $\lambda$=200 to 700 nm, or by irradiation with high-energy electrons (electron radiation; 150 to 300 keV). Examples of radiation sources used include high-pressure mercury vapor lamps, lasers, pulsed lamps (flash light), halogen lamps or excimer lamps. The radiation dose typically sufficient for crosslinking in the case of UV curing is situated in the range from 80 to 3000 mJ/cm2.

It is contemplated that two or more radiation sources for curing may be used in certain embodiments of the method. For example, two to four radiation sources may be used. These sources may also each emit in different wavelength ranges.

Drying and/or thermal treatment may also take place, in addition to or instead of the thermal treatment, by means of NIR radiation, which here refers to electromagnetic radiation in the wavelength range from 760 nm to 2.5 $\mu$m.

Irradiation can optionally also be carried out in the absence of oxygen, such as under an inert gas atmosphere. Suitable inert gases include nitrogen, noble gases, carbon dioxide, or combustion gases. In some embodiments irradiation can be carried out in the manner described in DE-A1 199 57 900.

Like the application process, the curing process can be run cold, warm, hot or at room temperature. Ranges may include, for example, -100°C to 400°C. Curing, crosslinking, drying or further reactions of the TMM or various layers to either cause bonding between or within the layers, or to facilitate the application of additional layers. These methods can be accomplished by any energy source. Examples are thermal, heat, light, UV, IR, radiation, solar, induction, pressure, sonic or sound waves, x-ray, radio waves, gamma waves, microwave, laser, e-beam, plasma, etc.

In some embodiments, multiple layers of PCM-TMMs with different compositions are utilized. It is contemplated that any PCM-TMM article, such as a tube, sleeve, or wrapped tape, can include as few or as many layers as required for various implementations.

FIG. 31 is a flowchart showing a method 3100 which may be traversed to form a PCM-TMM film according to embodiments of the present disclosure. First, at 3101, the method may comprise mixing at least three compounds, wherein a first compound comprises a polymeric phase change material, a second compound comprises an additive, and a third compound comprises a molecule that, in its liquid form, acts as a solvent of the first and second compounds, and which is curable into a solid form as a polymer. Next, at 3102, the method may comprise applying a mixture of the first, second, and third compounds in a liquid form to a substrate. Then, at 3103, the method may comprise curing the mixture into a solid state.

It should be clearly understood that by providing examples of specific compositions, applicant does not intend to limit the scope of the claims to any of these specific composition. To the contrary, it is anticipated that any combination of the functional groups, polymeric phase change materials, and articles described herein may be utilized to achieve the novel aspects of the present disclosure. The claims are not intended to be limited to any of the specific compounds described in this disclosure, any disclosure incorporated herein or the example given above.

While the present disclosure has been described with reference to the specific embodiments thereof, it should be understood by those skilled in the art that various changes may be made and equivalents may be substituted without departing from the scope of the disclosure as defined by the appended claims. In addition, many modifications may be made to adapt a particular situation, material, composition of matter, method, process step or steps, to the objective, spirit and scope of the present disclosure. All such modifications are intended to be within the scope of the claims

appended hereto. In particular, while the methods disclosed herein have been described with reference to particular steps performed in a particular order, it will be understood that these steps may be combined, sub-divided, or re-ordered to form an equivalent method without departing from the teachings of the present disclosure. Accordingly, unless specifically indicated herein, the order and grouping of the steps is not a limitation of the present disclosure.

In the following paragraphs [00379] to [00394] further embodiments of the invention are described:

**Embodiment 1:** A method of manufacturing a polymeric film with thermal management capabilities, the method comprising the steps of: mixing at least three compounds, wherein: a first compound comprises a polymeric phase change material, a second compound comprises an additive, a third compound comprises a molecule that, in its liquid form, acts as a solvent of the first and second compounds, and which is curable into a solid form as a polymer, applying a mixture of the first, second, and third compounds in a liquid form to a substrate, and curing the mixture into a solid state.

**Embodiment 2:** The method of embodiment 1, wherein the molecule is a monomer.

**Embodiment 3:** The method of embodiment 1, wherein the polymer is a polymeric phase change material, and especially wherein the polymeric phase change material is a functional polymeric phase change material.

**Embodiment 4:** The method of embodiment 1, wherein the third compound comprises between one and four radiation-curable compounds, and especially wherein each of the radiation-curable compounds comprises at least one free-radically polymerizable group, and wherein optionally at least one of the radiation-curable compounds can be reacted to form a polymeric phase change material and/or a functional polymeric phase change material.

**Embodiment 5:** The method of embodiment 1, wherein the curing comprises UV curing.

**Embodiment 6:** The method of embodiment 1, wherein the substrate is adhesive and/or wherein the substrate comprises metal.

**Embodiment 7:** The method of embodiment 1, wherein the applying is performed by one of casting or coating.

**Embodiment 8:** A film with thermal management capabilities, comprising: at least one polymeric phase change material, at least one additive, and a solid polymeric matrix formed from a cured liquid monomeric solvent.

**Embodiment 9:** The film of embodiment 8, further comprising a substrate backing, especially wherein the substrate backing is adhesive and/or wherein the substrate backing comprises metal.

**Embodiment 10:** The film of embodiment 8, wherein the solid polymeric matrix comprises at least one other polymeric phase change material.

**Embodiment 11:** The film of embodiment 8, wherein the at least one additive is one of a fire retardant additive, a waterproofing additive, a corrosion-resistant additive, an energy conductive additive, and an impact resistant additive, and/or wherein the film is configured to be shrink-wrapped through the application of heat.

**Embodiment 12:** The film of embodiment 8, further comprising a transdermal medication.

**Embodiment 13:** A system for thermal management comprising: an object that emits heat, and a film with thermal management capabilities wrapped around the object that emits heat, the film comprising; at least one polymeric phase change material, at least one additive, a solid polymeric matrix formed from a cured liquid monomeric solvent, wherein, the phase change material has a transition temperature and a latent heat storage range that dissipates heat from the object that emits heat.

**Embodiment 14:** The system of embodiment 13, further comprising; a material for protecting the object from ambient cold temperatures, especially wherein the material comprises a second phase change material with a transition temperature and latent heat storage range that releases heat onto the object and/or wherein the material comprises a reflective insulating layer.

**Embodiment 15:** The system of embodiment 13, wherein the film is wrapped completely around the object or wherein the film is wrapped more than once around the object.

**Embodiment 16:** The system of embodiment 13, wherein the object is a pipe, an electrochemical cell, a tank for storing liquid or gaseous materials, a component of a hydraulic system, of a vehicle, or of a computer system or wherein the object is an electrical wire.

## Claims

**1.** A temperature management material (TMM) for thermal management comprising:

a mixture of:

phase change material (PCM) particles;
a compound having a free-radically polymerizable group that, when in a liquid form, is a solvent for the PCM particles and, when cured, forms a polymeric phase change material (pPCM) having a latent heat of

fusion greater than 5 Joules per gram and a transition temperature between 5°C and 120°C; and optionally, a photoinitiator, a thermally activatable initiator, or a combination thereof;

wherein, after exposure to a means for curing, the compound having a free-radically polymerizable group is crosslinked and the TMM is a gelled composition having the PCM particles dispersed therein;
wherein the TMM has a first transition temperature for absorption or release of latent heat associated with the PCM particles and a second transition temperature for absorption or release of latent heat associated with the pPCM, and an average of 0.05-1.0 crosslinks per polymeric chain.

**2.** The temperature management material of claim 1, wherein the PCM particles are selected from the group consisting of microencapsulated PCMs, cured polymeric PCMs, cured functional polymeric PCMs, raw PCMs, and combinations thereof.

**3.** The temperature management material of claim 1, wherein the PCM particles comprise over 80% by weight of the gelled composition.

**4.** The temperature management material of clam 1, wherein the compound having a free-radically polymerizable group comprises a polyolefin random or block copolymer, a polyester, a silicon rubber, or a polyurethane elastomer.

**5.** The temperature management material of claim 1, wherein the mixture further comprises an additive.

**6.** The temperature management material of claim 5, wherein the additive is selected from the group consisting of antioxidants, stabilizers, fillers, pigments, dyes, antistatic agents, flame retardants, thickeners, thixotropic agents, surface-active agents, viscosity modifiers, plasticizers, chelating agents, energy conductive additives, waterproofing additives, corrosion-resistant additive, impact resistant additives, and combinations thereof.

**7.** The temperature management material of claim 1, wherein the compound having a free-radically polymerizable group is a liquid prior to exposure to the means for curing.

**8.** The temperature management material of claim 1, wherein the pPCM is a functional pPCM.

**9.** The temperature management material of claim 1, wherein the compound having a free-radically polymerizable group is a reaction product of an acrylic acid and a polyol or a polyesterol in a molar ration of hydroxy groups to acrylic acid in an esterification of 1: 0.75-2.5.

**10.** The temperature management material of claim 9, wherein the polyol is an alkoxylated polyol.

**11.** The temperature management material of claim 9, wherein the polyesterol has a molecular mass in a range of 1000 to 4000 g/mol.

**12.** The temperature management material of claim 1, wherein the mixture further comprises a radiation-curable compound having one free-radically polymerizable group, two-radically polymerizable groups, or 3-10 free radically polymerizable groups, the free-radically polymerizable groups being selected from vinyl ether and/or $\alpha,\beta$-ethylenically unsaturated carboxylic acids.

**13.** The temperature management material of claim 12, wherein the radiation-curable compound is an ester of $\alpha,\beta$-ethylenically unsaturated carboxylic acids with alcohols containing 1 to 40 carbon atoms.

**14.** The temperature management material of claim 12, wherein the radiation-curable compound is selected from the group consisting of styrene, divinylbenzene, acrylonitrile, methacrylonitrile, acrolein, methacrolein, vinyl acetate, vinyl propionate, vinyl chloride, vinylidene chloride, butadiene, isoprene, chloroprene, ethylene, propylene, 1-butene, 2-butene, isobutene, cyclopentene, cyclohexene, cyclododecene, N-vinylformamide, allylacetic acid, vinylacetic acid, acrylic acid, methacrylic acid, dimethacrylic acid. ethylacrylic acid, maleic acid, citraconic acid, methylenemalonic acid, crotonic acid, fumaric acid, mesaconic acid, itaconic acid, N-vinylpyrrolidone, N-vinylcaprolactom, N-vinyl-N-alkylcarboxamides, N-vinylacetamide, N-methylformamide, N-vinyl-N-methylacetamide, methyl vinyl ether, ethyl vinyl ether, n-propyl vinyl ether, isopropyl vinyl ether, n-butyl vinyl ether, sec-butyl vinyl ether, isobutyl vinyl ether, tertbutyl vinyl ether, 4-hydroxybutyl vinyl ether, and mixtures thereof.

**15.** The temperature management material of claim 1, wherein the mixture comprises a thermally-curable compound and a thermal initiator.

**16.** The temperature management material of claim 1, wherein the gelled composition has elastomeric properties and a flex modulus of greater than 15 pounds per square inch and a break elongation of greater than 20%.

**17.** The temperature management material of claim 1, wherein the compound having a free-radically polymerizable group has a degree of unsaturation in a range of 0-99 mole %, more preferably 0-15 mole %.

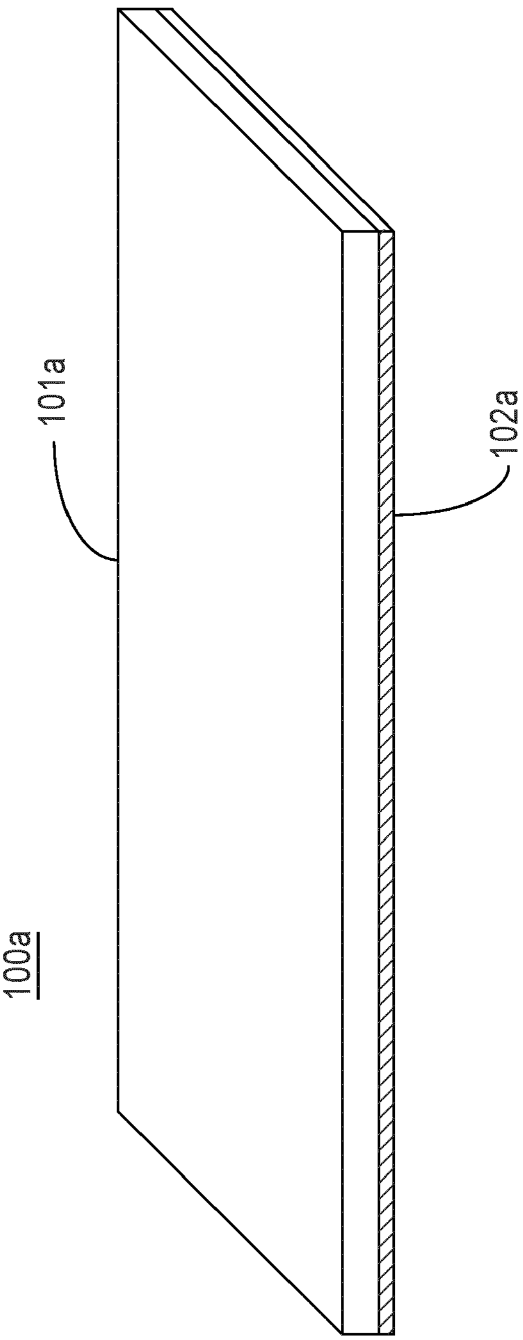


FIG.1A

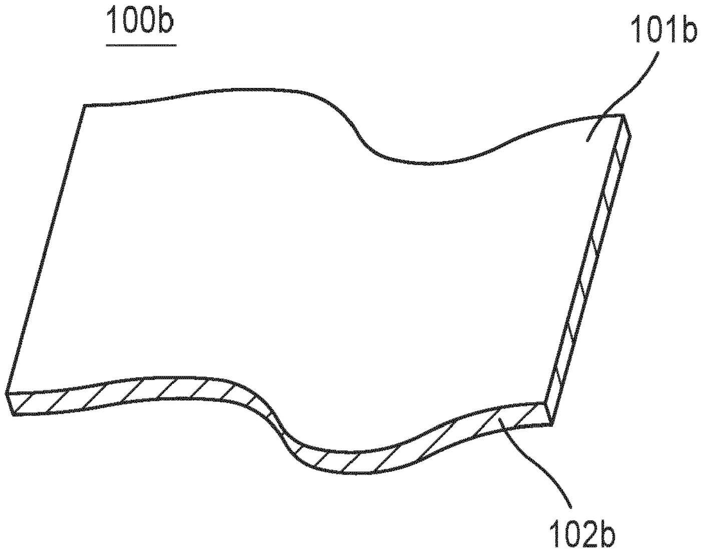


FIG.1B

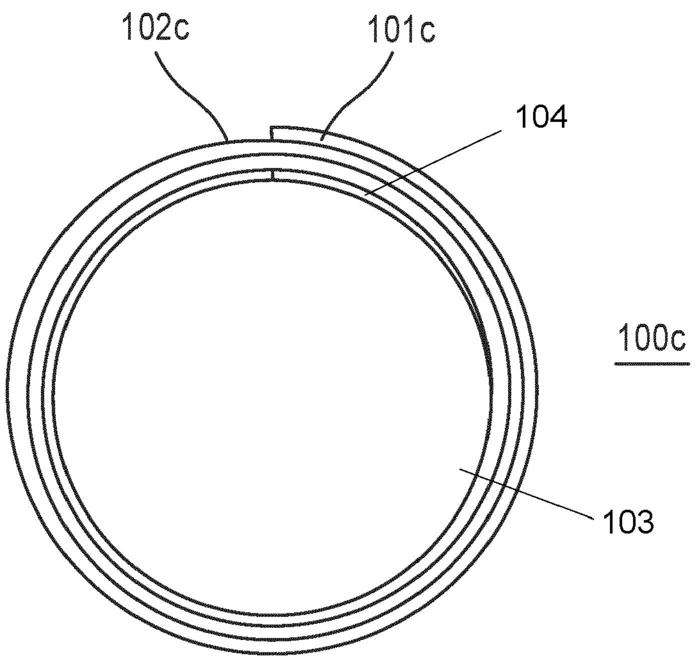


FIG.1C

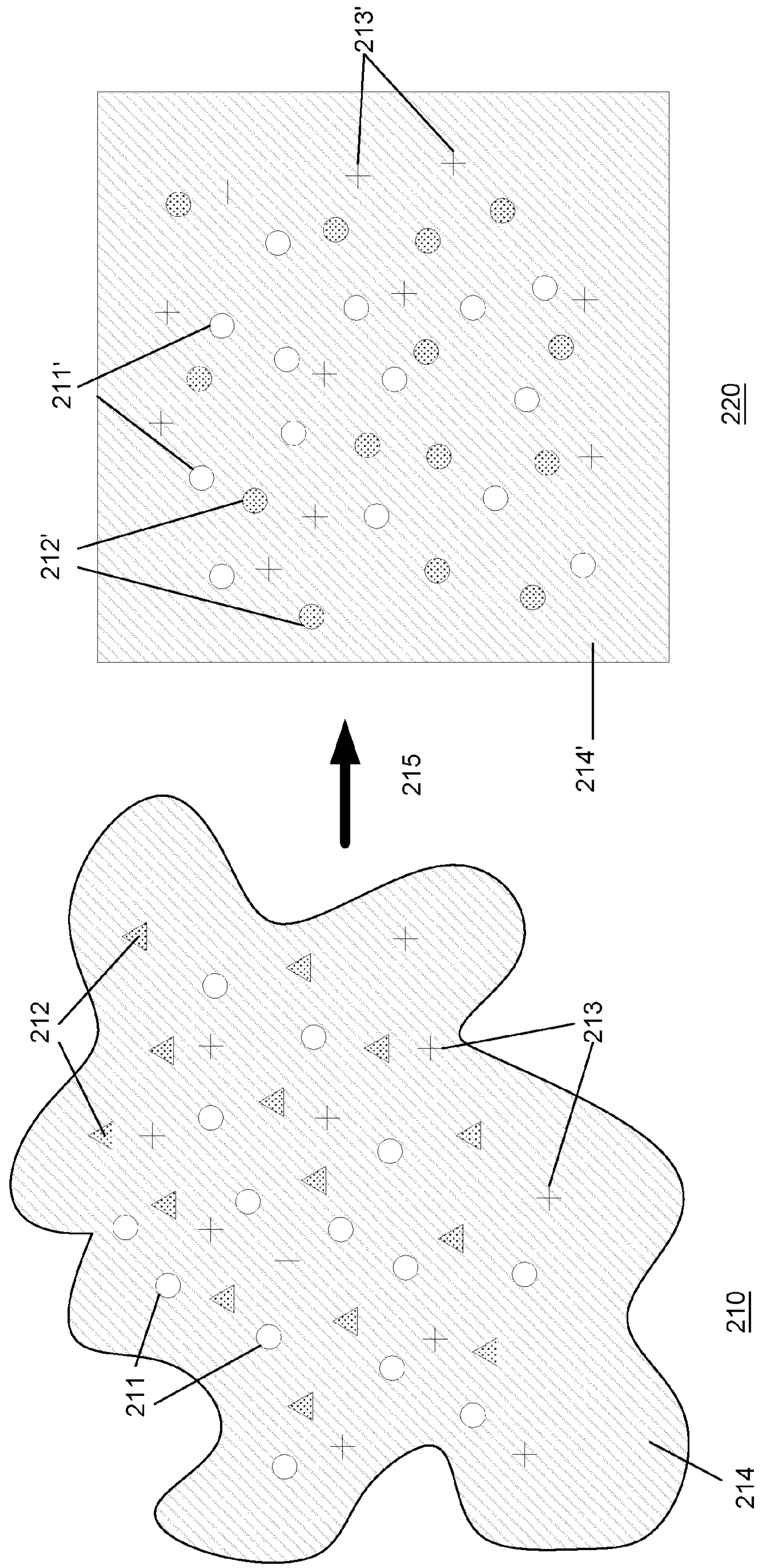


FIG. 2

FIG.3

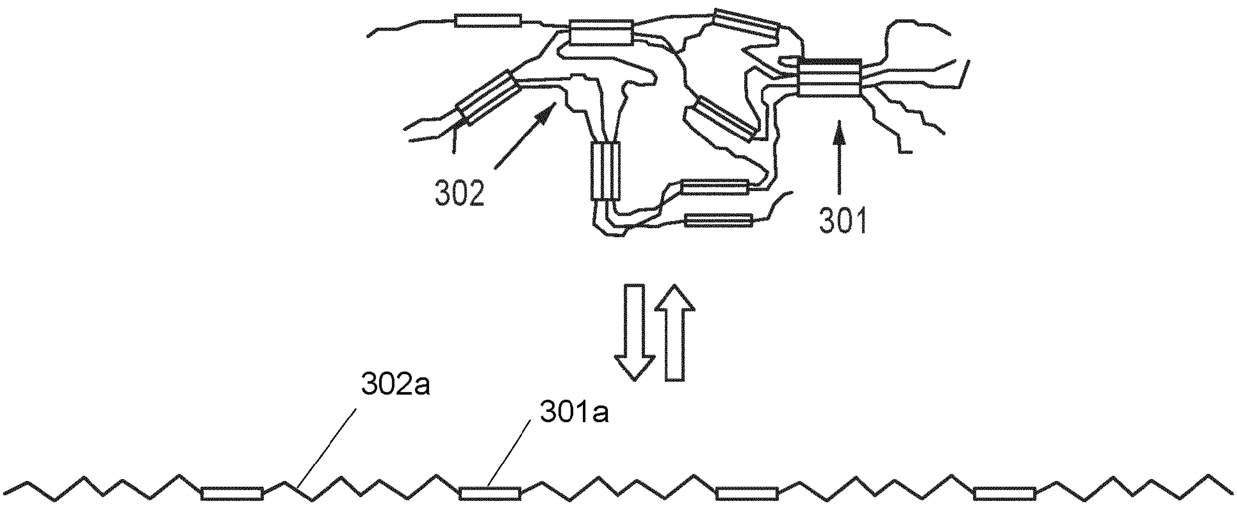

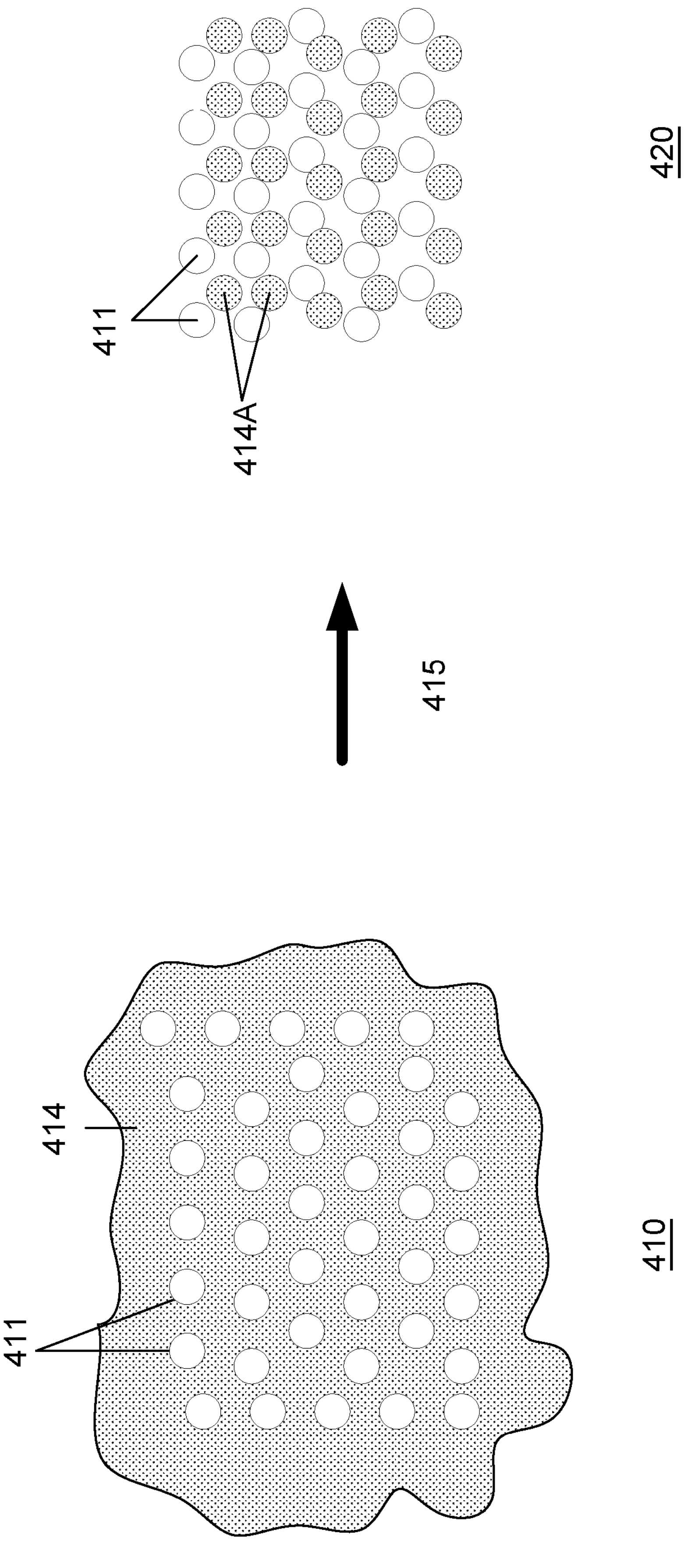


FIG. 4

505

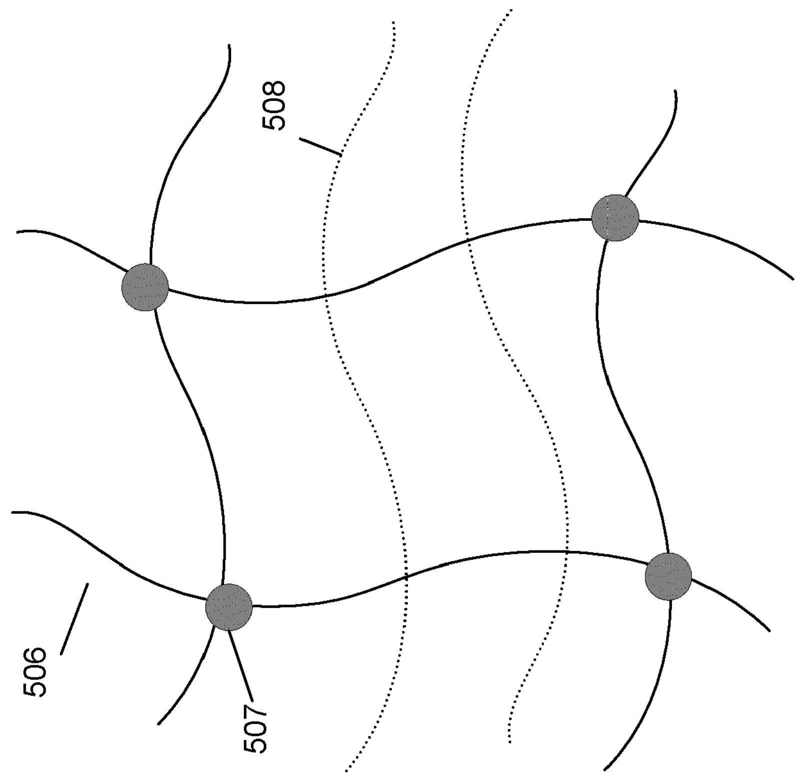


FIG. 5B

500

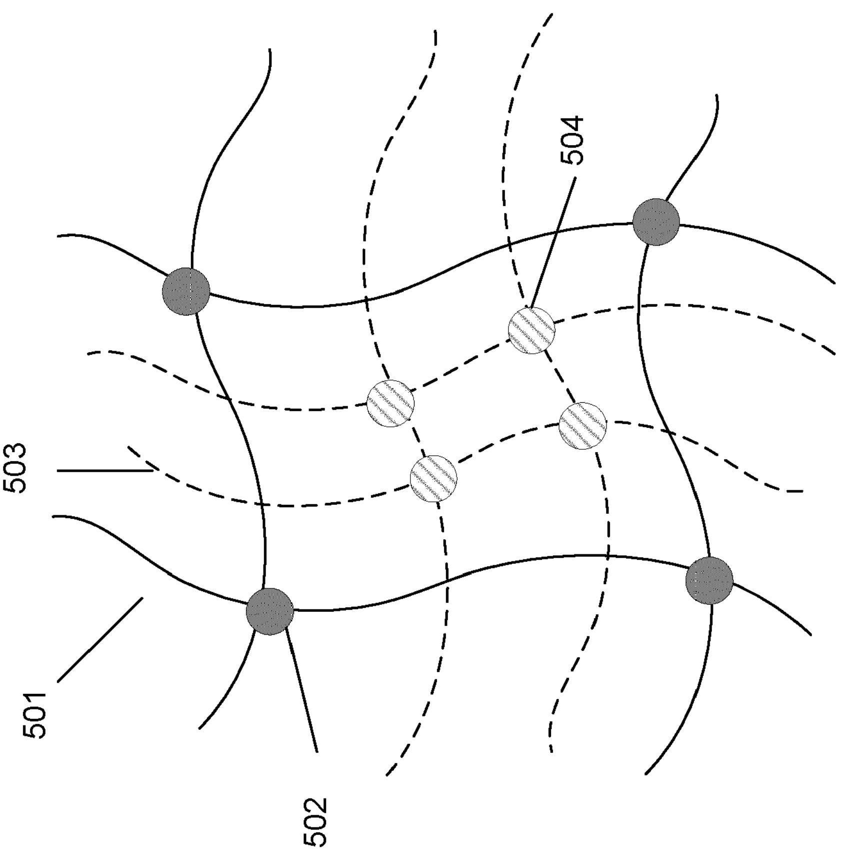


FIG. 5A

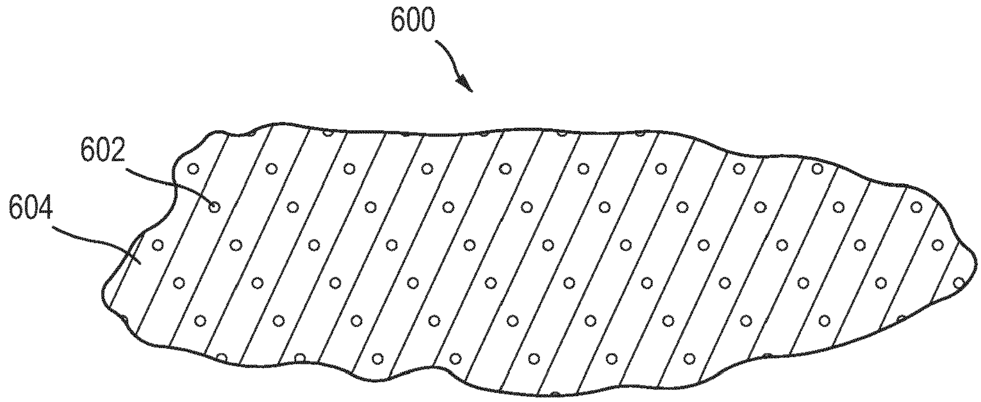


FIG.6A

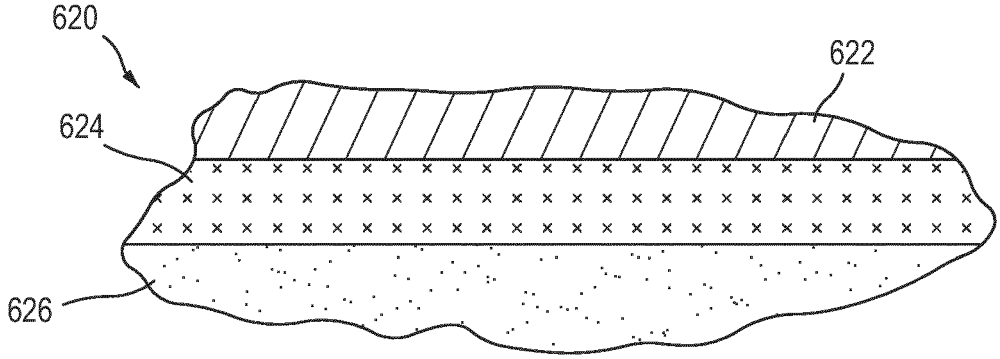


FIG.6B

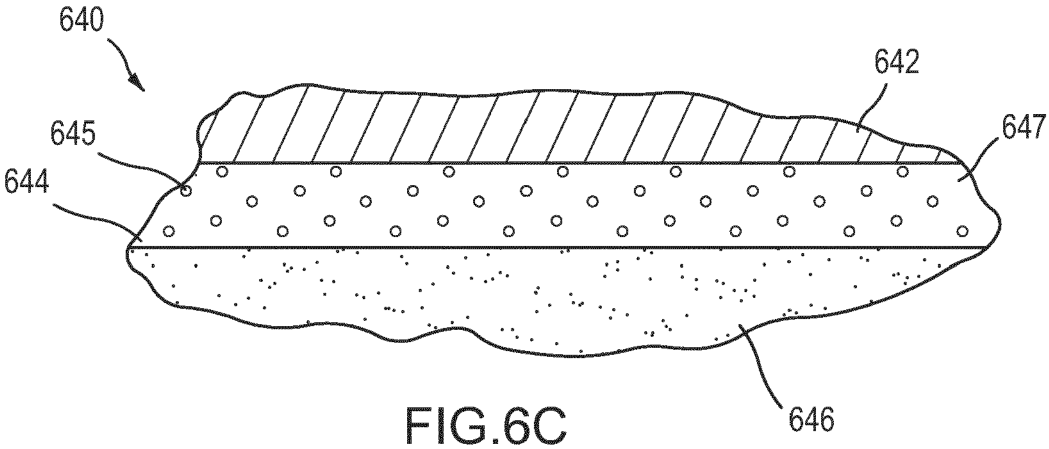


FIG.6C

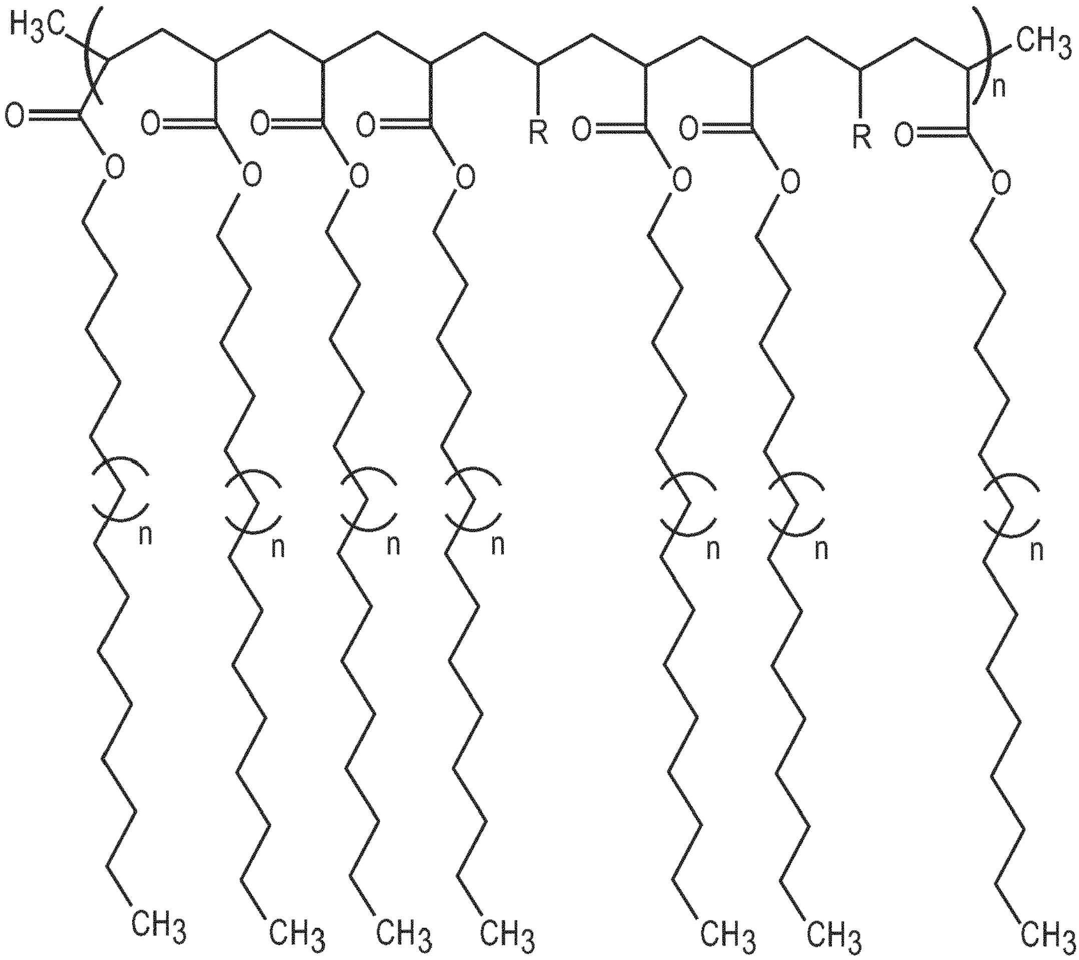

FIG.7

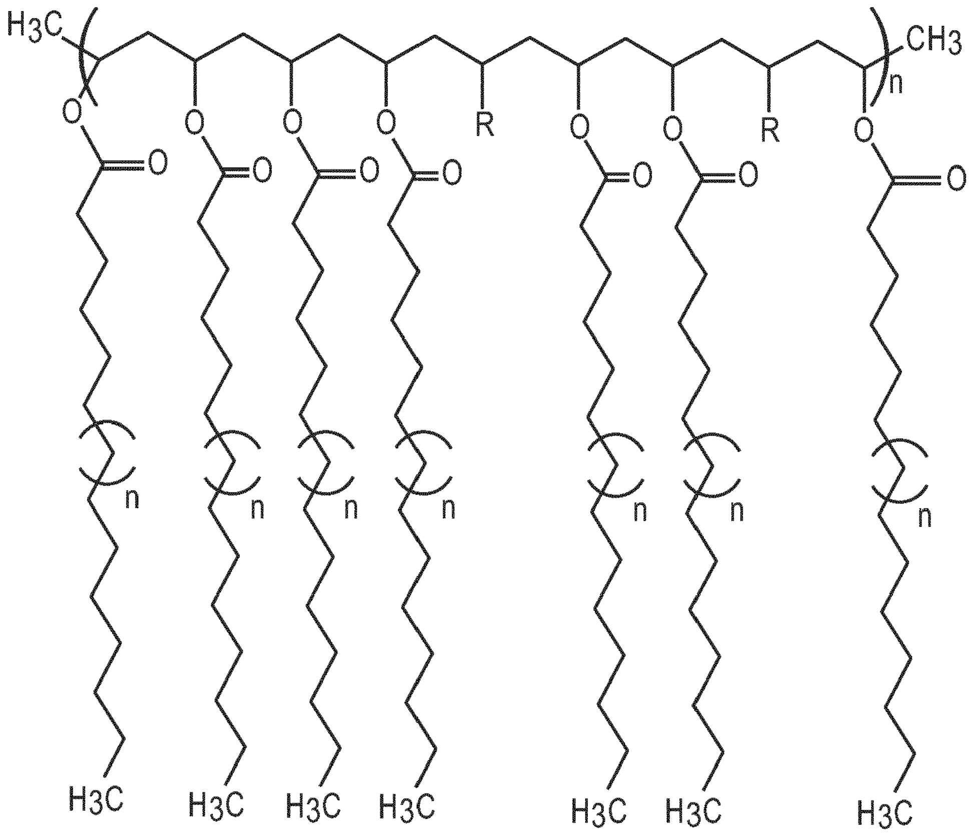

FIG.7A

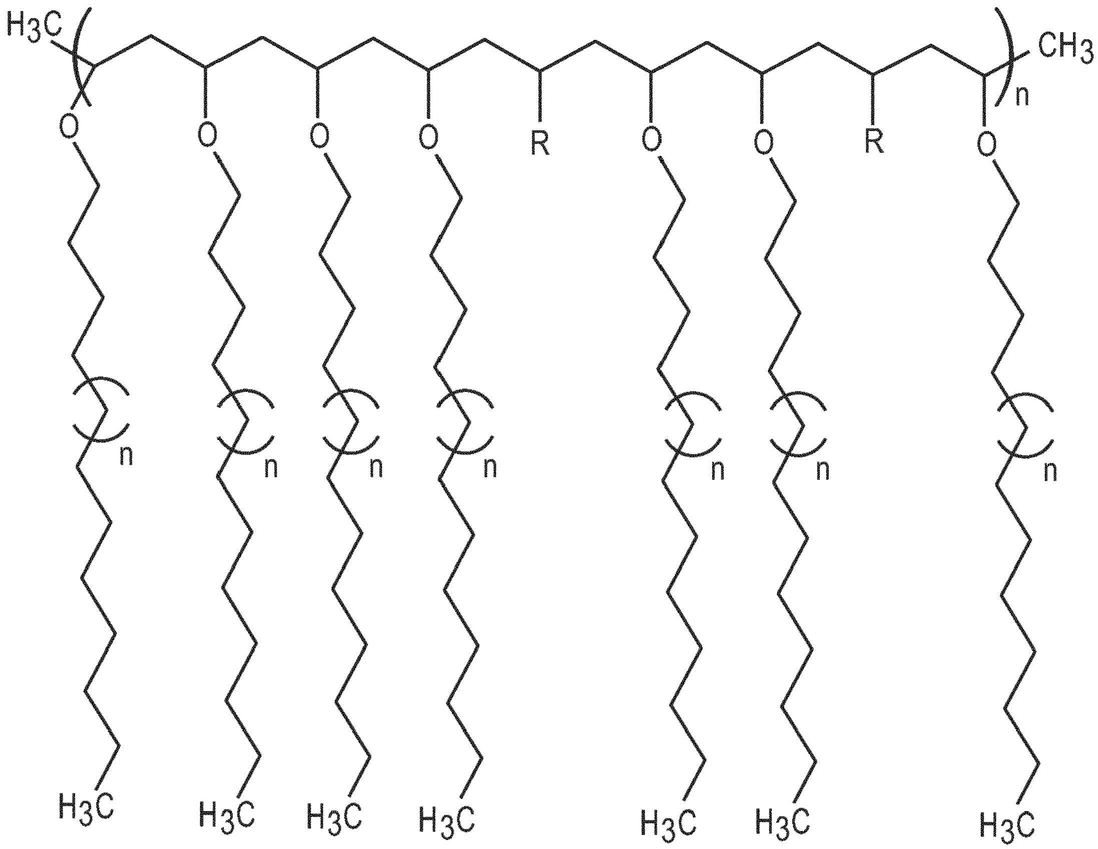

FIG.7B

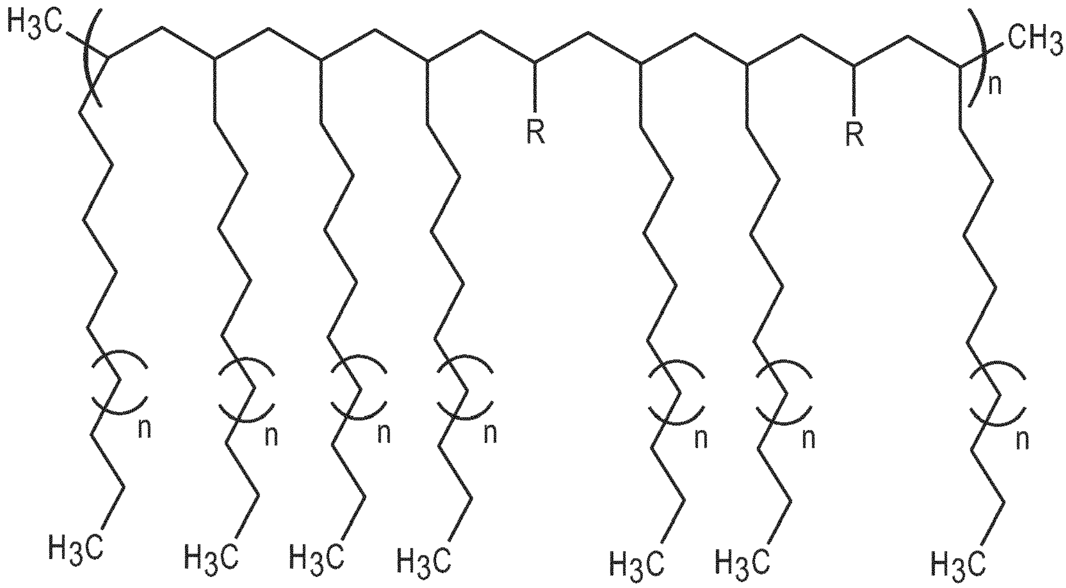

FIG.7C

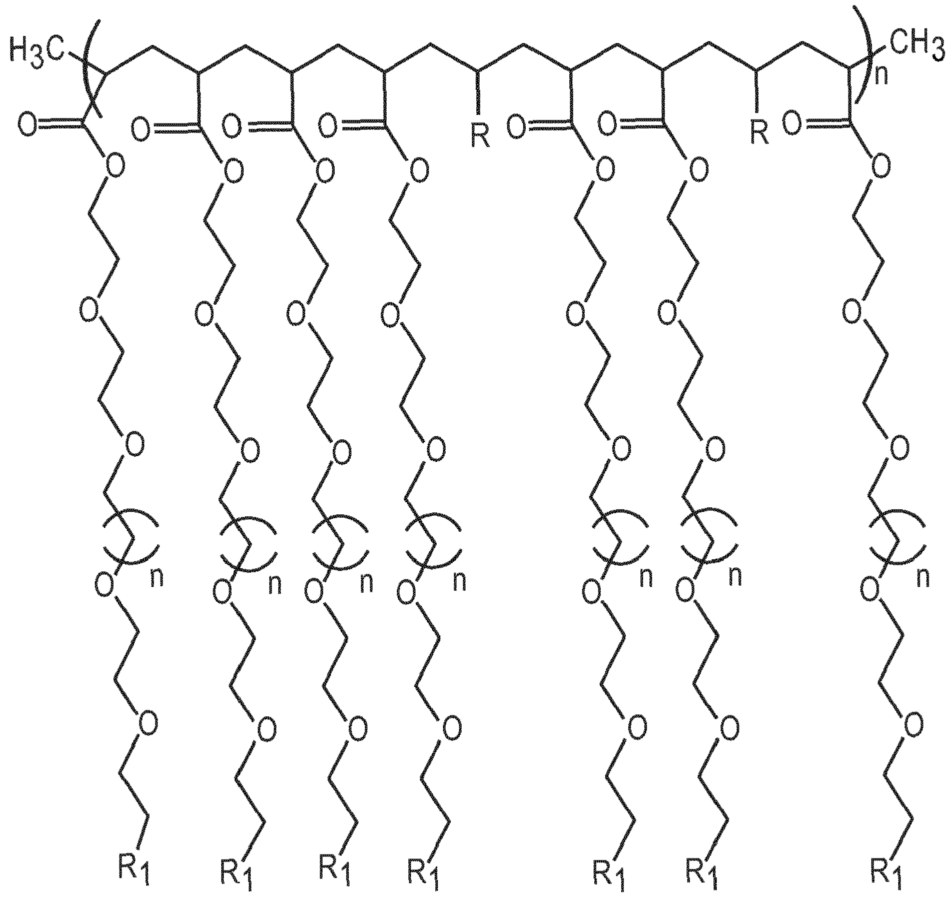

$R_1$ = OH or $OCH_3$

FIG.8

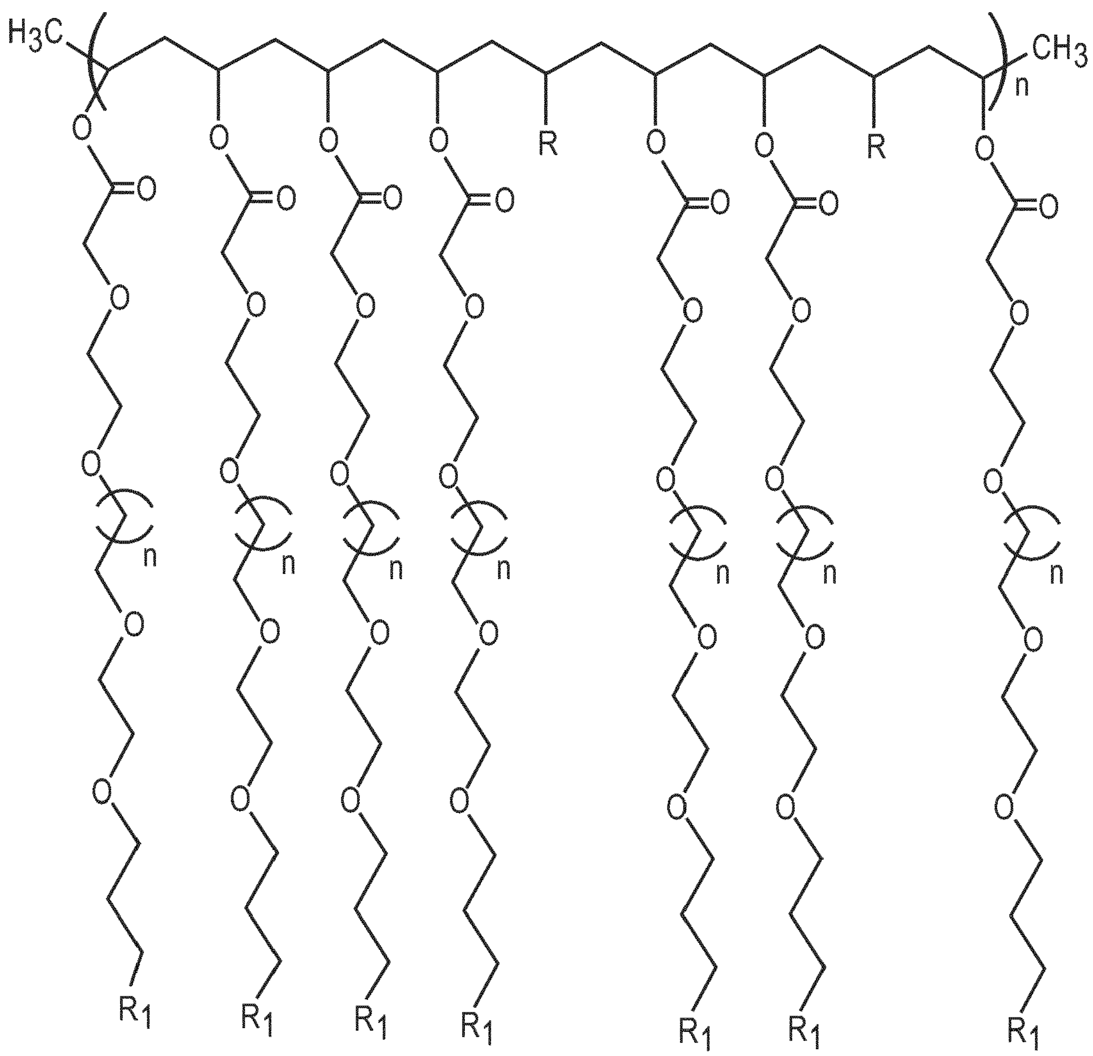

$R_1$ = OH or $OCH_3$

FIG.8A

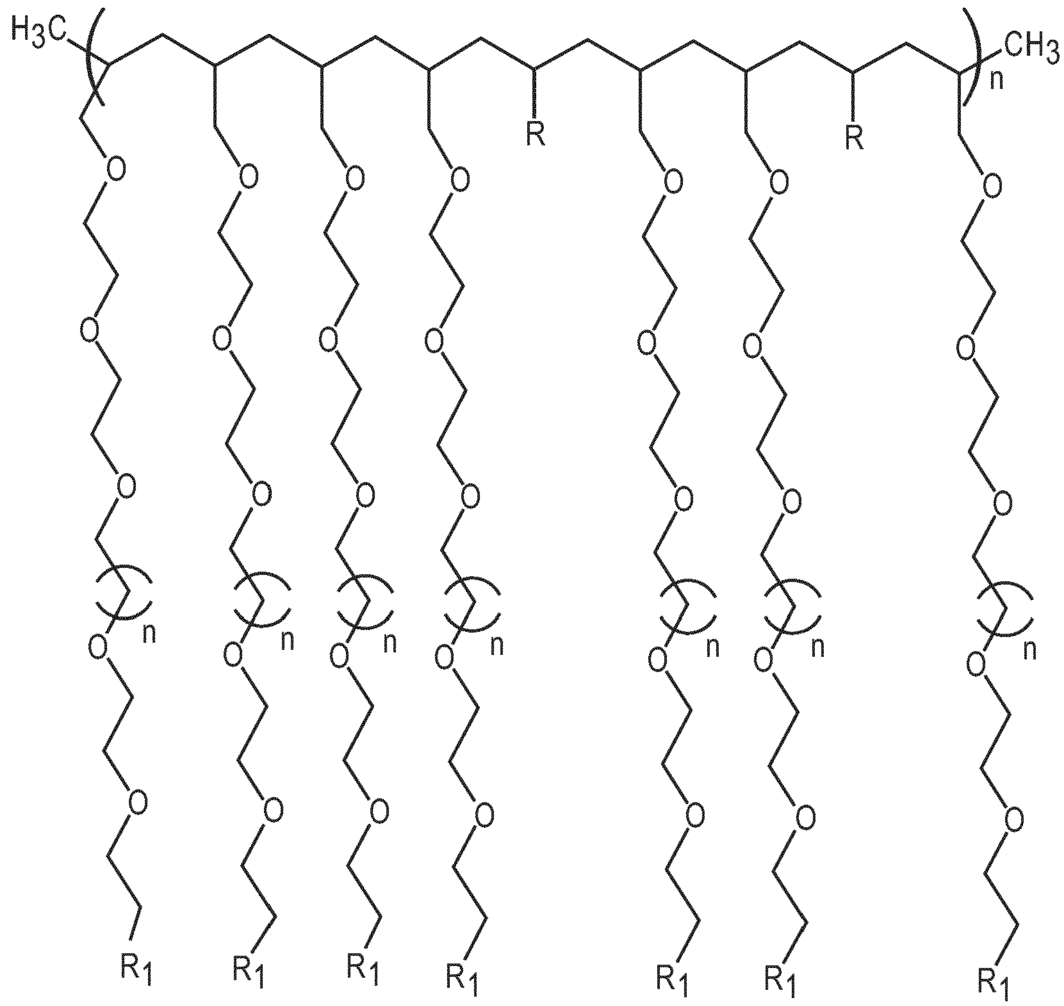

$R_1$ = OH or $OCH_3$

FIG.8B

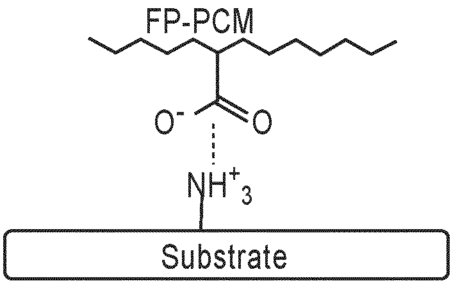

FIG.9A

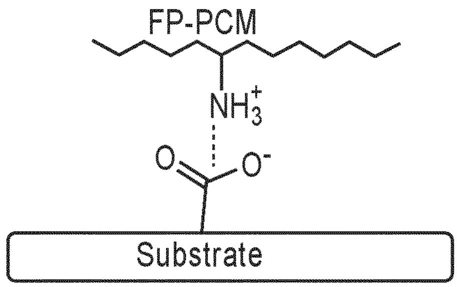

FIG.9B

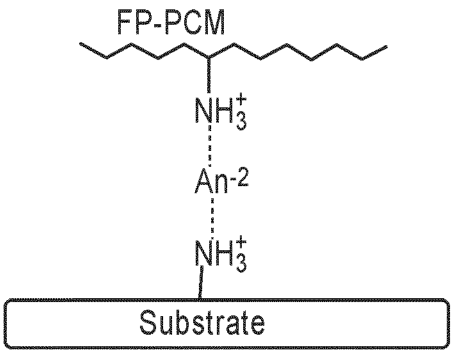

FIG.9C

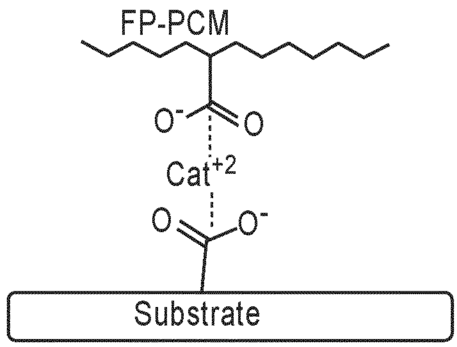

FIG.9D

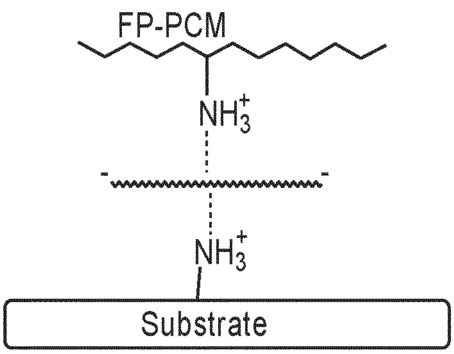

FIG.9E

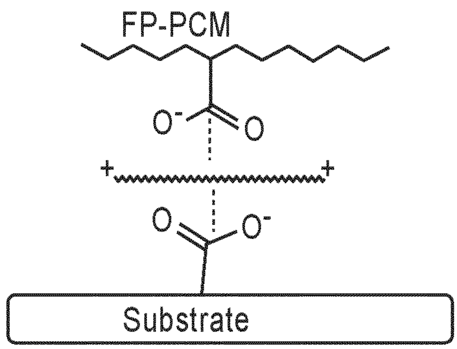

FIG.9F

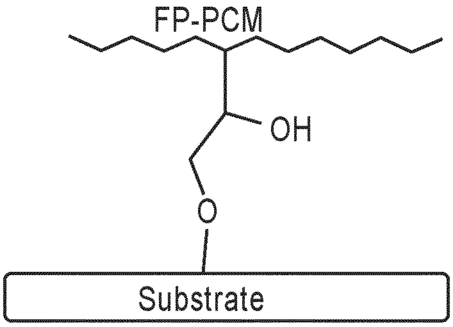

FIG.10A

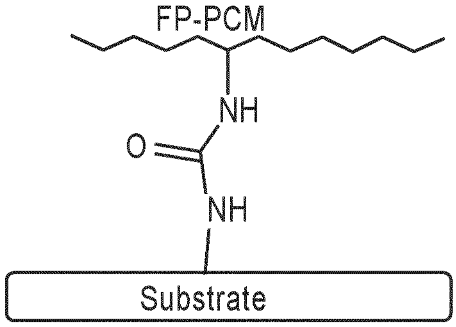

FIG.10B

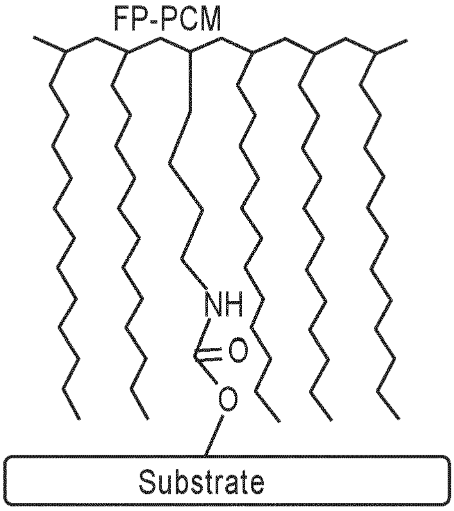

FIG.10C

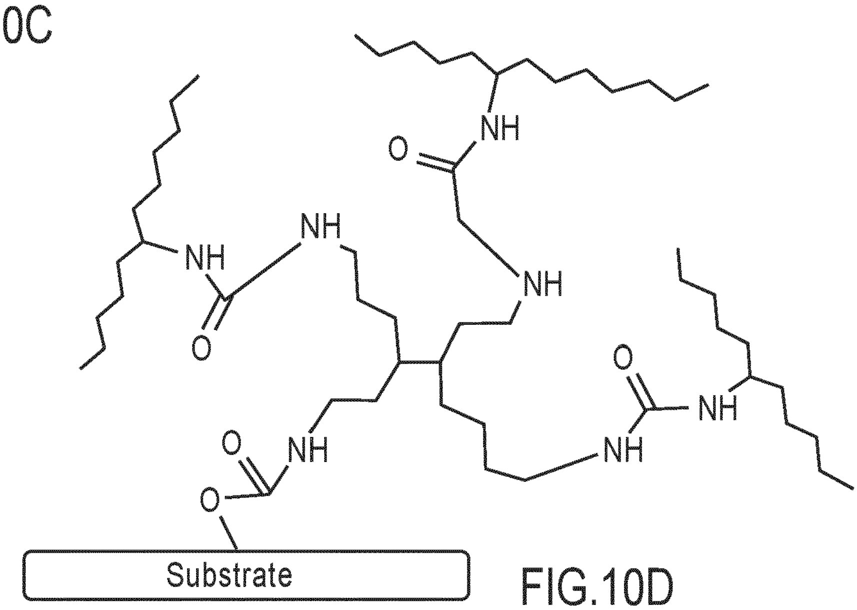

FIG.10D

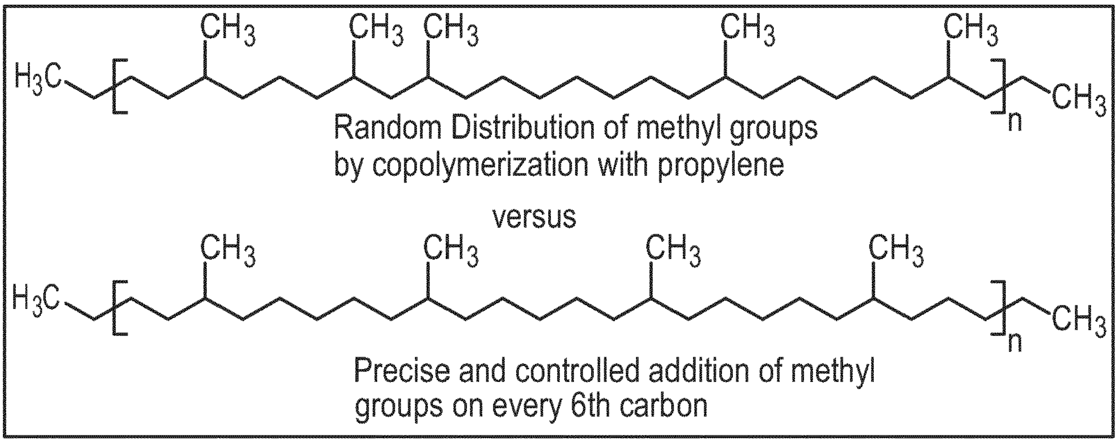
Random Distribution of methyl groups by copolymerization with propylene
versus
Precise and controlled addition of methyl groups on every 6th carbon

FIG. 11

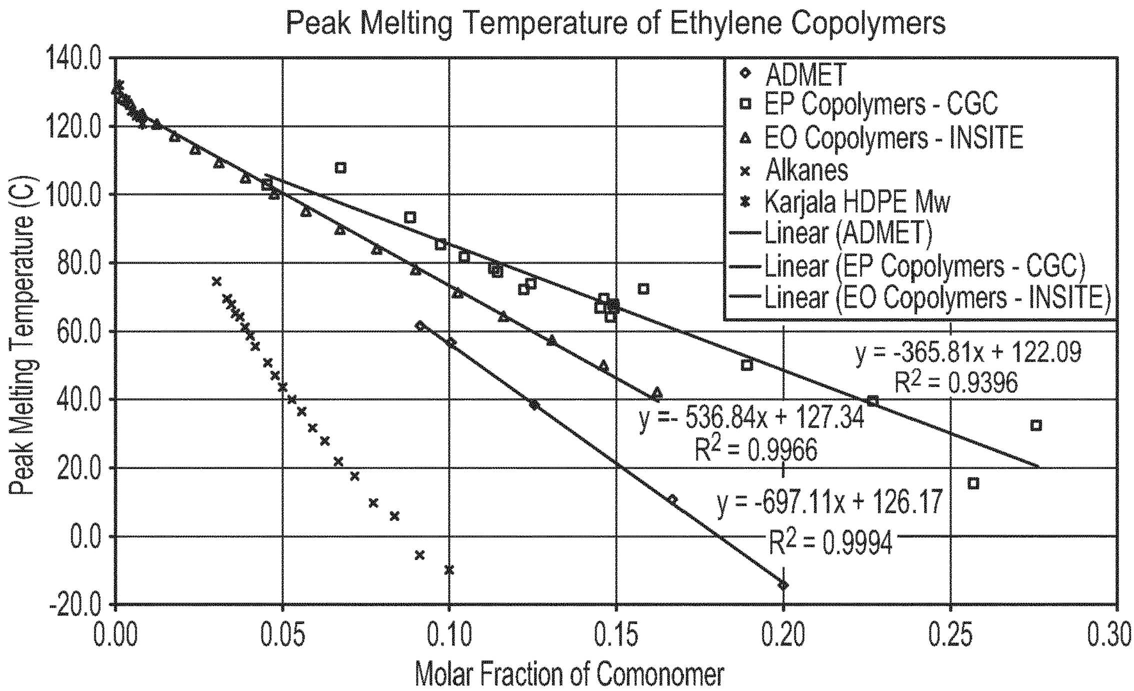
Peak Melting Temperature of Ethylene Copolymers
Peak Melting Temperature (C)
Molar Fraction of Comonomer
ADMET
EP Copolymers - CGC
EO Copolymers - INSITE
Alkanes
Karjala HDPE Mw
Linear (ADMET)
Linear (EP Copolymers - CGC)
Linear (EO Copolymers - INSITE)
y = -365.81x + 122.09
R2 = 0.9396
y =- 536.84x + 127.34
R2 = 0.9966
y = -697.11x + 126.17
R2 = 0.9994
140.0
120.0
100.0
80.0
60.0
40.0
20.0
0.0
-20.0
0.00
0.05
0.10
0.15
0.20
0.25
0.30

FIG. 12

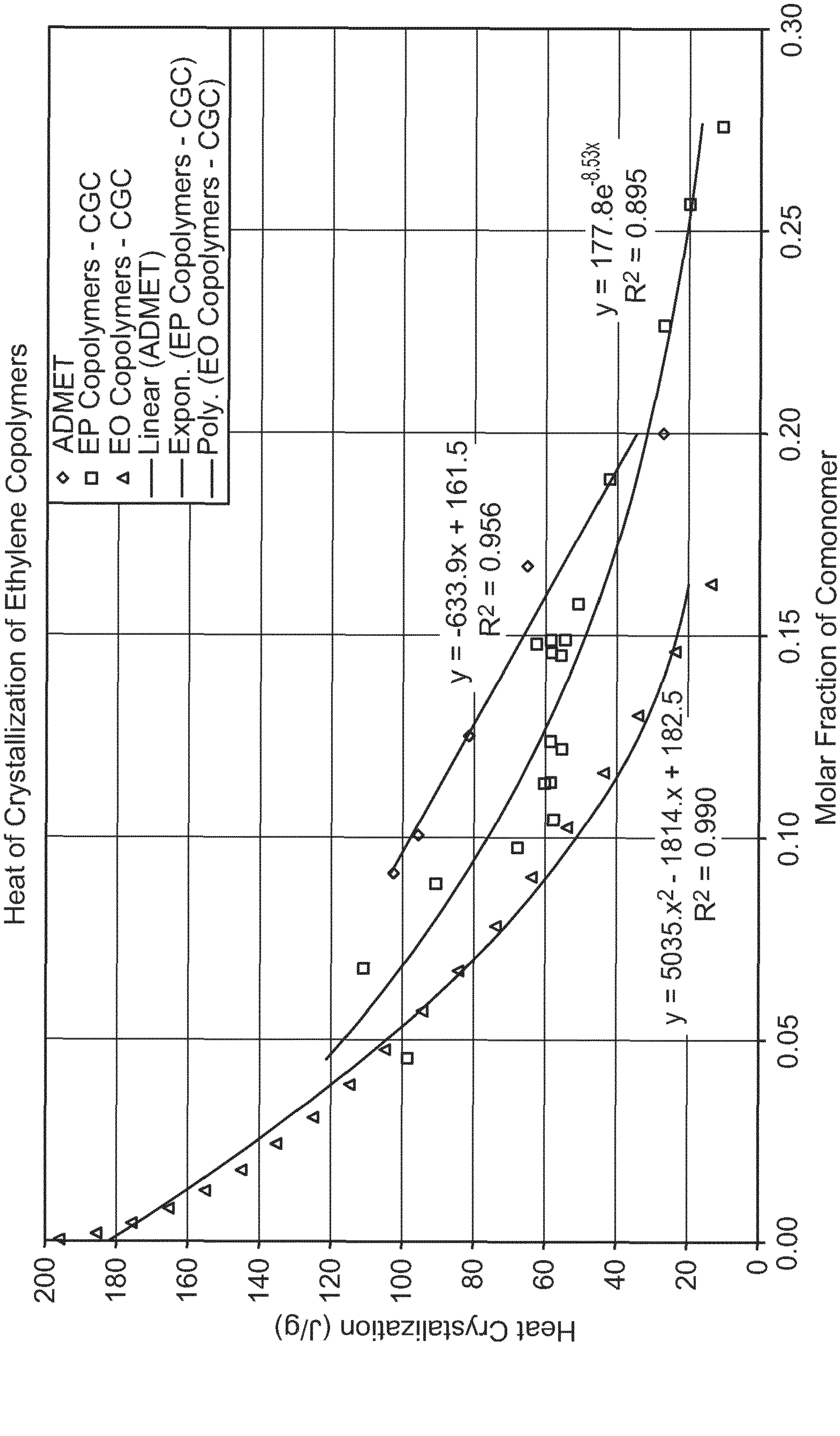
Heat of Crystallization of Ethylene Copolymers
ADMET
EP Copolymers - CGC
EO Copolymers - CGC
Linear (ADMET)
Expon. (EP Copolymers - CGC)
Poly. (EO Copolymers - CGC)
y = -633.9x + 161.5
R2 = 0.956
y = 177.8e-8.53x
R2 = 0.895
y = 5035.x2 - 1814.x + 182.5
R2 = 0.990
Heat Crystalization (J/g)
Molar Fraction of Comonomer
0
20
40
60
80
100
120
140
160
180
200
0.00
0.05
0.10
0.15
0.20
0.25
0.30

FIG. 13

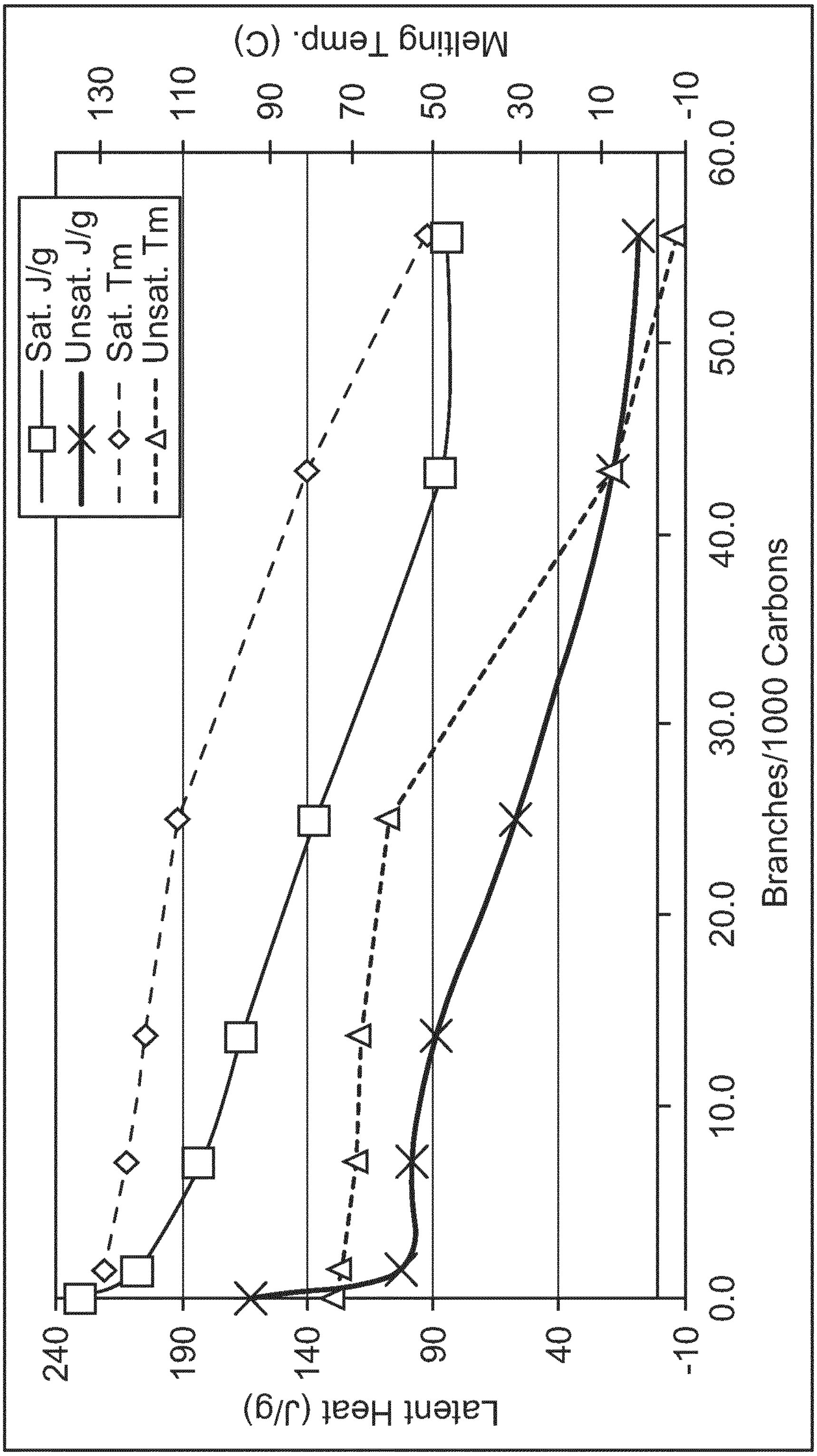
Melting Temp. (C)
130
110
90
70
50
30
10
-10
Sat. J/g
Unsat. J/g
Sat. Tm
Unsat. Tm
240
190
140
90
40
-10
Latent Heat (J/g)
0.0
10.0
20.0
30.0
40.0
50.0
60.0
Branches/1000 Carbons

FIG. 14

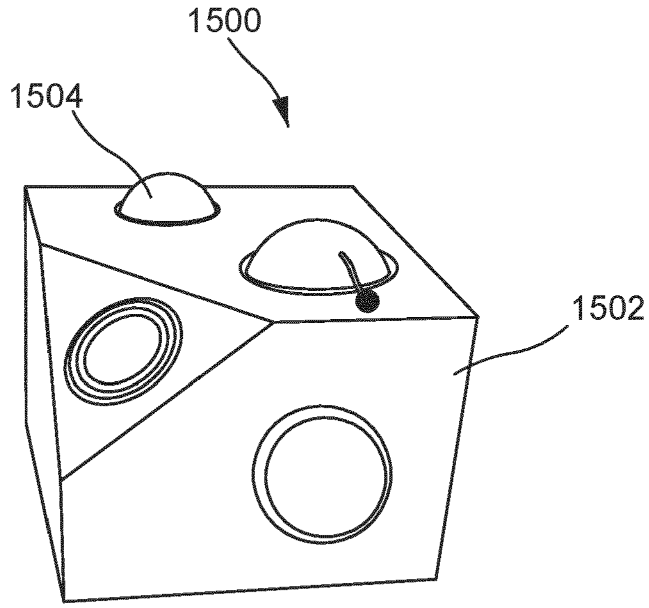


FIG. 15A

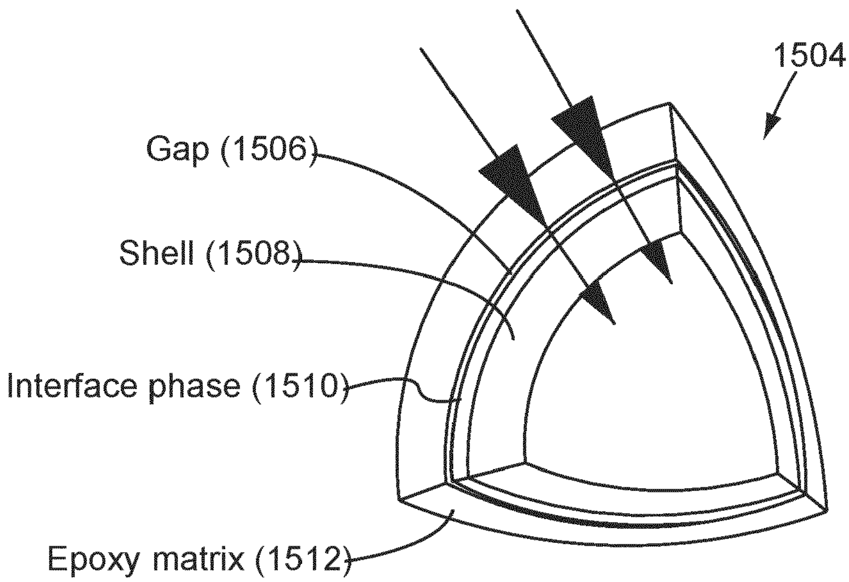


FIG. 15B

| | |
|---|---|
| L7 | L8 |
| L5 | L6 |
| L3 | L4 |
| L1 | L2 |

FIG.16A

| |
|---|
| L6 |
| L5 |
| L4 |
| L3 |
| L2 |
| L1 |

FIG.16B

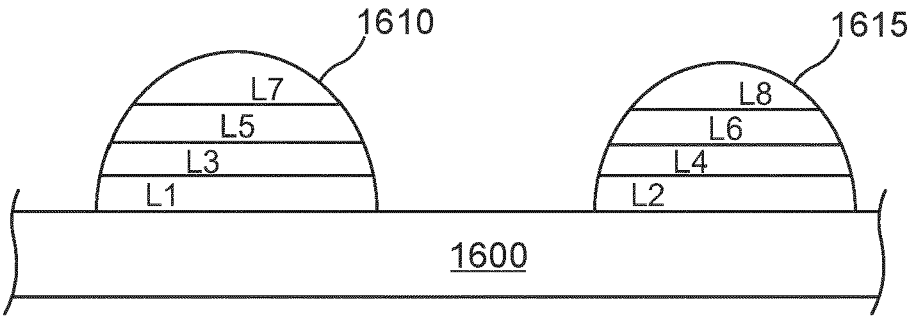


FIG.16C

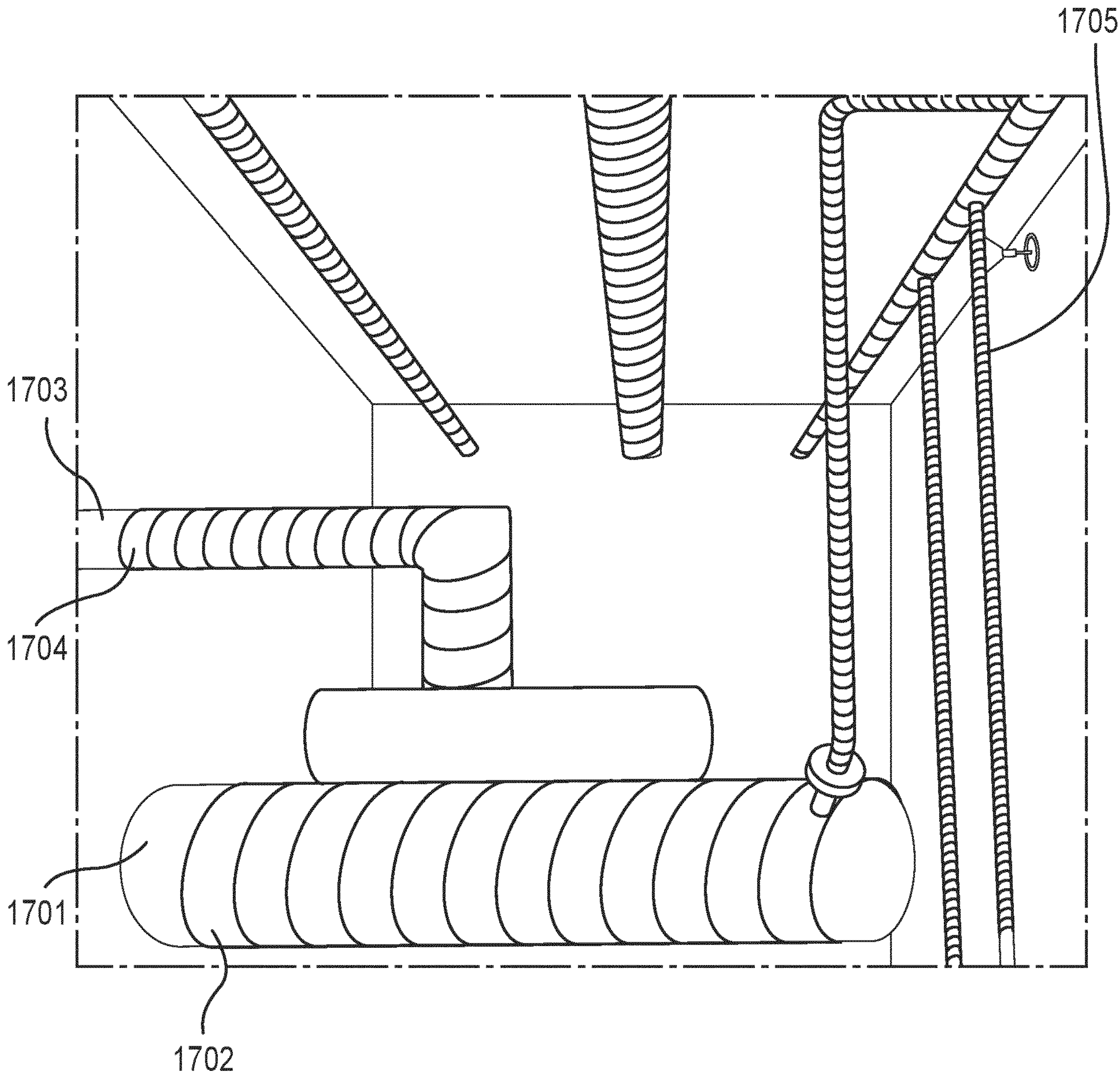


FIG.17

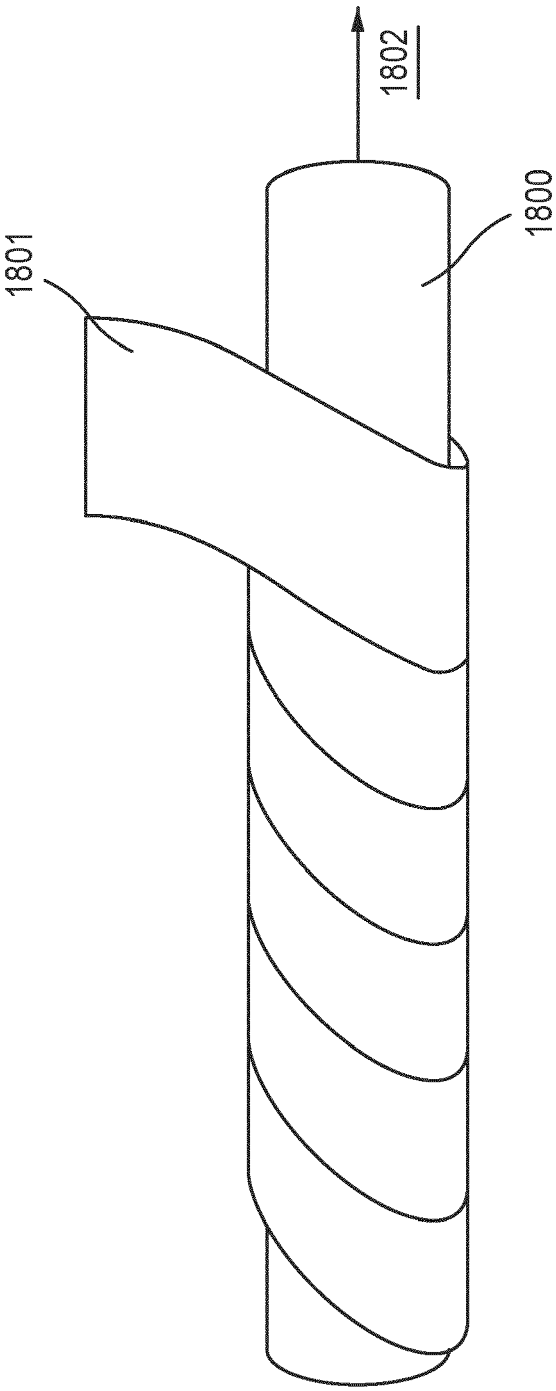


FIG.18

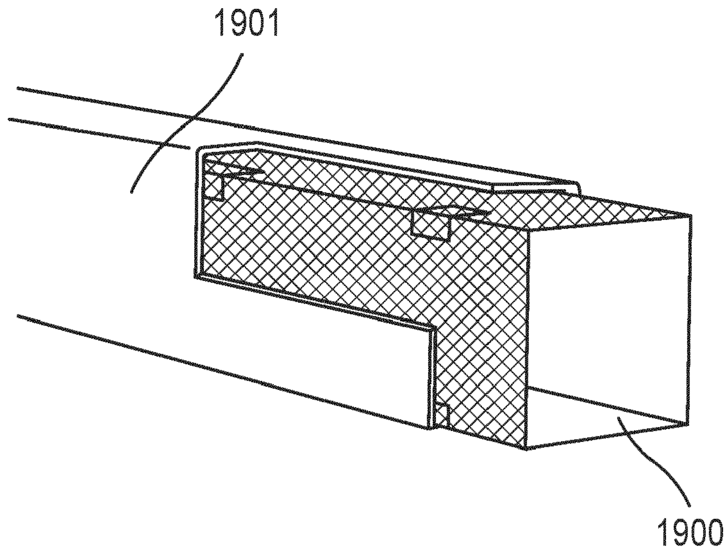


FIG.19

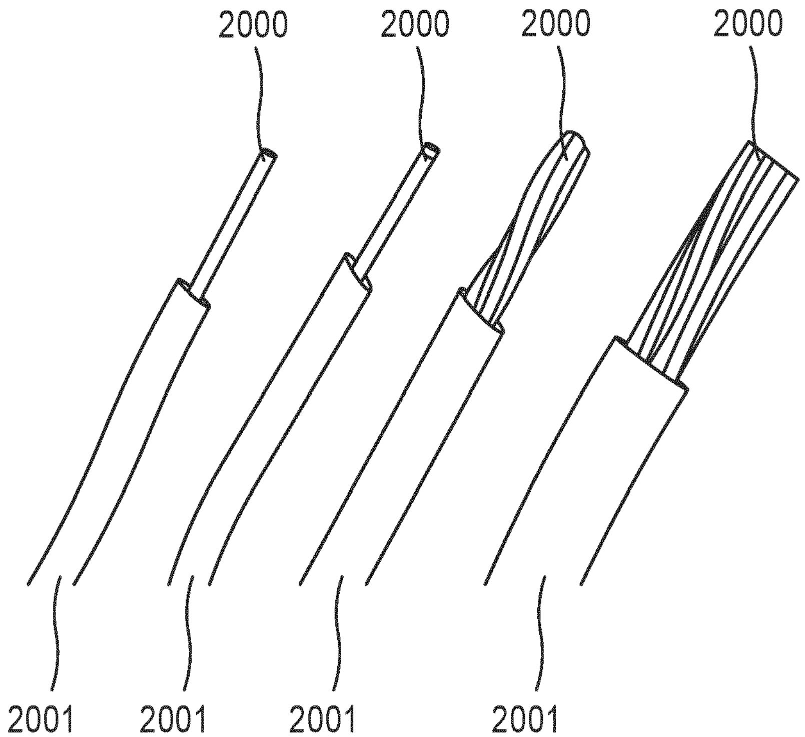


FIG.20

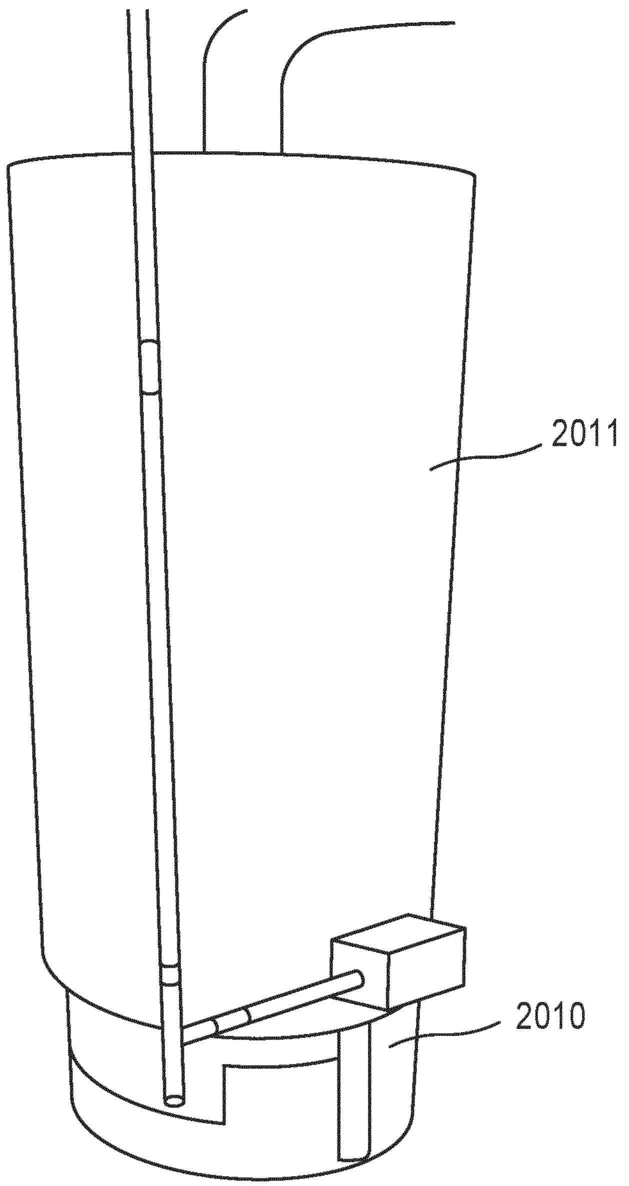


FIG.20A

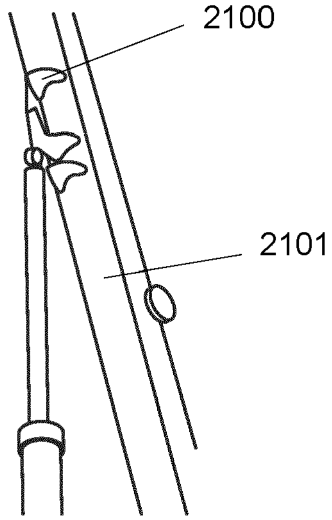


FIG.21A

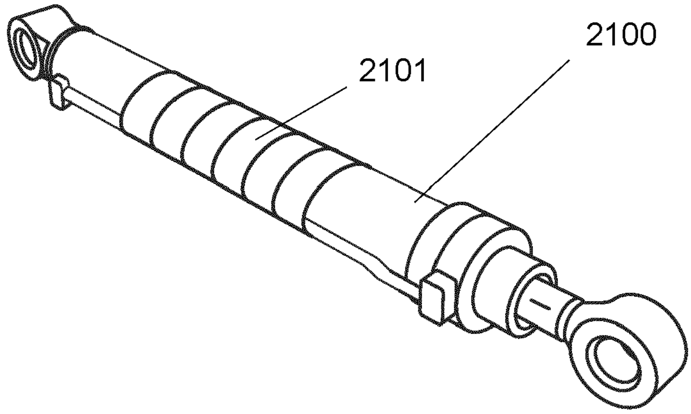


FIG.21B

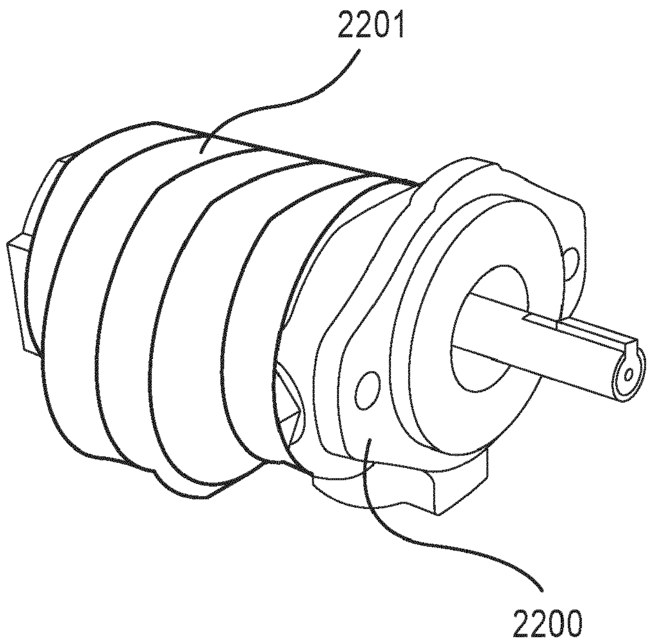


FIG.22

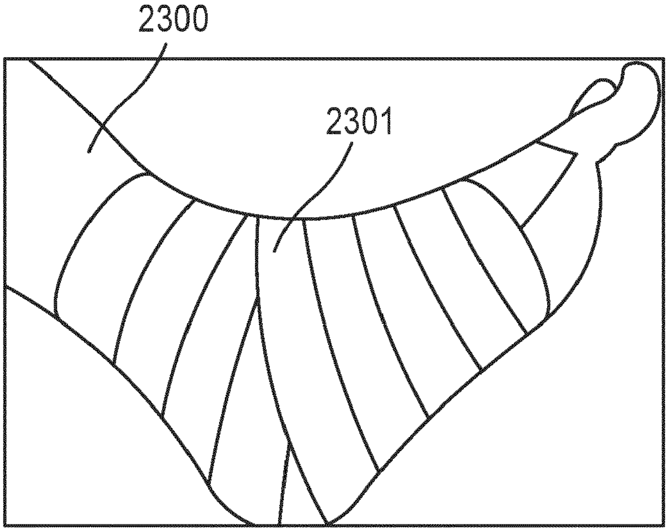


FIG.23

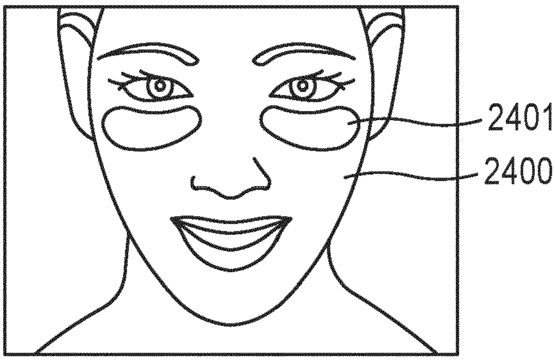


FIG.24

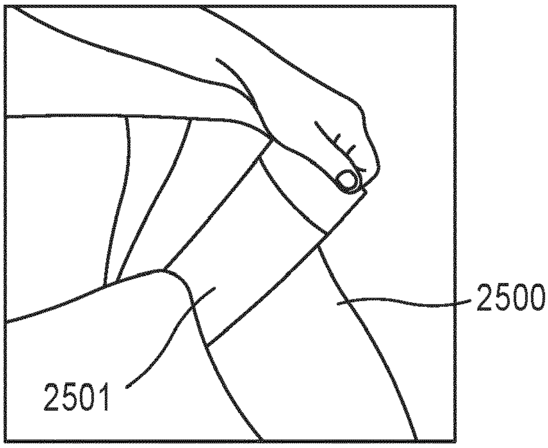


FIG.25

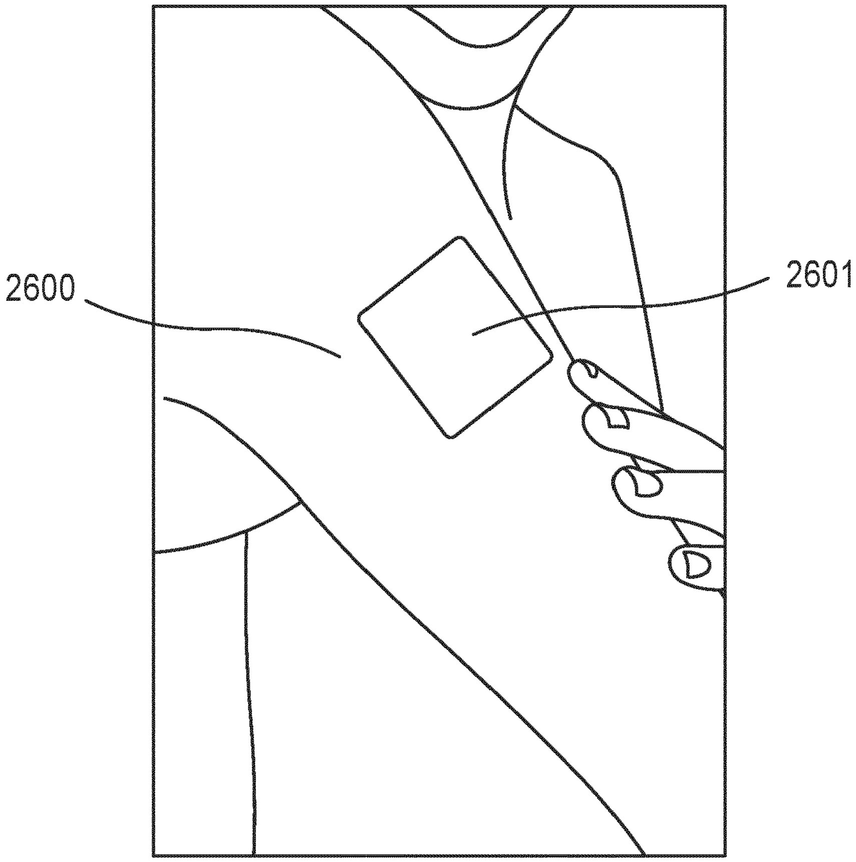


FIG.26

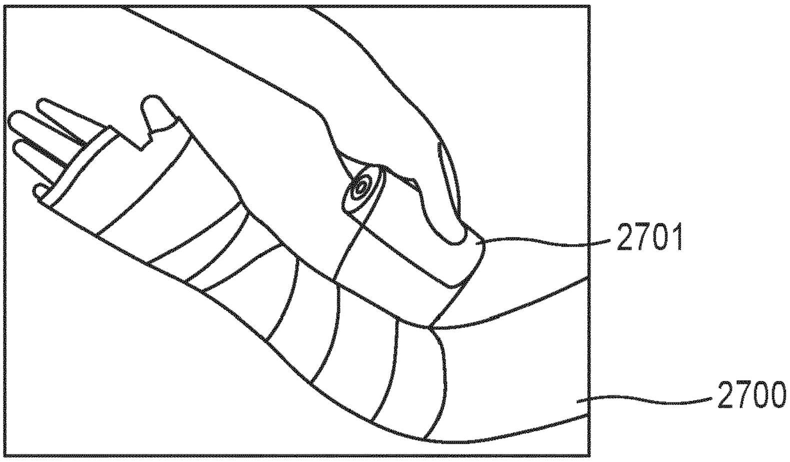


FIG.27

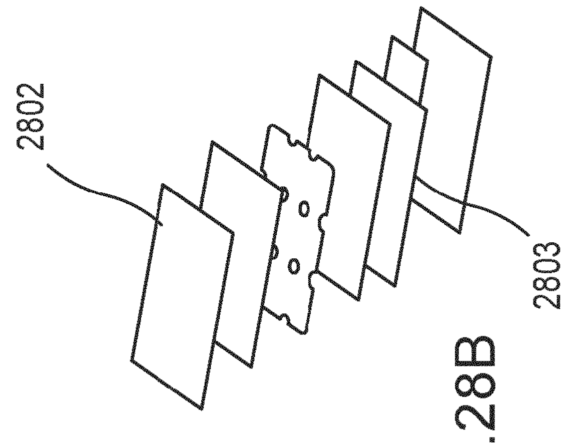


FIG.28B

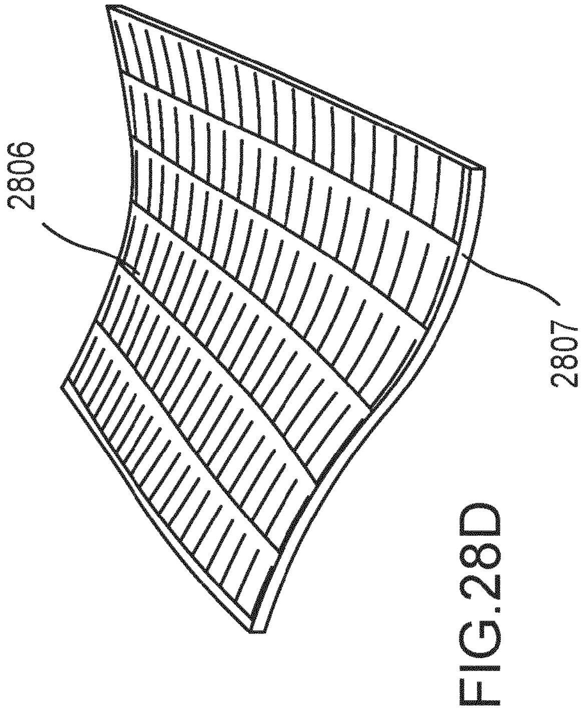


FIG.28D

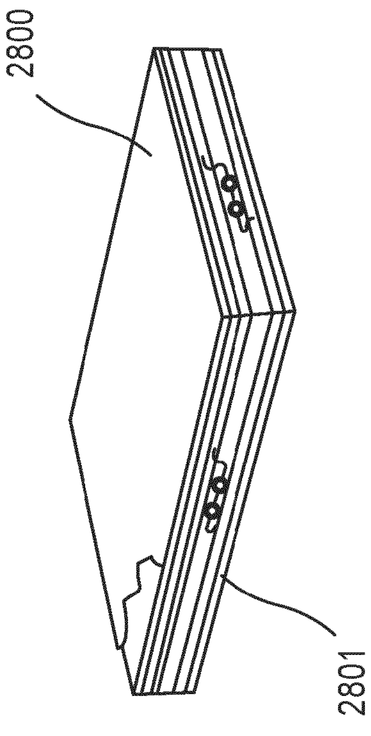


FIG.28A

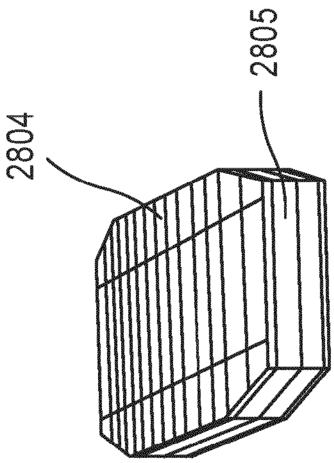


FIG.28C

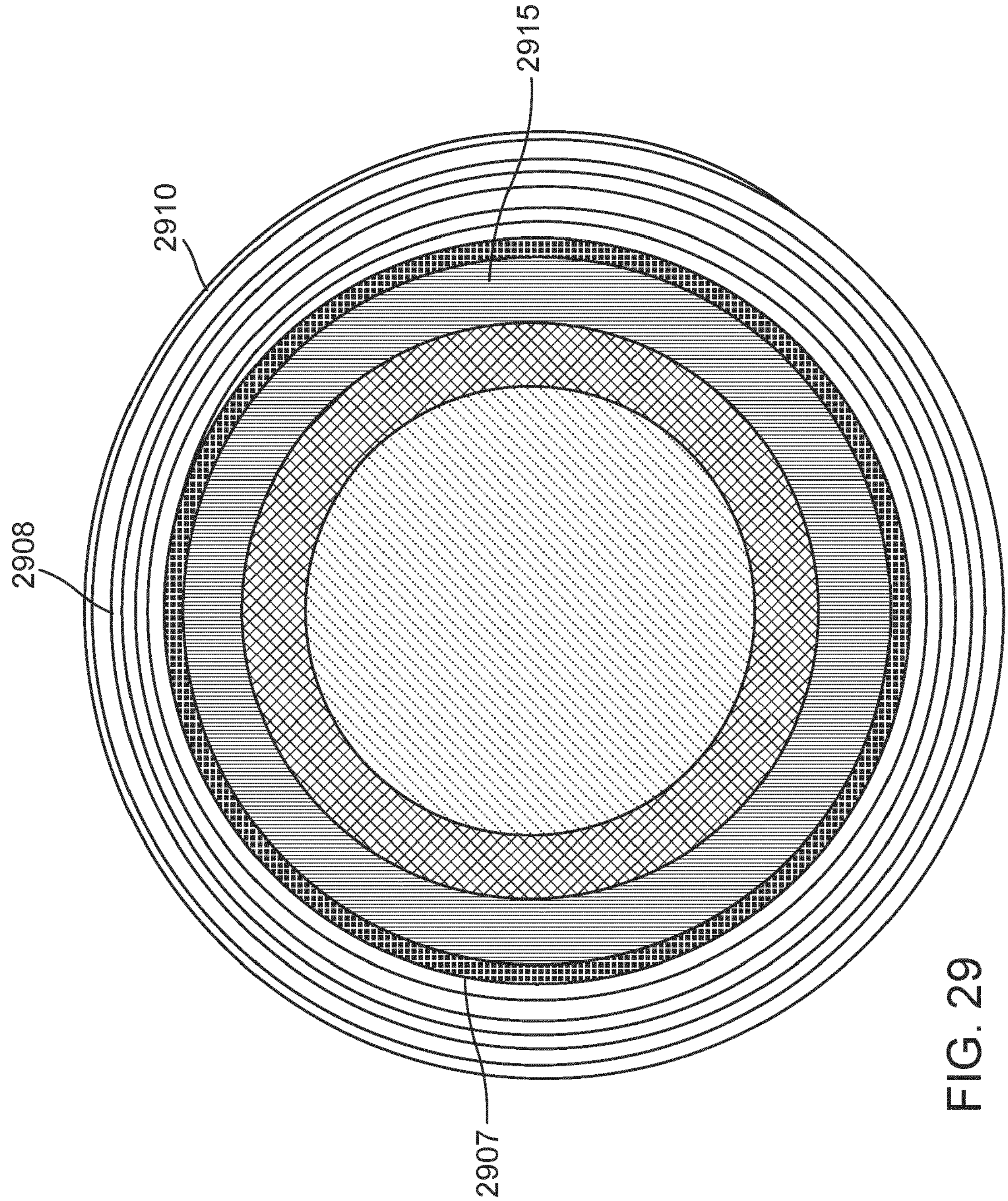


FIG. 29

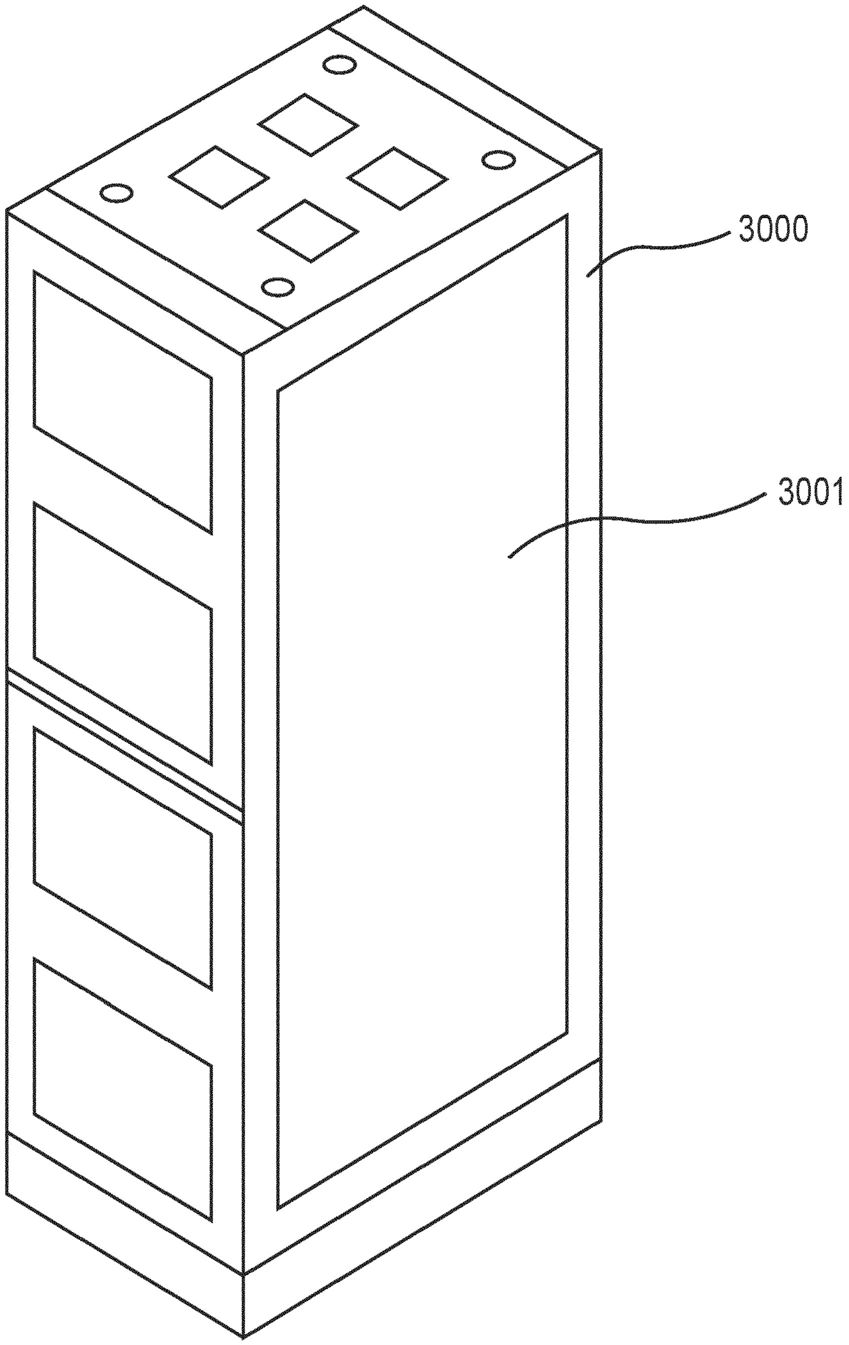


FIG.30

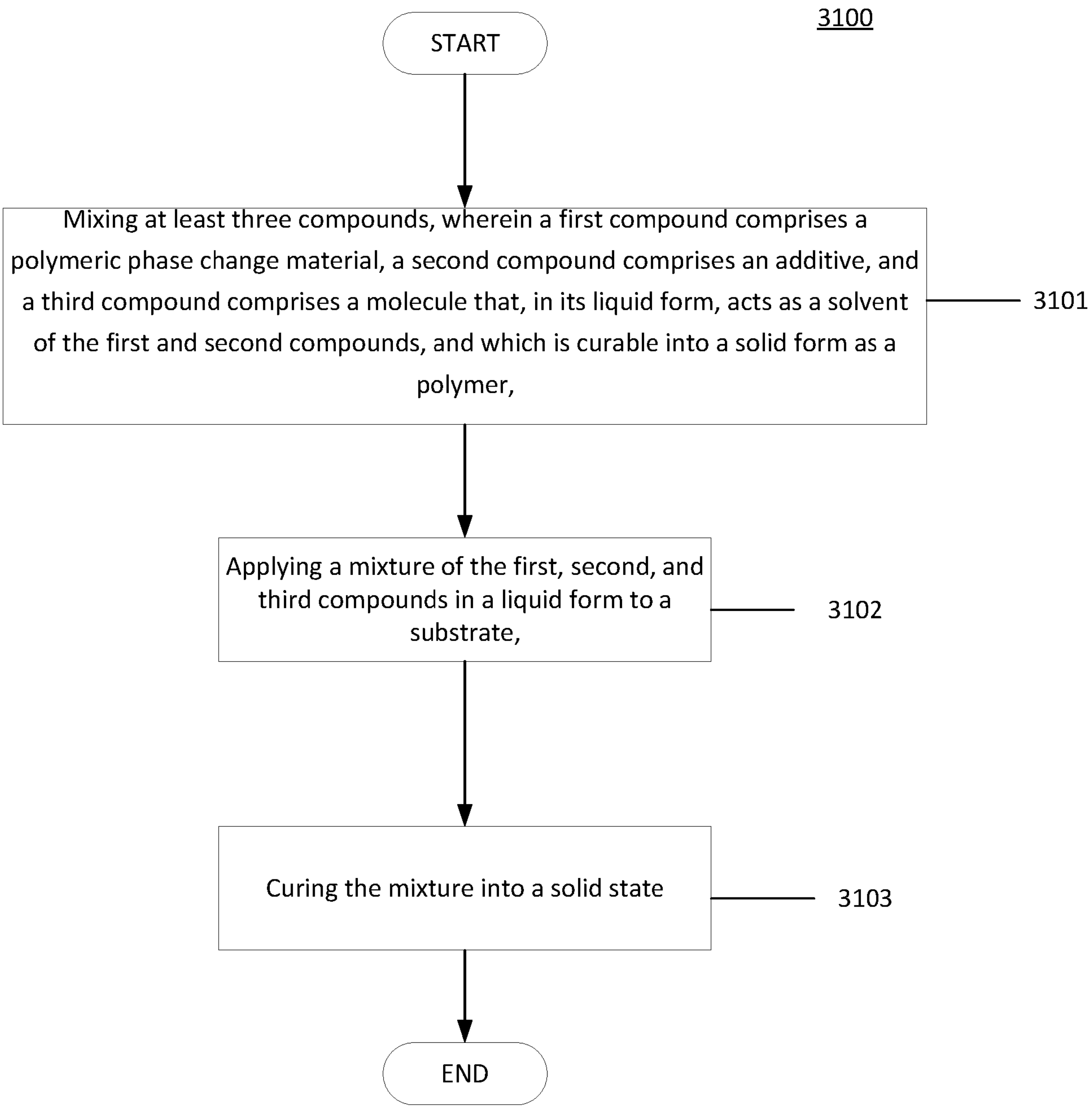
3100
START
Mixing at least three compounds, wherein a first compound comprises a polymeric phase change material, a second compound comprises an additive, and a third compound comprises a molecule that, in its liquid form, acts as a solvent of the first and second compounds, and which is curable into a solid form as a polymer,
3101
Applying a mixture of the first, second, and third compounds in a liquid form to a substrate,
3102
Curing the mixture into a solid state
3103
END

FIG. 31

Europäisches Patentamt
European Patent Office
Office européen des brevets

# EUROPEAN SEARCH REPORT

Application Number
EP 22 15 9578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CH 692 574 A5 (SCHOELLER TEXTIL AG [CH]) 15 August 2002 (2002-08-15) * pages 2-4 * | 1-17 | INV. B41M5/40 H05K7/20 H01L23/36 A61F7/02 C09K5/06 F28D20/02 |
| A | US 2007/241303 A1 (ZHONG HONG [US] ET AL) 18 October 2007 (2007-10-18) * paragraphs [0011] - [0080] * | 1-17 | |
| A | US 2010/264353 A1 (HARTMANN MARK [US] ET AL) 21 October 2010 (2010-10-21) * columns 4-15 * | 1-17 | |
| A | US 6 644 395 B1 (BERGIN JONATHAN M [US]) 11 November 2003 (2003-11-11) * columns 5-12; figures 1-4 * | 1-17 | |
| A | US 2004/024110 A1 (HAMERSKY MARK WILLIAM [US] ET AL) 5 February 2004 (2004-02-05) * paragraphs [0002], [0010], [0011], [0084] - [0090] * | 1-17 | TECHNICAL FIELDS SEARCHED (IPC) |
| A | WO 95/34609 A1 (GATEWAY TECHNOLOGY [US]; R H WYNER ASSOCIATES INC [US]) 21 December 1995 (1995-12-21) * pages 8-26; figure 1 * | 1-17 | F28D H01L |
| A | EP 2 145 934 A1 (OUTLAST TECHNOLOGIES INC [US]) 20 January 2010 (2010-01-20) * pages 5-24 * | 1-17 | |

The present search report has been drawn up for all claims

1

| Place of search | Date of completion of the search | Examiner |
|---|---|---|
| Munich | 29 September 2022 | Merkt, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 15 9578

This annex lists the patent family members relating to the patent documents cited in the above-mentioned European search report. The members are as contained in the European Patent Office EDP file on
The European Patent Office is in no way liable for these particulars which are merely given for the purpose of information.

29-09-2022

| Patent document cited in search report | Publication date | Patent family member(s) | Publication date |
|---|---|---|---|
| CH 692574 A5 | 15-08-2002 | AT 317102 T | 15-02-2006 |
| | | CH 692574 A5 | 15-08-2002 |
| | | EP 1387995 A1 | 11-02-2004 |
| | | JP 2004519369 A | 02-07-2004 |
| | | KR 20030026314 A | 31-03-2003 |
| | | US 2005227047 A1 | 13-10-2005 |
| | | WO 02095314 A1 | 28-11-2002 |
| US 2007241303 A1 | 18-10-2007 | NONE | |
| US 2010264353 A1 | 21-10-2010 | BR 112012026529 A2 | 10-10-2017 |
| | | CN 103038260 A | 10-04-2013 |
| | | EP 2558504 A1 | 20-02-2013 |
| | | JP 6189888 B2 | 30-08-2017 |
| | | JP 2013525518 A | 20-06-2013 |
| | | JP 2015134931 A | 27-07-2015 |
| | | US 2010264353 A1 | 21-10-2010 |
| | | US 2013040526 A1 | 14-02-2013 |
| | | US 2017130112 A1 | 11-05-2017 |
| | | US 2019359871 A1 | 28-11-2019 |
| | | WO 2011129854 A1 | 20-10-2011 |
| US 6644395 B1 | 11-11-2003 | NONE | |
| US 2004024110 A1 | 05-02-2004 | AT 412030 T | 15-11-2008 |
| | | AU 2003249250 A1 | 16-02-2004 |
| | | CA 2489571 A1 | 05-02-2004 |
| | | CN 1671785 A | 21-09-2005 |
| | | EP 1554339 A2 | 20-07-2005 |
| | | EP 1881021 A2 | 23-01-2008 |
| | | JP 2005533906 A | 10-11-2005 |
| | | US 2004024110 A1 | 05-02-2004 |
| | | WO 2004011541 A2 | 05-02-2004 |
| WO 9534609 A1 | 21-12-1995 | AU 2943795 A | 05-01-1996 |
| | | CA 2191342 A1 | 21-12-1995 |
| | | EP 0766720 A1 | 09-04-1997 |
| | | JP H10502137 A | 24-02-1998 |
| | | US 6514362 B1 | 04-02-2003 |
| | | US 2002193028 A1 | 19-12-2002 |
| | | WO 9534609 A1 | 21-12-1995 |
| EP 2145934 A1 | 20-01-2010 | EP 2145934 A1 | 20-01-2010 |
| | | EP 2300223 A1 | 30-03-2011 |
| | | US 2010012883 A1 | 21-01-2010 |
| | | WO 2010008907 A1 | 21-01-2010 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 15 9578

This annex lists the patent family members relating to the patent documents cited in the above-mentioned European search report. The members are as contained in the European Patent Office EDP file on The European Patent Office is in no way liable for these particulars which are merely given for the purpose of information.

29-09-2022

| Patent document cited in search report | Publication date | Patent family member(s) | Publication date |
|---|---|---|---|
| ------------------------------------------------------------------ | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 14614236 A
- US 8587945 B
- US 14614223 A
- US 8404341 B
- US 8221910 B
- US 7569630 B
- US 7879933 B
- US 6169142 B
- EP 1101167 A2
- US 6921431 B, Evans
- US 7105047 B, Simmons
- US 947377
- US 12806267 B, Xu
- US 5929164 A
- EP 92269 A
- WO 2006005491 A1
- US 20060009589 A1
- DE 19826712 A
- DE 19913353 A
- WO 9833761 A
- DE 19957900 A1


### Non-patent literature cited in the description


- **W. FLACK ; D. SOONG ; A. BELL ; D. HESS.** A Mathematical Model for Spin Coating Polymer Resists. *J. Appl. Phys.,* 1984, vol. 56, 1199
- UV Coatings: Basics, Recent Developments, and New Applications. Reinhold Schwalm. Elsevier, 2007
- Stereochemistry. **ANSLYN ; DOUGHERTY.** Modern Physical Organic Chemistry. University Science Books, 2005
- **ATIQULLAH M.** Synthesis of Functional Polylefins using Metallocenes: A Comprehensive Review. *Polymer Reviews,* 2010, vol. 50, 178-230
- Comprehensive Organometallic Chemistry III. **FUJITA T. ; MAKIO H.** Polymerization of Alkenes. Elsevier Ltd, 2007, vol. 11, 691-734
- Functionalized Ethylene Copolymers and Materials. **BAUGHMAN, T.** Olefin Metathesis Polymerization. Univ. of Florida Dissertation, 2006
- **RAKOTOMALALA.** Recent Developments in Halogen Free Flame Retardants for Epoxy Resins for Electrical and Electronic Applications. *Materials,* 2010, vol. 3, 4300-4327
- Innovative Flame Retardants in E&E Applications. Phosphorous, Inorganic and Nitrogen Flame Retardants Association, June 2009
- **FELDMAN.** Polymer Nanocomposites: Flammability. *Journal of Macromolecular Science, Part A: Pure and Applied Chemistry,* 2013
- Effect of Stabilization of Polypropylene During Processing and Its Influence on Long-Term Behavior under Thermal Stress. **ZWEIFEL, HANS.** Polymer Durability, Ciba-Geigy AG, Additives Division, CH-4002, Basel, Switzerland. American Chemical Society, 1996, vol. 25, 375-396
- Ullmanns Encyklopadie der technischen Chemie. vol. 19, 62-65
- *CHEMICAL ABSTRACTS,* 13446-85-0
- *CHEMICAL ABSTRACTS,* 66072-39-7
- *CHEMICAL ABSTRACTS,* 9003-35-4
- *CHEMICAL ABSTRACTS,* 37382-79-9
- *CHEMICAL ABSTRACTS,* 27043-37-4
- *CHEMICAL ABSTRACTS,* 16096-30-3
- *CHEMICAL ABSTRACTS,* 13410-58-7
- Advances in Polymer Science. Springer Berlin, 1974, vol. 14
- **K. K. DIETLIKER.** Chemistry and Technology of UV- and EB-Formulation for Coatings. *Inks and Paints,* vol. 3
- Photoinitiators for Free Radical and Cationic Polymerization. SUA Technology Ltd
- Polymer Handbook. Wiley & Sons